# EUROPEAN PATENT SPECIFICATION

(11) **EP 2 411 522 B1**
(45) Date of publication and mention of the grant of the patent: **06.09.2017**
(21) Application number: 10761136.0
(22) Date of filing: 29.03.2010
(51) Int. Cl.: C12N 15/12, C07H 21/00, C07K 14/47, C07K 16/18, C12Q 1/68, G01N 33/50, G01N 33/574, G01N 33/68, C07K 19/00, C12N 15/62

(54) **ABERRANT MITOCHONDRIAL DNA, ASSOCIATED FUSION TRANSCRIPTS AND TRANSLATION PRODUCTS AND HYBRIDIZATION PROBES THEREFOR**
ABERRANTE MITOCHONDRIALE DNA, ENTSPRECHENDE FUSIONSTRANSKRIPTE SOWIE ÜBERSETZUNGSPRODUKTE UND HYBRIDISIERUNGSSONDEN DAFÜR
ADN MITOCHONDRIAL ABERRANT, TRANSCRITS DE FUSION ET PRODUITS DE TRADUCTION ASSOCIES ET SONDES D'HYBRIDATION COMPLEMENTAIRES

(30) Priority: 27.03.2009 WO PCT/CA2009/000351
(43) Date of publication of application: 01.02.2012
(73) Proprietor: MDNA Life Sciences Inc., Wilmington, DE 19801 (US)
(72) Inventor: PARR, Ryan, Thunder Bay, Ontario P7G 1J4 (CA); DAKUBO, Gabriel, Thunder Bay, Ontario P7J 1H7 (CA); HARBOTTLE, Andrew, Newcastle upon Tyne Tyne and Wear NE1 3DW (GB); REGULY, Brian, Vancouver, British Columbia V5Z 1R3 (CA); CREED, Jennifer, Broomfield, CO 80023 (US); ROBINSON, Kerry, Thunder Bay, Ontario P7C 4A5 (CA); KLEIN, Daniel, Thunder Bay, Ontario P7E 5S8 (CA)
(74) Representative: Nederlandsch Octrooibureau
(86) International application number: PCT/CA2010/000423
(87) International publication number: WO 2010/115261

(56) References cited:
- WO-A1-2009/117811
- G. GASPARRE ET AL: "Disruptive mitochondrial DNA mutations in complex I subunits are markers of oncocytic phenotype in thyroid tumors", PROCEEDINGS OF THE NATIONAL ACADEMY OF SCIENCES, vol. 104, no. 21, 22 May 2007 (2007-05-22) , pages 9001-9006, XP055044227, ISSN: 0027-8424, DOI: 10.1073/pnas.0703056104 -& DATABASE UniProt [Online] 21 August 2007 (2007-08-21), "Cytochrome c oxidase subunit 2;", XP002687300, retrieved from EBI accession no. UNIPROT:A6ZHA9 Database accession no. A6ZHA9
- JENNIFER MAKI ET AL: "Mitochondrial genome deletion aids in the identification of false- and true-negative prostate needle core biopsy specimens", AMERICAN JOURNAL OF CLINICAL PATHOLOGY, AMERICAN SOCIETY FOR CLINICAL PATHOLOGY, US, vol. 129, no. 1, 1 January 2008 (2008-01-01), pages 57-66, XP008132881, ISSN: 0002-9173, DOI: 10.1309/UJJTH4HFEPWAQ78Q
- ZHU WEIZHU ET AL: "Large-scale mitochondrial DNA deletion mutations and nuclear genome instability in human breast cancer", CANCER DETECTION AND PREVENTION, ELSEVIER SCIENCE, vol. 28, no. 2, 1 January 2004 (2004-01-01), pages 119-126, XP002524596, ISSN: 0361-090X, DOI: 10.1016/J.CDP.2003.11.008
- VERMA ET AL: "Application of mitochondrial genome information in cancer epidemiology", CLINICA CHIMICA ACTA, ELSEVIER BV, AMSTERDAM, NL, vol. 383, no. 1-2, 4 July 2007 (2007-07-04), pages 41-50, XP022142301, ISSN: 0009-8981, DOI: 10.1016/J.CCA.2007.04.018
- DATABASE UniProt [Online] 1 March 2002 (2002-03-01), "NADH-ubiquinone oxidoreductase chain 3; EC=1.6.5.3;", XP002687301, retrieved from EBI accession no. UNIPROT:Q8WCW0 Database accession no. Q8WCW0
- MITA S. ET AL.: 'Recombination via flanking direct repeats is a major cause of large- scale deletions of human mitochondrial DNA.' NUCLEIC ACIDS RESEARCH vol. 18, no. 3, 1990, pages 561 - 567, XP008154366
- UCHIDA T. ET AL.: 'Down-regulation of mitochondrial gene expression by the anti- tumor arotinoid mofarotene (Ro 40-8757).' INTERNATIONAL JOURNAL OF CANCER vol. 58, no. 6, 1994, pages 891 - 897, XP008154368
- ZHU W. ET AL.: 'Large-scale mitochondrial DNA deletion mutations and nuclear genome instability in human breast cancer.' CANCER DETECTION AND PREVENTION vol. 28, no. 2, 2004, pages 119 - 126, XP002524596
- DAI J.G. ET AL.: 'Mitochondrial DNA 4977 BP deletion mutations in lung carcinoma.' INDIAN JOURNAL OF CANCER. vol. 43, no. 1, January 2006, pages 20 - 25, XP008154370

## Description

### FIELD OF THE INVENTION

The present invention relates to the field of mitochondrial genomics and proteomics. In one aspect, the invention relates to the identification and use of mitochondrial genome fusion transcripts and translation products, as well as probes that hybridize thereto.

### BACKGROUND OF THE INVENTION

### Mitochondrial Genome

The mitochondrial genome is a compact yet critical sequence of nucleic acids. Mitochondrial DNA, or "mtDNA", comprises a small genome of 16,569 nucleic acid base pairs (bp) (Anderson et al., 1981; Andrews et al., 1999) in contrast to the immense nuclear genome of 3.3 billion bp (haploid). Its genetic complement is substantially smaller than that of its nuclear cell mate (0.0005%). However, individual cells carry anywhere from 10³ to 10⁴ mitochondria depending on specific cellular functions (Singh and Modica-Napolitano 2002). Communication or chemical signalling routinely occurs between the nuclear and mitochondrial genomes (Sherratt et al., 1997). Moreover, specific nuclear components are responsible for the maintenance and integrity of mitochondrial sequences (Croteau et al., 1999). All mtDNA genomes in a given individual are identical due to the clonal expansion of mitochondria within the ovum, once fertilization has occurred. However mutagenic events can induce sequence diversity reflected as somatic mutations. These mutations may accumulate in different tissues throughout the body in a condition known as heteroplasmy.

### Mitochondrial Proteome

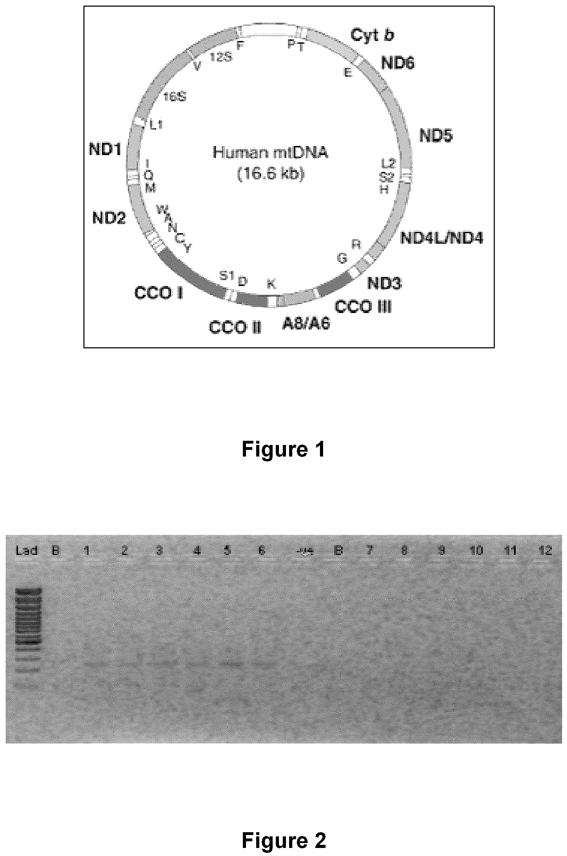

About 3,000 nuclear genes are required to construct, operate and maintain mitochondria, with only thirty-seven of these coded by the mitochondrial genome, indicating heavy mitochondrial dependence on nuclear loci. The mitochondrial genome codes for a complement of 24 genes, including 2 rRNAs and 22 tRNAs that ensure correct translation of the remaining 13 genes which are vital to electron transport (see Figure 1). The mitochondrial genome is dependent on seventy nuclear encoded proteins to accomplish the oxidation and reduction reactions necessary for this vital function, in addition to the thirteen polypeptides supplied by the mitochondrial genome. Both nuclear and mitochondrial proteins form complexes spanning the inner mitochondrial membrane and collectively generate 80-90% of the chemical fuel adenosine triphosphate, or ATP, required for cellular metabolism. In addition to energy production, mitochondria play a central role in other metabolic pathways as well. A critical function of the mitochondria is mediation of cell death, or apoptosis (see Green and Kroemer, 2005). Essentially, there are signal pathways which permeabilize the outer mitochondrial membrane, or in addition, the inner mitochondrial membrane as well. When particular mitochondrial proteins are released into the cytosol, non-reversible cell death is set in motion. This process highlights the multi-functional role that some mitochondrial proteins have. These multi-tasking proteins suggest that there are other mitochondrial proteins as well which may have alternate functions.

### Mitochondrial Fusion Transcriptome/Proteome

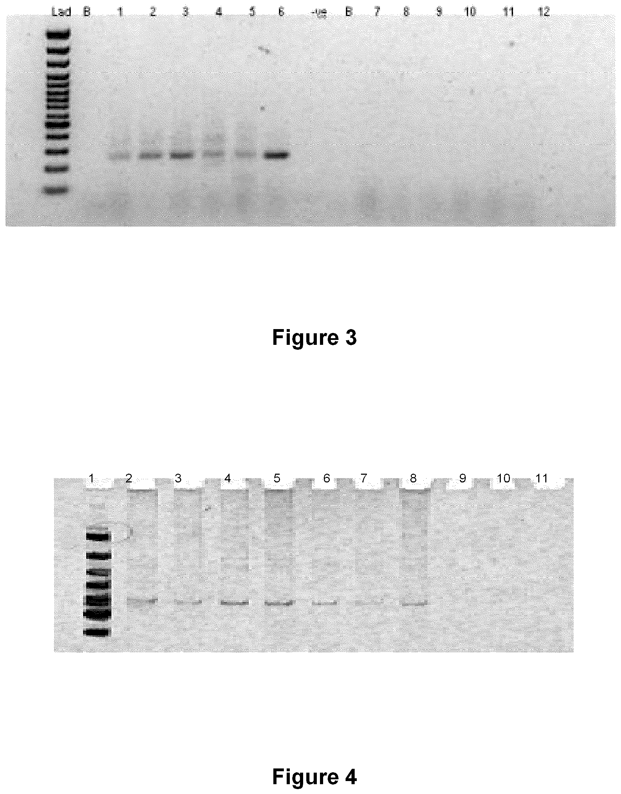

The mitochondrial genome is unusual in that it is a circular, intron-less DNA molecule. The genome is interspersed with repeat motifs which flank specific lengths of sequences. Sequences between these repeats are prone to deletion under circumstances which are not well understood. Given the number of repeats in the mitochondrial genome, there are many possible deletions. The best known example is the 4977 "common deletion." This deletion has been associated with several purported conditions and diseases and is thought to increase in frequency with aging (Dai et al., 2004; Ro et al., 2003; Barron et al., 2001; Lewis et al., 2000; Muller-Hocker, 1998; Porteous et al., 1998) (Figure 4). The current thinking in the field of mitochondrial genomics is that mitochondrial deletions are merely deleterious by-products of damage to the mitochondrial genome by such agents as reactive oxygen species and UVR. (Krishnan et al 2008, Nature Genetics). Further, though it is recognized that high levels of mtDNA deletions can have severe consequences on the cell's ability to produce energy in the form of ATP as a result of missing gene sequences necessary for cellular respiration, it is not anticipated that these deleted mitochondrial molecules may be a component of downstream pathways, have an intended functional role, and possibly may be more aptly viewed as alternate natural forms of the recognized genes of the mitochondria.

The sequence dynamics of mtDNA are important diagnostic tools. Mutations in mtDNA are often preliminary indicators of developing disease. For example, it has been demonstrated that point mutations in the mitochondrial genome are characteristic of tumour foci in the prostate. This trend also extends to normal appearing tissue both adjacent to and distant from tumour tissue (Parr et al. 2006). This suggests that mitochondrial mutations occur early in the malignant transformation pathway.

For example, the frequency of a 3.4kb mitochondrial deletion has excellent utility in discriminating between benign and malignant prostate tissues (Maki et al. 2008). Furthermore, an investigation of the disease associated deletions and the novel sequences, created through reclosure of the molecule identifies many open reading frames, suggesting the possibility of unique mitochondrial fusion proteins.

Mitochondrial fusion transcripts have been reported previously in the literature, first in soybeans (Morgens et al. 1984) and then later in two patients with Kearns-Sayre Syndrome, a rare neuromuscular disorder (Nakase et al 1990). Importantly, these transcripts were not found to have (or investigated regarding) association with any human cancers.

### Nuclear Fusion Proteome

There is important nuclear precedence for fusion proteins and their resulting effects on cancer. Nuclear *MLL* gene partner translocations are well established in correlation with high risk acute leukemia and therapy-related acute myeloid leukemias following treatment with agents that target topoisomerase II (Libura et al., 2005). Currently, around 50 translocations of the human *MLL* gene are known to be associated with these cancers (Meyer et al., 2005). Break points for these mutations, whether partial tandem duplications or translocations, for the majority of these events, occur within nuclear specific repetitive motifs such as *Alu* I. Most of these mutations are reciprocal translocations (84%) and include about 40 different genes (Libura et al. 2005).

There are known functional chimeric proteins created from some of these rearrangements which affect the course of malignant disease. For example, murine cells which express the protein from *MLL-ENL* accelerate the prevalence of chromosome abnormalities in cells which survive exposure to etoposide (Eguchi et al., 2006). Of particular interest is *MLL-SMAP1* and the reciprocal *SMAP1-MLL. SMAP1* binds calcium and as such participates in cell signalling and trafficking.

Mitochondrial fusion proteins may be assumed to have similar attributes to nuclear fusion proteins, especially since mitochondria and mitochondrial proteins play similar roles in signalling and apoptosis.

### SUMMARY OF THE INVENTION

An object of the present invention to provide aberrant mitochondrial DNA, associated fusion transcripts and translation products and hybridization probes therefor.

In accordance with an aspect of the invention, there is provided an isolated mitochondrial fusion transcript associated with cancer.

In accordance with another aspect of the invention, there is provided an isolated mtDNA encoding a fusion transcript of the invention.

In accordance with another aspect of the invention, there is provided a hybridization probe having a nucleic acid sequence complementary to at least a portion of a mitochondrial fusion transcript or an mtDNA of the invention.

In accordance with another aspect of the invention, there is provided a method of detecting a cancer in a mammal, the method comprising assaying a tissue sample from the mammal for the presence of at least one mitochondrial fusion transcript associated with cancer by hybridizing the sample with at least one hybridization probe having a nucleic acid sequence complementary to at least a portion of a mitochondrial fusion transcript according to the invention.

In accordance with another aspect of the invention, there is provided a method of detecting a cancer in a mammal, the method comprising assaying a tissue sample from the mammal for the presence of at least one aberrant mtDNA associated with cancer by hybridizing the sample with at least one hybridization probe having a nucleic acid sequence complementary to at least a portion of an mtDNA according to the invention.

In accordance with another aspect of the invention, there is provided a kit for conducting an assay for detecting the presence of a cancer in a mammal, said kit comprising at least one hybridization probe complementary to at least a portion of a fusion transcript or an mtDNA of the invention.

In accordance with another aspect of the invention, there is provided a mitochondrial fusion protein, the protein having an amino acid sequence resulting from the translation of a mitochondrial fusion transcript of the invention.

In accordance with another aspect of the invention, there is provided a method of detecting a cancer in a mammal, the method comprising assaying a tissue sample from the mammal for the presence of at least one mitochondrial fusion protein, the protein having an amino acid sequence resulting from the translation of a mitochondrial fusion transcript according to the invention.

### BRIEF DESCRIPTION OF THE DRAWINGS

The embodiments of the invention will now be described by way of example only with reference to the appended drawings wherein:
Figure 1 is an illustration showing mitochondrial protein coding genes.
Figure 2 shows polyadenalated fusion transcripts in prostate samples invoked by the loss of the 3.4kb deletion.
Figure 3 shows polyadenalated fusion transcripts in prostate samples invoked by the loss of the 4977kb common deletion.
Figure 4 shows polyadenalated fusion transcripts in breast samples invoked by the loss of the 3.4 kb segment from the mtgenome.
Figures 5a and 5b show an example of a mitochondrial DNA region before and after splicing of genes.
Figures 6a to 6g illustrate the results for transcripts 2, 3, 8, 9, 10, 11 and 12 of the disclosure in the identification of colorectal cancer tumours.
Figures 7a to 7d illustrate the results for transcripts 6, 8, 10 and 20 of the disclosure in the identification of lung cancer tumours.
Figures 8a to 8j illustrate the results for transcripts 6, 10, 11, 14, 15, 16 and 20 of the disclosure in the identification of melanomas.
Figures 9a to 9h illustrate the results for transcripts 1, 2, 3, 6, 11, 12, 15 and 20 of the disclosure in the identification of ovarian cancer.
Figures 10 to 18 illustrate the results for transcripts 2, 3, 4, 11, 12, 13, 15, 16 and 20 of the disclosure in the identification of testicular cancer.
Figure 19 illustrates the SDS PAGE gel of cytosolic and mitochondrial fractions of RWPE1 and WPE1-NA22 celll lines conducted during the fusion protein discovery phase.
Figure 20a illustrates the identified protein of fusion transcript P0026 based on the peptides ILYMTDEVNDPSLTIK and STPYECGFDPMSP.
Figure 20b illustrates the wild-type CO2 protein identified in mitochondrial NA22 cell line gel slice 5 of Figure 19 after searching the Human (SwissProt) database.
Figure 21a illustrates the identified protein of fusion transcript P0062 based on the peptides KGPNVVGPYGLLQPFADAMK, YDQLMHLLWK and LITTQQWLIK.
Figure 21b illustrates the identified peptides of ND1 identified in gel slice 5 of Figure 19 after searching the Human (SwissProt) database.
Figure 22 illustrates the identified protein of fusion transcript P0064 based on the peptides KGPNVVGPYGLLQPFADAMK and WAIIEEFTK.
Figure 23a illustrates the identified protein of fusion transcript P0176 based on the peptides KGPNVVGPYGLLQPFADAMK, VFSWLATLHGSNMK and VLMVEEPSMNLEWLYGCPPPYHTFEEPVYMK.
Figure 23b illustrates the wild-type CO1 protein identified in mitochondrial NA22 cell line gel slice 4 of Figure 19 after searching the Human (SwissProt) database.
Figures 24a to 24d illustrate the results of quantitative measurements of fusion transcripts P0026, P0062, P0064 and P0176, respectively.

### DETAILED DESCRIPTION OF THE INVENTION

The present invention provides novel mitochondrial fusion transcripts, the parent mutated mtDNA molecules, and the resulting translation products that are useful for predicting, diagnosing and/or monitoring cancer. The invention further provides hybridization probes for the detection of fusion transcripts and associated mtDNA molecules and the use of such probes.

### Definitions

Unless defined otherwise, all technical and scientific terms used herein have the same meaning as commonly understood by one of ordinary skill in the art to which this invention belongs.

The terms "comprise", "comprises", "comprised" or "comprising" may be used in the present description. As used herein (including the specification and/or the claims), these terms are to be interpreted as specifying the presence of the stated features, integers, steps or components, but not as precluding the presence of one or more other feature, integer, step, component or a group thereof as would be apparent to persons having ordinary skill in the relevant art.

As used herein, "aberration" or "mutation" encompasses any modification in the wild type mitochondrial DNA sequence that results in a fusion transcript and includes, without limitation, insertions, translocations, deletions, duplications, recombinations, rearrangements or combinations thereof.

As defined herein, "biological sample" refers to a tissue or bodily fluid containing cells from which a molecule of interest can be obtained. For example, the biological sample can be derived from tissue such as prostate, breast, colorectal, lung and skin, or from blood, saliva, cerebral spinal fluid, sputa, urine, mucous, synovial fluid, peritoneal fluid, amniotic fluid and the like. The biological sample may be a surgical specimen or a biopsy specimen. The biological sample can be used either directly as obtained from the source or following a pre-treatment to modify the character of the sample. Thus, the biological sample can be pre-treated prior to use by, for example, preparing plasma or serum from blood, disrupting cells, preparing liquids from solid materials, diluting viscous fluids, filtering liquids, distilling liquids, concentrating liquids, inactivating interfering components, adding reagents, and the like.

A "continuous" transcript is a fusion transcript that keeps the reading frame from the beginning to the end of both spliced genes. An "end" transcript is a fusion transcript that results in a premature termination codon before the original termination codon of a second spliced gene.

As used herein, "mitochondrial DNA" or "mtDNA" is DNA present in mitochondria.

As used herein, the expression "mitochondrial fusion protein" or "fusion protein" refers to a peptide product produced by the transcription and translation of a mutated mitochondrial DNA, wherein such mutations comprise deletions or other "large-scale" mitochondrial DNA rearrangements. In addition, or alternatively, an in-frame protein may be translated from alternate initiation and termination codons within that sequence.

As used herein, the expression "mitochondrial fusion transcript" or "fusion transcript" refers to an RNA transcription product produced as a result of the transcription of a mutated mitochondrial DNA sequence wherein such mutations may comprise mitochondrial deletions and other large-scale mitochondrial DNA rearrangements.

As used herein, the expression "mitochondrial translation product" or "translation product" refers to any amino acid chain derived from a mitochondrial fusion transcript including peptides, polypeptides and proteins. It will be understood that "mitochondrial translation products" comprise "fusion proteins", as defined above.

### Computer Analysis and Sequence Targetting

As discussed above, mitochondrial fusion transcripts have been reported in soybeans (Morgens et al. 1984) and in humans suffering from a rare neuromuscular disorder (Nakase et al 1990). Fusion transcripts associated with human cancer have not, however, been described.

Using the knowledge gained from mapping the large-scale deletions of the human mitochondrial genome associated with cancer, the observation of high frequencies of these deletions, and the evidence in another organism and another disease type of trancriptionally active mutated mtDNA molecules, the present inventors hypothesized that such deletions may have importance beyond the DNA molecule and the damage and repair processes as it relates to cancer. To test this hypothesis computer analysis of the mitochondrial genome was conducted, specific for repeat elements, which suggested many potential deletion sites. Following this initial step of identifying unique repeats in the mitochondrial sequence having non-adjacent or non-tandem locations, a filter was then applied to identify those repeats that upon initiating a deletion event in the DNA molecule would then likely reclose or religate to produce a fused DNA sequence having an open reading frame (ORF) and thus capable of being transcribed by the mitochondrial transcription machinery. A subset of 18 of these molecules were then selected for targetting to investigate whether: they existed in the natural biological state of humans; they were polyadenylated and thus expected to proceed to protein synthesis; they had relevance to malignancy. Results from these investigations proved positive for all three queries and are described hereinafter.

### Genomic Mutations

Mitochondrial DNA (mtDNA) dynamics are an important diagnostic tool. Mutations in mtDNA are often preliminary indicators of developing disease and may act as biomarkers indicative of risk factors associated with disease onset. According to the present invention, mutations in the mitochondrial genome result in the generation of fusion transcripts associated with cancer. Thus, the use of mtDNA encoding such transcripts and probes directed thereto for the detection, diagnosis and monitoring of cancer is provided.

One of skill in the art will appreciate that the mtDNA molecules for use in the methods of the present invention may be derived through the isolation of naturally-occurring mutants or may be based on the complementary sequence of any of the fusion transcripts described herein. Exemplary mtDNA sequences and fusion transcripts are disclosed in Applicant's co-pending U.S. Application No. 61/040,616 and published PCT application no. PCT/CA2009/000351 (published as WO 2009/117811).

### Detection of Mutant Genomic Sequences

Mutant mtDNA sequences according to the present invention may comprise any modification that results in the generation of a fusion transcript. Non-limiting examples of such modifications include insertions, translocations, deletions, duplications, recombinations, rearrangements or combinations thereof. While the modification or change can vary greatly in size from only a few bases to several kilobases, preferably the modification results in a substantive deletion or other large-scale genomic aberration.

Extraction of DNA to detect the presence of such mutations may take place using art-recognized methods, followed by amplification of all or a region of the mitochondrial genome, and may include sequencing of the mitochondrial genome, as described in Current Protocols in Molecular Biology.

The step of detecting the mutations can be selected from any technique known in the art. For example, analyzing mtDNA can comprise sequencing the mtDNA, amplifying mtDNA by PCR, Southern, Northern, Western South-Western blot hybridizations, denaturing HPLC, hybridization to microarrays, biochips or gene chips, molecular marker analysis, biosensors, melting temperature profiling or a combination of any of the above.

Any suitable means to sequence mitochondrial DNA may be used. Preferably, mtDNA is amplified by PCR prior to sequencing. The method of PCR is well known in the art and may be performed as described in Mullis and Faloona, 1987, Methods Enzymol., 155: 335. PCR products can be sequenced directly or cloned into a vector which is then placed into a bacterial host. Examples of DNA sequencing methods are found in Brumley, R. L. Jr. and Smith, L.M., 1991, Rapid DNA sequencing by horizontal ultrathin gel electrophoresis, Nucleic Acids Res. 19:4121-4126 and Luckey, J.A., et al, 1993, High speed DNA sequencing by capillary gel electrophoresis, Methods Enzymol. 218: 154-172. The combined use of PCR and sequencing of mtDNA is described in Hopgood, R., et al, 1992, Strategies for automated sequencing of human mtDNA directly from PCR products, Biotechniques 13:82-92 and Tanaka, M. et al, 1996, Automated sequencing of mtDNA, Methods Enzymol. 264: 407-421.

Methods of selecting appropriate sequences for preparing various primers are also known in the art. For example, the primer can be prepared using conventional solid-phase synthesis using commercially available equipment, such as that available from Applied Biosystems USA Inc. (Foster City, California), DuPont, (Wilmington, Del.), or Milligen (Bedford, Mass.).

According to an aspect of the invention, to determine candidate genomic sequences, a junction point of a sequence deletion is first identified. Sequence deletions are primarily identified by direct and indirect repetitive elements which flank the sequence to be deleted at the 5' and 3' end. The removal of a section of the nucleotides from the genome followed by the ligation of the genome results in the creation of a novel junction point.

Upon identification of the junction point, the nucleotides of the genes flanking the junction point are determined in order to identify a spliced gene. Typically the spliced gene comprises the initiation codon from the first gene and the termination codon of the second gene, and may be expressed as a continuous transcript, *i*.*e*. one that keeps the reading frame from the beginning to the end of both spliced genes. Some known mitochondrial deletions discovered to have an open reading frame (ORF) when the rearranged sequences are rejoined at the splice site are provided in Table 1.

Exemplary mtDNA molecules for use in the methods of the present disclosure are provided below. These mtDNAs are based on modifications of the known mitochondrial genome (SEQ ID NO: 1) and have been assigned a fusion or "FUS" designation, wherein A:B represents the junction point between the last mitochondrial nucleotide of the first spliced gene and the first mitochondrial nucleotide of the second spliced gene. The identification of the spliced genes is provided in parentheses followed by the corresponding sequence identifier. Where provided below, (AltMet) and (OrigMet) refer to alternate and original translation start sites, respectively.
FUS 8469:13447 (AltMet) (ATP synthase F0 subunit 8 to NADH dehydrogenase subunit) (SEQ ID No: 2)
FUS 10744:14124 (NADH dehydrogenase subunit 4L (ND4L) to NADH dehydrogenase subunit 5 (ND5)) (SEQ ID No: 3)
FUS 7974:15496 (Cytochrome c oxidase subunit II (COII) to Cytochrome b (Cytb)) (SEQ ID No: 4)
FUS 7992:15730 (Cytochrome c oxidase subunit II (COII) to Cytochrome b (Cytb)) (SEQ ID No: 5)
FUS 8210:15339 (Cytochrome c oxidase subunit II (COII) to Cytochrome b (Cytb)) (SEQ ID No: 6)
FUS 8828:14896 (ATP synthase F0 subunit 6 (ATPase6) to Cytochrome b (Cytb)) (SEQ ID No: 7)
FUS 10665:14856 (NADH dehydrogenase subunit 4L (ND4L) to Cytochrome b (Cytb)) (SEQ ID No: 8)
FUS 6075:13799 (Cytochrome c oxidase subunit I (COI) to NADH de hydrogenase subunit 5 (ND5)) (SEQ ID No: 9)
FUS 6325:13989 (Cytochrome c oxidase subunit I (COI) to NADH dehydrogenase subunit 5 (ND5)) (SEQ ID No: 10)
FUS 7438:13476 (Cytochrome c oxidase subunit I (COI) to NADH dehydrogenase subunit 5 (ND5)) (SEQ ID No: 11)
FUS 7775:13532 (Cytochrome c oxidase subunit II (COII) to NADH dehydrogenase subunit 5 (ND5)) (SEQ ID No: 12)
FUS 8213:13991 (Cytochrome c oxidase subunit II (COII) to NADH dehydrogenase subunit 5 (ND5)) (SEQ ID No: 13)
FUS 9191:12909 (ATP synthase F0 subunit 6 (ATPase6) to NADH dehydrogenase subunit 5 (ND5)) (SEQ ID No: 14)
FUS 9574:12972 (Cytochrome c oxidase subunit III (COIII) to NADH dehydrogenase subunit 5 (ND5)) (SEQ ID No: 15)
FUS 10367:12829 (NADH dehydrogenase subunit 3 (ND3) to NADH dehydrogenase subunit 5 (ND5)) (SEQ ID No: 16)
FUS 11232:13980 (NADH dehydrogenase subunit 4 (ND4) to NADH dehydrogenase subunit 5 (ND5) (SEQ ID No: 17)
FUS 8469:13447 (OrigMet) (ATP synthase F0 subunit 8 to NADH dehydrogenase subunit) (SEQ ID No: 18)
FUS 9144:13816 ((ATP synthase F0 subunit 6 (ATPase6) to NADH dehydrogenase subunit 5 (ND5)) (SEQ ID No: 54)

The present disclosure also provides the use of variants or fragments of these sequences for predicting, diagnosing and/or monitoring cancer.

"Variant", as used herein, refers to a nucleic acid differing from an mtDNA sequence of the present invention, but retaining essential properties thereof. Generally, variants are overall closely similar, and, in many regions, identical to a select mtDNA sequence. Specifically, the variants of the present invention comprise at least one of the nucleotides of the junction point of the spliced genes, and may further comprise one or more nucleotides adjacent thereto. In one embodiment of the disclosure, the variant sequence is at least 80%, 85%, 90%, 95%, 96%, 97%, 98% or 99% identical to any one of the mtDNA sequences of the invention, or the complementary strand thereto.

In the present invention, "fragment" refers to a short nucleic acid sequence which is a portion of that contained in the disclosed genomic sequences, or the complementary strand thereto. This portion includes at least one of the nucleotides comprising the junction point of the spliced genes, and may further comprise one or more nucleotides adjacent thereto. The fragments of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A fragment "at least 20 nt in length," for example, is intended to include 20 or more contiguous bases of any one of the mtDNA sequences listed above. In this context "about" includes the particularly recited value, a value larger or smaller by several (5, 4, 3, 2, or 1) nucleotides, at either terminus or at both termini. These fragments have uses that include, but are not limited to, as diagnostic probes and primers as discussed herein. Of course, larger fragments (e.g., 50, 150, 500, 600, 2000 nucleotides) are also contemplated.

Thus, in specific embodiments of the disclosure, the mtDNA sequences are selected from the group consisting of:
SEQ ID NO: 2 (FUS 8469:13447; AltMet)
SEQ ID NO: 3 (FUS 10744:14124)
SEQ ID NO: 4 (FUS 7974:15496)
SEQ ID NO: 5 (FUS 7992:15730)
SEQ ID NO: 6 (FUS 8210:15339)
SEQ ID NO: 7 (FUS 8828:14896)
SEQ ID NO: 8 (FUS 10665:14856)
SEQ ID NO: 9 (FUS 6075:13799)
SEQ ID NO: 10 (FUS 6325:13989)
SEQ ID NO: 11 (FUS 7438:13476)
SEQ ID NO: 12 (FUS 7775:13532)
SEQ ID NO: 13 (FUS 8213:13991)
SEQ ID NO: 14 (FUS 9191:12909)
SEQ ID NO: 15 (FUS 9574:12972)
SEQ ID NO: 16 (FUS 10367:12829)
SEQ ID NO: 17 (FUS 11232:13980)
SEQ ID NO: 18 (FUS 8469:13447; OrigMet)
SEQ ID NO: 54 (FUS 9144:13816),
- and fragments or variants thereof.

### Probes

Another aspect of the invention is to provide a hybridization probe capable of recognizing an aberrant mtDNA sequence of the invention. As used herein, the term "probe" refers to an oligonucleotide which forms a duplex structure with a sequence in the target nucleic acid, due to complementarity of at least one sequence in the probe with a sequence in the target region. The probe may be labeled, according to methods known in the art.

Once aberrant mtDNA associated with particular disease is identified, hybridization of mtDNA to, for example, an array of oligonucleotides can be used to identify particular mutations, however, any known method of hybridization may be used.

As with the primers of the present invention, probes may be generated directly against exemplary mtDNA fusion molecules of the invention, or to a fragment or variant thereof. For instance, the sequences set forth in SEQ ID NOs: 2-18 and 54 and those disclosed in Table 1 can be used to design primers or probes that will detect a nucleic acid sequence comprising a fusion sequence of interest. As would be understood by those of skill in the art, primers or probes which hybridize to these nucleic acid molecules may do so under highly stringent hybridization conditions or lower stringency conditions, such conditions known to those skilled in the art and found, for example, in Current Protocols in Molecular Biology (John Wiley & Sons, New York (1989)), 6.3.1-6.3.6.

In specific embodiments of the invention, the probes of the invention contain a sequence complementary to at least a portion of the aberrant mtDNA comprising the junction point of the spliced genes. This portion includes at least one of the nucleotides involved in the junction point A:B, and may further comprise one or more nucleotides adjacent thereto. In this regard, the present invention encompasses any suitable targeting mechanism that will select an mtDNA molecule using the nucleotides involved and/or adjacent to the junction point A:B.

Various types of probes known in the art are contemplated by the present invention. For example, the probe may be a hybridization probe, the binding of which to a target nucleotide sequence can be detected using a general DNA binding dye such as ethidium bromide, SYBR^{®} Green, SYBR^{®} Gold and the like. Alternatively, the probe can incorporate one or more detectable labels. Detectable labels are molecules or moieties a property or characteristic of which can be detected directly or indirectly and are chosen such that the ability of the probe to hybridize with its target sequence is not affected. Methods of labelling nucleic acid sequences are well-known in the art (see, for example, Ausubel et al., (1997 & updates) Current Protocols in Molecular Biology, Wiley & Sons, New York).

Labels suitable for use with the probes of the present invention include those that can be directly detected, such as radioisotopes, fluorophores, chemiluminophores, enzymes, colloidal particles, fluorescent microparticles, and the like. One skilled in the art will understand that directly detectable labels may require additional components, such as substrates, triggering reagents, light, and the like to enable detection of the label. The present invention also contemplates the use of labels that are detected indirectly.

The probes of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A probe of "at least 20 nt in length," for example, is intended to include 20 or more contiguous bases that are complementary to an mtDNA sequence of the invention. Of course, larger probes (e.g., 50, 150, 500, 600, 2000 nucleotides) may be preferable.

The probes of the invention will also hybridize to nucleic acid molecules in biological samples, thereby enabling the methods of the invention. Accordingly, in one aspect of the invention, there is provided a hybridization probe for use in the detection of cancer, wherein the probe is complementary to at least a portion of an aberrant mtDNA molecule. In another aspect the present invention provides probes and a use of (or a method of using) such probes for the detection of colorectal cancer, lung cancer, breast cancer, ovarian cancer, testicular, cancer, prostate cancer and/or melanoma skin cancer.

### Assays

Measuring the level of aberrant mtDNA in a biological sample can determine the presence of one or more cancers in a subject. The present invention, therefore, encompasses methods for predicting, diagnosing or monitoring cancer, comprising obtaining one or more biological samples, extracting mtDNA from the samples, and assaying the samples for aberrant mtDNA by: quantifying the amount of one or more aberrant mtDNA sequences in the sample and comparing the quantity detected with a reference value. As would be understood by those of skill in the art, the reference value is based on whether the method seeks to predict, diagnose or monitor cancer. Accordingly, the reference value may relate to mtDNA data collected from one or more known non-cancerous biological samples, from one or more known cancerous biological samples, and/or from one or more biological samples taken over time.

In one aspect, the invention provides a method of detecting cancer in a mammal, the method comprising assaying a tissue sample from the mammal for the presence of an aberrant mitochondrial DNA described above. The present invention also provides for methods comprising assaying a tissue sample from the mammal by hybridizing the sample with at least one hybridization probe. The probe may be generated against a mutant mitochondrial DNA sequence of the invention as described herein.

In another aspect, the invention provides a method as above, wherein the assay comprises:
a) conducting a hybridization reaction using at least one of the probes to allow the at least one probe to hybridize to a complementary aberrant mitochondrial DNA sequence;
b) quantifying the amount of the at least one aberrant mitochondrial DNA sequence in the sample by quantifying the amount of the mitochondrial DNA hybridized to the at least one probe; and,
c) comparing the amount of the mitochondrial DNA in the sample to at least one known reference value.

Also included in the present invention are methods for predicting, diagnosing or monitoring cancer comprising diagnostic imaging assays as described below. The diagnostic assays of the invention can be readily adapted for high-throughput. High-throughput assays provide the advantage of processing many samples simultaneously and significantly decrease the time required to screen a large number of samples. The present invention, therefore, contemplates the use of the nucleotides of the present invention in high-throughput screening or assays to detect and/or quantitate target nucleotide sequences in a plurality of test samples.

### Fusion Transcripts

The present invention further provides the identification of fusion transcripts and associated hybridization probes useful in methods for predicting, diagnosing and/or monitoring cancer. One of skill in the art will appreciate that such molecules may be derived through the isolation of naturally-occurring transcripts or, alternatively, by the recombinant expression of mtDNAs isolated according to the methods of the invention. As discussed, such mtDNAs typically comprise a spliced gene having the initiation codon from the first gene and the termination codon of the second gene. Accordingly, fusion transcripts derived therefrom comprise a junction point associated with the spliced genes.

### Detection of Fusion Transcripts

Naturally occurring fusion transcripts can be extracted from a biological sample and identified according to any suitable method known in the art, or may be conducted according to the methods described in the examples. In one embodiment of the invention, stable polyadenylated fusion transcripts are identified using Oligo(dT) primers that target transcripts with poly-A tails, followed by RT-PCR using primer pairs designed against the target transcript.

The following exemplary fusion transcripts were detected using such methods and found useful in predicting, diagnosing and/or monitoring cancer as indicated in the examples. Likewise, fusion transcripts derived from the ORF sequences identified in Table 1 may be useful in predicting, diagnosing and/or monitoring cancer.
SEQ ID NO: 19 (Transcript 1; 8469:13447; AltMet)
SEQ ID NO: 20 (Transcript 2;10744:14124)
SEQ ID NO: 21 (Transcript 3;7974:15496)
SEQ ID NO: 22 (Transcript 4;7992:15730)
SEQ ID NO: 23 (Transcript 5;8210:15339)
SEQ ID NO: 24 (Transcript 6;8828:14896)
SEQ ID NO: 25 (Transcript 7;10665:14856)
SEQ ID NO: 26 (Transcript 8;6075:13799)
SEQ ID NO: 27 (Transcript 9;6325:13989)
SEQ ID NO: 28 (Transcript 10;7438:13476)
SEQ ID NO: 29 (Transcript 11 ;7775:13532)
SEQ ID NO: 30 (Transcript 12;8213:13991)
SEQ ID NO: 31 (Transcript 14;9191:12909)
SEQ ID NO: 32 (Transcript 15;9574:12972)
SEQ ID NO: 33 (Transcript 16;10367:12829)
SEQ ID NO: 34 (Transcript 17;11232:13980)
SEQ ID NO: 35 (Transcript 20;8469:13447; OrigMet)
SEQ ID NO: 53 (Transcript 13; 9144:13816)

Fusion transcripts can also be produced by recombinant techniques known in the art. Typically this involves transformation (including transfection, transduction, or infection) of a suitable host cell with an expression vector comprising an mtDNA sequence of interest.

Variants or fragments of the fusion transcripts identified herein are also provided. Such sequences may adhere to the size limitations and percent identities described above with respect to genomic variants and fragments, or as determined suitable by a skilled technician.

### Probes

Once a fusion transcript has been characterized, primers or probes can be developed to target the transcript in a biological sample. Such primers and probes may be prepared using any known method (as described above) or as set out in the examples provided below. A probe may, for example, be generated for the fusion transcript, and detection technologies, such as QuantiGene 2.0™ by Panomics ™, used to detect the presence of the transcript in a sample. Primers and probes may be generated directly against exemplary fusion transcripts of the invention, or to a fragment or variant thereof. For instance, the sequences set forth in SEQ ID NOs: 19-35 and 53, as well as those disclosed in Table 1, can be used to design probes that will detect a nucleic acid sequence comprising a fusion sequence of interest.

As would be understood by those skilled in the art, probes designed to hybridize to the fusion transcripts of the invention contain a sequence complementary to at least a portion of the transcript expressing the junction point of the spliced genes. This portion includes at least one of the nucleotides complementary to the expressed junction point, and may further comprise one or more complementary nucleotides adjacent thereto. In this regard, the present invention encompasses any suitable targeting mechanism that will select a fusion transcript that uses the nucleotides involved and adjacent to the junction point of the spliced genes.

Various types of probes and methods of labelling known in the art are contemplated for the preparation of transcript probes. Such types and methods have been described above with respect to the detection of genomic sequences. The transcript probes of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A probe of "at least 20 nt in length," for example, is intended to include 20 or more contiguous bases that are complementary to an mtDNA sequence of the invention. Of course, larger probes (e.g., 50, 150, 500, 600, 2000 nucleotides) may be preferable.

In one aspect, the invention provides a hybridization probe for use in the detection of cancer, wherein the probe is complementary to at least a portion of a mitochondrial fusion transcript provided above.

In another aspect, the present invention provides probes and a use of (or a method of using) such probes for the detection of colorectal cancer, lung cancer, breast cancer, ovarian cancer, testicular cancer, prostate cancer or melanoma skin cancer.

### Assays

Measuring the level of mitochondrial fusion transcripts in a biological sample can determine the presence of one or more cancers in a subject. The present invention, therefore, provides methods for predicting, diagnosing or monitoring cancer, comprising obtaining one or more biological samples, extracting mitochondrial RNA from the samples, and assaying the samples for fusion transcripts by: quantifying the amount of one or more fusion transcripts in the sample and comparing the quantity detected with a reference value. As would be understood by those of skill in the art, the reference value is based on whether the method seeks to predict, diagnose or monitor cancer. Accordingly, the reference value may relate to transcript data collected from one or more known non-cancerous biological samples, from one or more known cancerous biological samples, and/or from one or more biological samples taken over time.

In one aspect, the invention provides a method of detecting a cancer in a mammal, the method comprising assaying a tissue sample from said mammal for the presence of at least one fusion transcript of the invention by hybridizing said sample with at least one hybridization probe having a nucleic acid sequence complementary to at least a portion of the mitochondrial fusion transcript.

In another aspect, the invention provides a method as above, wherein the assay comprises:
a) conducting a hybridization reaction using at least one of the above-noted probes to allow the at least one probe to hybridize to a complementary mitochondrial fusion transcript;
b) quantifying the amount of the at least one mitochondrial fusion transcript in the sample by quantifying the amount of the transcript hybridized to the at least one probe; and,
c) comparing the amount of the mitochondrial fusion transcript in the sample to at least one known reference value.

As discussed above, the diagnostic assays of the invention may also comprise diagnostic imaging methods as described herein and can be readily adapted for high-throughput. The present invention, therefore, contemplates the use of the fusion transcripts and associated probes of the present invention in high-throughput screening or assays to detect and/or quantitate target nucleotide sequences in a plurality of test samples.

### Translation Products

To date, mitochondrial fusion proteins have not been detected or isolated. However, the levels of mitochondrial fusion transcripts observed from the examples provided below and the indications that they are polyadenylated provide further evidence supporting the existence of such mitochondrial fusion proteins. Accordingly, the present invention provides the identification of fusion proteins for predicting, diagnosing, and/or monitoring of cancer.

Fusion proteins contemplated for use in the disclosed methods may be derived through the isolation of naturally-occurring polypeptides or through gene expression. Such polypeptides can be prepared by methods known in the art, such as purification from cell extracts or the use of recombinant techniques.

Putative protein sequences corresponding to transcripts 1-17 and 20 are provided below along with their respective sequence identifier. These, as well as the putative protein sequences corresponding to the deletion sequences disclosed in Table 1, are herein contemplated for use in the methods of the present invention.
SEQ ID NO: 36 (Transcripts 1)
SEQ ID NO: 37 (Transcript 2)
SEQ ID NO: 38 (Transcript 3)
SEQ ID NO: 39 (Transcript 4)
SEQ ID NO: 40 (Transcript 5)
SEQ ID NO: 41 (Transcript 6)
SEQ ID NO: 42 (Transcript 7)
SEQ ID NO: 43 (Transcript 8)
SEQ ID NO: 44 (Transcript 9)
SEQ ID NO: 45 (Transcript 10)
SEQ ID NO: 46 (Transcript 11)
SEQ ID NO: 47 (Transcript 12)
SEQ ID NO: 48 (Transcript 14)
SEQ ID NO: 49 (Transcript 15)
SEQ ID NO: 50 (Transcript 16)
SEQ ID NO: 51 (Transcript 17)
SEQ ID NO: 52 (Transcripts 20)
SEQ ID NO: 55 (Transcript 13)

### Detection of Fusion Proteins

Fusion proteins of the invention can be recovered and purified from a biological sample by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography, hydrophobic charge interaction chromatography and lectin chromatography. Most preferably, high performance liquid chromatography ("HPLC") is employed for purification.

Assaying fusion protein levels in a biological sample can occur using a variety of techniques. For example, protein expression in tissues can be studied with classical immunohistological methods (Jalkanen et al., J. Cell. Biol. 101:976-985 (1985); Jalkanen, M., et al., J. Cell. Biol. 105:3087-3096 (1987)). Other methods useful for detecting protein expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (<125> I, <121> I), carbon (<14> C), sulfur (<35> S), tritium (<3> H), indium (<112> In), and technetium (<99m> Tc), and fluorescent labels, such as fluorescein and rhodamine, and biotin.

The polypeptides of the invention can also be produced by recombinant techniques known in the art. Typically this involves transformation (including transfection, transduction, or infection) of a suitable host cell with an expression vector comprising a polynucleotide encoding the protein or polypeptide of interest.

### Antibodies

Protein specific antibodies for use in the assays of the present invention can be raised against the wild-type or expressed mitochondrial fusion proteins of the invention or an antigenic polypeptide fragment thereof, which may be presented together with a carrier protein, such as an albumin, to an animal system (such as rabbit or mouse) or, if it is long enough (at least about 25 amino acids), without a carrier.

As used herein, the term "antibody" (Ab) or "monoclonal antibody" (Mab) is meant to include intact molecules as well as antibody fragments, or antigen-binding fragments, thereof (such as, for example, Fab and F(ab')2 fragments) which are capable of specifically binding to, or having "specificity to", a mitochondrial fusion protein. Fab and F(ab')2 fragments lack the Fc fragment of intact antibody, clear more rapidly from the circulation, and may have less non-specific tissue binding of an intact antibody (Wahl et al., J. Nucl. Med. 24:316-325 (1983)). Thus, these fragments are preferred.

The antibodies of the present invention may be prepared by any of a variety of methods. For example, cells expressing the mitochondrial fusion protein or an antigenic fragment thereof can be administered to an animal in order to induce the production of sera containing polyclonal antibodies. In one method, a preparation of mitochondrial fusion protein is prepared and purified to render it substantially free of natural contaminants. Such a preparation is then introduced into an animal in order to produce polyclonal antisera of greater specific activity.

In a related method, the antibodies of the present invention are monoclonal antibodies. Such monoclonal antibodies can be prepared using hybridoma technology (Kohler et al., Nature 256:495 (1975); Kohler et al., Eur. J. Immunol. 6:511 (1976); Kohler et al., Eur. J. Immunol. 6:292 (1976); Hammerling et al., in: Monoclonal Antibodies and T-Cell Hybridomas, Elsevier, N.Y., (1981) pp. 563-681). In general, such procedures involve immunizing an animal (preferably a mouse) with a mitochondrial fusion protein antigen or with a mitochondrial fusion protein-expressing cell.

The present invention comprises immunological assays using antibodies or antigen-binding fragments having specificity to the fusion proteins described herein. Such immunological assays may be facilitated by kits containing the antibodies or antigen-binding fragments along with any other necessary reagents, test strips, materials, instructions etc.

### Assays

Measuring the level of a translation product such as a fusion protein in a biological sample can determine the presence of one or more cancers in a subject. The present invention, therefore, provides methods for predicting, diagnosing or monitoring cancer, comprising obtaining one or more biological samples, extracting mitochondrial fusion proteins from the samples, and assaying the samples for such molecules by: quantifying the amount of one or more molecules in the sample and comparing the quantity detected with a reference value. As would be understood by those of skill in the art, the reference value is based on whether the method seeks to predict, diagnose or monitor cancer. Accordingly, the reference value may relate to protein data collected from one or more known non-cancerous biological samples, from one or more known cancerous biological samples, and/or from one or more biological samples taken over time.

Techniques for quantifying proteins in a sample are well known in the art and include, for instance, classical immunohistological methods (Jalkanen et al., J. Cell. Biol. 101:976-985 (1985); Jalkanen, M., et al., J. Cell. Biol. 105:3087-3096 (1987)). Additional methods useful for detecting protein expression include immunoassays such as the radioimmunoassay (RIA) and the enzyme linked immunosorbent assay (ELISA).

In one aspect, the invention provides a method of detecting a cancer in a mammal, the method comprising assaying a tissue sample from the mammal for the presence of at least one mitochondrial fusion protein. In another aspect, the present invention provides for the detection of mitochondrial fusion proteins in the diagnosis of colorectal cancer, lung cancer, breast cancer, ovarian cancer, testicular cancer, prostate cancer and/or melanoma skin cancer.

### Diagnostic Imaging

### Diagnostic Devices

The disclosure includes diagnostic devices such as biochips, gene chips or microarrays used to diagnose specific diseases or identify specific mutations. All sequenced mitochondrial genomes are assessed to create a consenus structure of the base pair arrangement and are assigned a prohibiting index for proportion of base pair deletions and mutations associated with a particular disease or disorder. The diagnostic arrangement is then used to create biochips, gene chips, or microarrays.

Once sequences associated with particular diseases, disease states or disorders are identified, hybridization of a mitochondrial nucleotide sample to an array of oligonucleotides can be used to identify particular mutations. Any known method of hybridization may be used. Preferably, an array is used, which has oligonucleotide probes matching the wild type or mutated region, and a control probe. Commercially available arrays such as microarrays or gene chips are suitable. These arrays contain thousands of matched and control pairs of probes on a slide or microchip, and are capable of sequencing the entire genome very quickly. Review articles describing the use of microarrays in genome and DNA sequence analysis are available on-line.

### Microarray

Polynucleotide arrays provide a high throughput technique that can assay a large number of polynucleotides in a sample comprising one or more target nucleic acid sequences. The arrays of the disclosure are useful for gene expression analysis, diagnosis of disease and prognosis of disease (e.g., monitoring a patient's response to therapy, and the like).

Any combination of the polynucleotide sequences of mtDNA indicative of disease, or disease progression are used for the construction of a microarray.

The target nucleic acid samples to be analyzed using a microarray are derived from any human tissue or fluid which contains adequate amounts of mtDNA, as previously described. The target nucleic acid samples are contacted with polynucleotide members under hybridization conditions sufficient to produce a hybridization pattern of complementary nucleic acid members/target complexes.

### Construction of a Microarray

The microarray comprises a plurality of unique polynucleotides attached to one surface of a solid support, wherein each of the polynucleotides is attached to the surface of the solid support in a non-identical preselected region. Each associated sample on the array comprises a polynucleotide composition, of known identity, usually of known sequence, as described in greater detail below. Any conceivable substrate may be employed in the disclosure.

The array is constructed using any known means. The nucleic acid members may be produced using established techniques such as polymerase chain reaction (PCR) and reverse transcription (RT). These methods are similar to those currently known in the art (see e.g. PCR Strategies, Michael A. Innis (Editor), et al. (1995) and PCR: Introduction to Biotechniques Series, C. R. Newton, A. Graham (1997)). Amplified polynucleotides are purified by methods well known in the art (e.g., column purification). A polynucleotide is considered pure when it has been isolated so as to be substantially free of primers and incomplete products produced during the synthesis of the desired polynucleotide. Preferably, a purified polynucleotide will also be substantially free of contaminants which may hinder or otherwise mask the binding activity of the molecule.

In the arrays of the disclosure, the polynucleotide compositions are stably associated with the surface of a solid support, wherein the support may be a flexible or rigid solid support.

Any solid support to which a nucleic acid member may be attached may be used in the disclosure. Examples of suitable solid support materials include, but are not limited to, silicates such as glass and silica gel, cellulose and nitrocellulose papers, nylon, polystyrene, polymethacrylate, latex, rubber, and fluorocarbon resins such as TEFLON™.

The solid support material may be used in a wide variety of shapes including, but not limited to slides and beads. Slides provide several functional advantages and thus are a preferred form of solid support. Due to their flat surface, probe and hybridization reagents are minimized using glass slides. Slides also enable the targeted application of reagents, are easy to keep at a constant temperature, are easy to wash and facilitate the direct visualization of RNA and/or DNA immobilized on the solid support. Removal of RNA and/or DNA immobilized on the solid support is also facilitated using slides.

The particular material selected as the solid support is not essential to the disclosure, as long as it provides the described function. Normally, those who make or use the disclosure will select the best commercially available material based upon the economics of cost and availability, the expected application requirements of the final product, and the demands of the overall manufacturing process.

Numerous methods are used for attachment of the nucleic acid members of the invention to the substrate (a process referred as spotting). For example, polynucleotides are attached using the techniques of, for example U.S. Pat. No. 5,807,522, for teaching methods of polymer attachment. Alternatively, spotting is carried out using contact printing technology.

The amount of polynucleotide present in each composition will be sufficient to provide for adequate hybridization and detection of target polynucleotide sequences during the assay in which the array is employed. Generally, the amount of each nucleic acid member stably associated with the solid support of the array is at least about 0.1 ng, preferably at least about 0.5 ng and more preferably at least about 1 ng, where the amount may be as high as 1000 ng or higher, but will usually not exceed about 20 ng.

Control polynucleotides may be spotted on the array and used as target expression control polynucleotides and mismatch control nucleotides to monitor non-specific binding or cross-hybridization to a polynucleotide in the sample other than the target to which the probe is directed. Mismatch probes thus indicate whether a hybridization is specific or not. For example, if the target is present the perfectly matched probes should be consistently brighter than the mismatched probes. In addition, if all central mismatches are present, the mismatch probes are used to detect a mutation.

### Target Preparation

The targets for the microarrays, may be derived from one or more biological samples. It may be desirable to amplify the target nucleic acid sample prior to hybridization. One of skill in the art will appreciate that whatever amplification method is used, if a quantitative result is desired, care must be taken to use a method that maintains or controls for the relative frequencies of the amplified polynucleotides. Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. The high density array may then include probes specific to the internal standard for quantification of the amplified polynucleotide. Detailed protocols for quantitative PCR are provided in PCR Protocols, A Guide to Methods and Applications, Innis et al., Academic Press, Inc. N.Y., (1990). Other suitable amplification methods include, but are not limited to polymerase chain reaction (PCR) (Innis, et al., PCR Protocols. A guide to Methods and Application. Academic Press, Inc. San Diego, (1990)), ligase chain reaction (LCR) (see Wu and Wallace, Genomics, 4: 560 (1989), Landegren, et al., Science, 241: 1077 (1988) and Barringer, et al., Gene, 89: 117 (1990), transcription amplification (Kwoh, et al., Proc. Natl. Acad. Sci. USA, 86: 1173 (1989)), and self-sustained sequence replication (Guatelli, et al., Proc. Nat. Acad. Sci. USA, 87: 1874 (1990)).

The invention provides for labeled target or labeled probe as described above. For the microarrays, any analytically detectable marker that is attached to or incorporated into a molecule may be used in the invention. An analytically detectable marker refers to any molecule, moiety or atom which is analytically detected and quantified. Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads (e.g., Dynabeads™), fluorescent dyes (e.g., fluorescein, texas red, rhodamine, green fluorescent protein, and the like), radiolabels (e.g., 3H, 125I, 35S, 14C, or 32P), enzymes (e.g., horseradish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Pat. Nos. 3,817,837; 3,850,752; 3,939,350; 3,996,345; 4,277,437; 4,275,149; and 4,366,241.

Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label.

The labels may be incorporated by any of a number of means well known to those of skill in the art. However, in a preferred embodiment, the label is simultaneously incorporated during the amplification step in the preparation of the sample polynucleotides. Thus, for example, polymerase chain reaction (PCR) with labeled primers or labeled nucleotides will provide a labeled amplification product. In a preferred embodiment, transcription amplification, as described above, using a labeled nucleotide (e.g. fluorescein-labeled UTP and/or CTP) incorporates a label into the transcribed polynucleotides. Alternatively, a label may be added directly to the original polynucleotide sample (e.g., mRNA, polyA mRNA, cDNA, etc.) or to the amplification product after the amplification is completed. Means of attaching labels to polynucleotides are well known to those of skill in the art and include, for example nick translation or end-labeling (e.g. with a labeled RNA) by kinasing of the polynucleotide and subsequent attachment (ligation) of a polynucleotide linker joining the sample polynucleotide to a label (e.g., a fluorophore).

In a preferred embodiment, the target will include one or more control molecules which hybridize to control probes on the microarray to normalize signals generated from the microarray. Labeled normalization targets are polynucleotide sequences that are perfectly complementary to control oligonucleotides that are spotted onto the microarray as described above. The signals obtained from the normalization controls after hybridization provide a control for variations in hybridization conditions, label intensity, "reading" efficiency and other factors that may cause the signal of a perfect hybridization to vary between arrays.

### Image Acquisition and Data Analysis

Following hybridization and any washing step(s) and/or subsequent treatments of a conventional nature, the resultant hybridization pattern is detected. In detecting or visualizing the hybridization pattern, the intensity or signal value of the label will be not only be detected but quantified, by which is meant that the signal from each spot of the hybridization will be measured and compared to a unit value corresponding to the signal emitted by a known number of end labeled target polynucleotides to obtain a count or absolute value of the copy number of each end-labeled target that is hybridized to a particular spot on the array in the hybridization pattern.

Methods for analyzing the data collected from hybridization to arrays are well known in the art. For example, where detection of hybridization involves a fluorescent label, data analysis can include the steps of determining fluorescent intensity as a function of substrate position from the data collected, removing outliers, i.e., data deviating from a predetermined statistical distribution, and calculating the relative binding affinity of the test polynucleotides from the remaining data. The resulting data is displayed as an image with the intensity in each region varying according to the binding affinity between associated oligonucleotides and/or polynucleotides and the test polynucleotides.

### Diagnostic Tests

Following detection or visualization, the hybridization pattern is used to determine quantitative information about the genetic profile of the labeled target polynucleotide sample that was contacted with the array to generate the hybridization pattern, as well as the state or condition of the tissue, fluid, organs, cell, etc. from which the sample was derived. In this regard, the invention further provides for diagnostic tests for detecting cancer. The invention also provides for monitoring a patient's condition. According to the method of the invention, the presence of cancer is detected by obtaining a biological sample from a patient. A test sample comprising nucleic acid is prepared from the biological sample. The nucleic acid extracted from the sample is hybridized to an array comprising a solid substrate and a plurality of nucleic acid members, wherein each member is indicative of the presence of disease or a predisposition to cancer. According to this diagnostic test, hybridization of the sample comprising nucleic acid to one or more nucleic acid members on the array is indicative of cancer or a predisposition to a cancer.

### Diagnostic Monitoring

The methods of the present invention may further comprise the step of recommending a monitoring regime or course of therapy based on the outcome of one or more assays. This allows clinicians to practice personalized medicine; e.g. cancer therapy, by monitoring the progression of the patient's cancer (such as by recognizing when an initial or subsequent mutation occurs) or treatment (such as by recognizing when a mutation is stabilized).

With knowledge of the boundaries of the sequence variation in hand, the information can be used to diagnose a pre-cancerous condition or existing cancer condition. Further, by quantitating the amount of aberrant mtDNA in successive samples over time, the progression of a cancer condition can be monitored. For example, data provided by assaying the patient's tissues at one point in time to detect a first set of mutations from wild-type could be compared against data provided from a subsequent assay, to determine if changes in the aberration have occurred.

Where a mutation is found in an individual who has not yet developed symptoms of cancer, the mutation may be indicative of a genetic susceptibility to develop a cancer condition. A determination of susceptibility to disease or diagnosis of its presence can further be evaluated on a qualitative basis based on information concerning the prevalence, if any, of the cancer condition in the patient's family history and the presence of other risk factors, such as exposure to environmental factors and whether the patient's cells also carry a mutation of another sort.

### Biological Sample

The present invention provides for diagnostic tests which involve obtaining or collecting one or more biological samples. In the context of the present invention, "biological sample" refers to a tissue or bodily fluid containing cells from which mtDNA, mtRNA and translation products or fusion proteins can be obtained. For example, the biological sample can be derived from tissue including, but not limited to, skin, lung, breast, prostate, nervous, muscle, heart, stomach, colon, rectal tissue and the like; or from blood, saliva, cerebral spinal fluid, sputa, urine, mucous, synovial fluid, peritoneal fluid, amniotic fluid and the like. The biological sample may be obtained from a cancerous or non-cancerous tissue and may be, but is not limited to, a surgical specimen or a biopsy specimen.

The biological sample can be used either directly as obtained from the source or following a pre-treatment to modify the character of the sample. Thus, the biological sample can be pre-treated prior to use by, for example, preparing plasma or serum from blood, disrupting cells, preparing liquids from solid materials, diluting viscous fluids, filtering liquids, distilling liquids, concentrating liquids, inactivating interfering components, adding reagents, and the like.

One skilled in the art will understand that more than one sample type may be assayed at a single time (i.e. for the detection of more than one cancer). Furthermore, where a course of collections are required, for example, for the monitoring of cancer over time, a given sample may be diagnosed alone or together with other samples taken throughout a test period. In this regard, biological samples may be taken once only, or at regular intervals such as biweekly, monthly, semi-annually or annually.

### Kits

The present invention provides diagnostic/screening kits for detecting cancer in a clinical environment. Such kits may include one or more sampling means, in combination with one or more probes according to the present invention. Alternatively, or in addition thereto, the kit may comprise means for detecting a translation product of the invention.

The kits can optionally include reagents required to conduct a diagnostic assay, such as buffers, salts, detection reagents, and the like. Other components, such as buffers and solutions for the isolation and/or treatment of a biological sample, may also be included in the kit. One or more of the components of the kit may be lyophilised and the kit may further comprise reagents suitable for the reconstitution of the lyophilised components.

Where appropriate, the kit may also contain reaction vessels, mixing vessels and other components that facilitate the preparation of the test sample. The kit may also optionally include instructions for use, which may be provided in paper form or in computer-readable form, such as a disc, CD, DVD or the like.

In one embodiment of the invention there is provided a kit for diagnosing cancer comprising sampling means and a hybridization probe of the invention.

In another embodiment, the kits of the present invention may comprise an immunological assay. In such case, the kits may comprise antibodies or antigen-binding fragments having specificity towards the fusion proteins described herein. It will be understood that various other reagents, test strips etc. required for such immunological assay will be contained in the kits as will the required instructions to users.

### EXAMPLES

Various aspects of the invention will be described by illustration using the following examples. The examples provided herein serve only to illustrate certain specific embodiments of the invention and are not intended to limit the scope of the invention in any way.

### Example 1: Detection of Mitochondrial Fusion Transcripts

The mitochondrial 4977 "common deletion" and a 3.4kb deletion previously identified by the present Applicant in PCT application no. PCT/CA2007/001711 (published under number WO 2009/039601,) result in unique open reading frames having active transcripts as identified by oligo-dT selection in prostate tissue (Figures 2 and 3). Examination of breast tissue samples also reveals the presence of a stable polyadenylated fusion transcript resulting from the 3.4kb deletion (Figure 4).

### Reverse transcriptase-PCR protocol for deletion transcript detection

### RNA isolation cDNA synthesis

Total RNA was isolated from snap frozen prostate and breast tissue samples (both malignant and normal samples adjacent to tumours) using the Aurum™ Total RNA Fatty and Fibrous Tissue kit (Bio-Rad, Hercules, CA) following the manufacturer's instructions. Since in this experiment, genomic DNA contamination was to be avoided, a DNase I treatment step was included, using methods as commonly known in the art. RNA quantity and quality were determined with an ND-1000 spectrophotometer (NanoDrop® technologies). From a starting material of about 100g, total RNA concentrations varied from 100 - 1000ng/ul with a 260/280 ratio between 1.89 - 2.10. RNA concentrations were adjusted to 100ng/ul and 2ul of each template were used for first strand DNA synthesis with SuperScript™ First-Strand Synthesis System for RT-PCR (Invitrogen) following the manufacturer's instructions. In order to identify stable polyadenylated fusion transcripts, Oligo(dT) primers that target transcripts with poly-A tails were used.

### PCR

Real time PCR was performed using 5ul of each cDNA template with the iQ™ SYBR^{®} Green Supermix (Bio-Rad, Hercules, CA) on DNA Engine Opticon^{®} 2 Continuous Fluorescence Detection System (Bio-Rad, Hercules, CA). The primer pairs targeting the 4977bp deletion are; 8416F 5'- CCTTACACTATTCCTCATCAC- 3', 13637R 5'- TGACCTGTTAGGGTGAGAAG - 3', and those for the 3.4 kb deletion are; ND4LF 5'- TCGCTCACACCTCATATCCTC -3', ND5R 5'-TGTGATTAGGAGTAGGGTTAGG -3'. The reaction cocktail included: 2X SYBR^{®} Green Supermix (100mM KCL, 40mM Tris-HCl, pH 8.4, 0.4mM of each dNTP [dATP, dCTP, dGTP, and dTTP], iTaq™ DNA polymerase, 50 units/ml, 6mM MgCl₂, SYBR^{®} Green 1, 20nM flourescein, and stabilizers), 250nM each of primers, and ddH₂O. PCR cycling parameters were as follows; (1) 95°C for 2 min, (2) 95°C for 30 sec, (3) 55°C (for the 4977bp deletion) and 63°C (for the 3.4 kb deletion) for 30 sec , (4) 72°C for 45 sec, (5) plate read, followed by 39 cycles of steps 3 to 5, and final incubation at 4°C. Apart from cycling threshold and melting curve analysis, samples were run on agarose gels for specific visualization of amplification products (see Figures 2 to 4).

Figure 2 is an agarose gel showing polyadenalated fusion transcripts in prostate samples invoked by the loss of 3.4kb from the mitochondrial genome. Legend for Figure 2: B-blank, Lanes 1-6 transcripts detected in cDNA; lanes 7-12 no reverse transcriptase (RT) controls for samples in lanes 1-6.

Figure 3 shows polyadenalated fusion transcripts in prostate samples invoked by the loss of the 4977kb common deletion. Legend for Figure 3: B-blank, Lanes 1-6 transcripts detected in cDNA; lanes 7-12 no RT controls for samples in lanes 1-6.

Figure 4 shows polyadenalated fusion transcripts in breast samples invoked by the loss of 3.4kb from the mtgenome. Legend for Figure 4: Lanes 2-8 transcripts from breast cDNAs; lane 9 negative (water) control; lanes 10 and 11, negative, no RT, controls for samples in lanes 2 and 3.

These results demonstrate the existence of stable mitochondrial fusion transcripts.

### Example 2: Identification and Targetting of Fusion Products

Various hybridization probes were designed to detect, and further demonstrate the presence of novel transcripts resulting from mutated mitochondrial genomes, such as the 3.4kb deletion. For this purpose, a single-plex branched DNA platform for quantitative gene expression analysis (QuantiGene 2.0™, Panomics™) was utilized. The specific deletions and sequences listed in this example are based on their relative positions with the entire mtDNA genome, which is recited in SEQ ID NO: 1. The nucleic acid sequences of the four transcripts to which the probes were designed in this example are identified herein as follows: Transcript 1 (SEQ ID NO: 19), Transcript 2 (SEQ ID NO: 20), Transcript 3 (SEQ ID NO: 21) and Transcript 4 (SEQ ID NO: 22).

An example of a continuous transcript from the 3.4kb mitochondrial genome deletion occurs with the genes ND4L (NADH dehydrogenase subunit 4L) and ND5 (NADH dehydrogenase subunit 5). A probe having a complementary sequence to SEQ ID NO: 20, was used to detect transcript 2. The repetitive elements occur at positions 10745-10754 in ND4L and 14124-14133 in ND5.

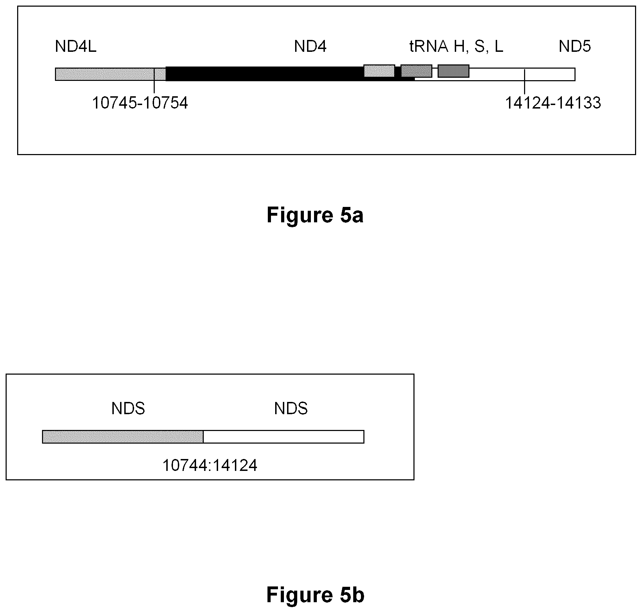

The 3.4kb deletion results in the removal of the 3' end of ND4L, the full ND4 gene, tRNA histidine, tRNA serine2, tRNA leucine2, and the majority of the 5' end of ND5 (see Figure 5a), resulting in a gene splice of ND4L and ND5 with a junction point of 10744(ND4L):14124(ND5) (Figure 5b).

By starting at the original initiation codon of the first gene, ND4L, the amino acid sequence was translated until a termination codon occurs. In this example the termination codon is the original termination codon of ND5. Therefore, despite splicing two genes together, the reading frame is kept intact resulting in a hypothetical or predicted transcript that is 100 amino acids (or 300 bp) in length. This fusion protein transcript product is identified herein as SEQ ID NO: 37. The nucleotide sequence (SEQ ID NO: 3) encoding such protein corresponds to the mitochondrial genome positions of 10470-10744:14124-14148. SEQ ID NO: 3 is the complementary DNA sequence to the RNA transcript (SEQ ID NO: 20) detected in the manner described above.

Similarly, transcript 1 is a fusion transcript between ATPase 8 and ND5 associated with positions 8469:13447 (SEQ ID NO: 19). Transcripts 3 and 4 (SEQ ID NO: 21 and SEQ ID NO: 22, respectively) are fusion transcripts between COII and Cytb associated with nucleotide positions 7974:15496 and 7992:15730 respectively. Table 3 provides a summary of the relationships between the various sequences used in this example. Table 3 includes the detected fusion transcript, the DNA sequence complementary to the fusion transcript detected and hypothetical translation products for each transcript.

### Example 3: Application to Prostate Cancer

Using the four fusion transcripts, i.e. transcripts 1 to 4, discussed above, two prostate tissue samples from one patient were analyzed to assess the quantitative difference of the novel predicted fusion transcripts. The results of the experiment are provided in Table 2 below, wherein "Homog 1" refers to the homogenate of frozen prostate tumour tissue from a patient and "Homog 2" refers to the homogenate of frozen normal prostate tissue adjacent to the tumour of the patient. These samples were processed according to the manufacturer's protocol (*QuantiGene*® *Sample Processing Kit for Fresh or Frozen Animal Tissues; and QuantiGene*® *2.0 Reagent System User Manual)* starting with 25.8 mg of Homog 1 and 28.9 mg of Homog 2 (the assay setup is shown in Tables 5a and 5b).

Clearly demonstrated is an increased presence of mitochondrial fusion transcripts in prostate cancer tissue compared to normal adjacent prostate tissue. The fusion transcript is present in the normal tissue, although at much lower levels. The relative luminescence units (RLU) generated by hybridization of a probe to a target transcript are directly proportional to the abundance of each transcript. Table 2 also indicates the coefficients of variation, CV, expressed as a percentage, of the readings taken for the samples. The CV comprises the Standard deviation divided by the average of the values. The significance of such stably transcribed mitochondrial gene products in cancer tissue has implications in disease evolution and progression.

### Example 4: Application to Breast Cancer

Using the same protocol from Example 3 but focusing only on Transcript 2, the novel fusion transcript associated with the 3.4kb mtgenome deletion, analyses were conducted on two samples of breast tumour tissue and two samples of tumour-free tissues adjacent to those tumours, as well as three samples of prostate tumour tissue, one sample comprising adjacent tumour-free tissue. Results for this example are provided in Table 4. The prostate tumour tissue sample having a corresponding normal tissue section demonstrated a similar pattern to the prostate sample analyzed in Example 3 in that the tumour tissue had approximately 2 times the amount of the fusion transcript than did the normal adjacent tissue. The breast tumour samples demonstrated a marked increase in the fusion transcript levels when compared to the adjacent non-tumour tissues. A 1:100 dilution of the homogenate was used for this analysis as it performed most reproducibly in the experiment cited in Example 3.

Thus, the above discussed results illustrate the application of the transcripts of the invention in the detection of tumours of both prostate and breast tissue.

### Example 5: Application to Colorectal Cancer

This study sought to determine the effectiveness of several transcripts of the disclosure in detecting colorectal cancer. A total of 19 samples were prepared comprising nine control (benign) tissue samples (samples 1 to 9) and ten tumour (malignant) tissue samples (samples 10 to 19). The samples were homogenized according to the manufacturer's recommendations (Quantigene® Sample Processing Kit for Fresh or Frozen Animal Tissues; and Quantigene 2.0 Reagent System User Manual). Seven target transcripts and one housekeeper transcript were prepared in the manner as outlined above in previous examples. The characteristics of the transcripts are summarized as follows:

**Table 7: Characteristics of Breast Cancer Transcripts**

| **Transcript ID** | **Junction Site** | **Gene Junction** |
|---|---|---|
| 2 | 10744:14124 | ND4L:ND5 |
| 3 | 7974:15496 | COII:Cytb |
| 10 | 7438:13476 | COI:ND5 |
| 11 | 7775:13532 | COII:ND5 |
| 12 | 8213:13991 | COII:ND5 |
| Peptidylpropyl isomerase B (PPIB) ("housekeeper") | N/A | N/A |

It is noted that transcripts 2 and 3 are the same as those discussed above with respect to Examples 3 and 4.

Homogenates were prepared using approximately 25mg of tissue from OCT blocks and diluted 1:1 for transcripts 2 and 4, and 1:8 for transcripts 10 and 11. The quantity of the transcripts was measured in Relative Luminenscence Units RLU on a Glomax™ Multi Detection System (Promega). All samples were assayed in triplicate for each transcript. Background measurements (no template) were done in triplicate as well. The analysis accounted for background by subtracting the lower limit from the RLU values for the samples. Input RNA was accounted for by using the formula log₂ a RLU - log₂ *h* RLU where a is the target fusion transcript and *h* is the housekeeper transcript.

The analysis of the data comprised the following steps:
a) Establish CV's (coefficients of variation) for triplicate assays; acceptable if ≤ 15%.
b) Establish average RLU value for triplicate assays of target fusion transcript(a) and housekeeper transcript (h).
c) Establish lower limit from triplicate value of background RLU (I).
d) Subtract lower limit (I) from (a).
e) Calculate log2 a RLU - log2 h RLU.

### Summary of Results:

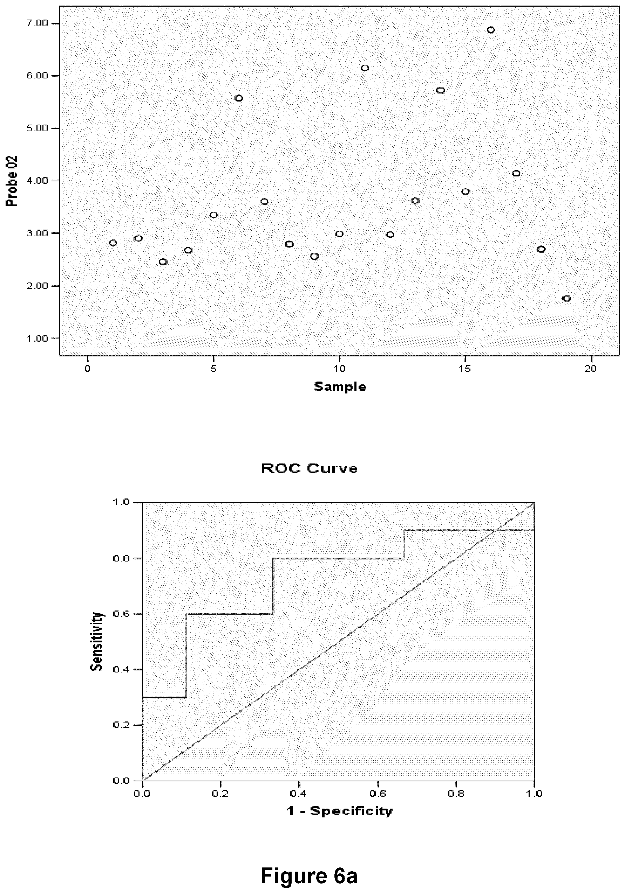

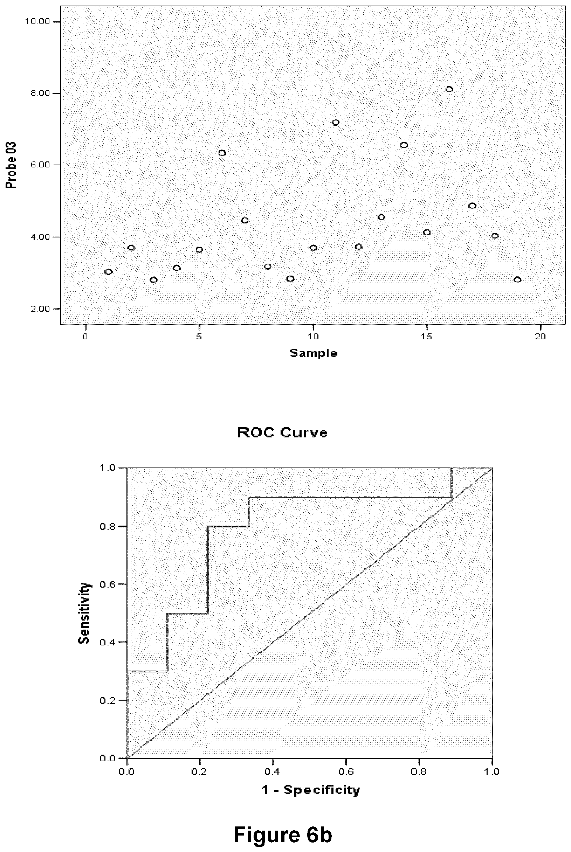

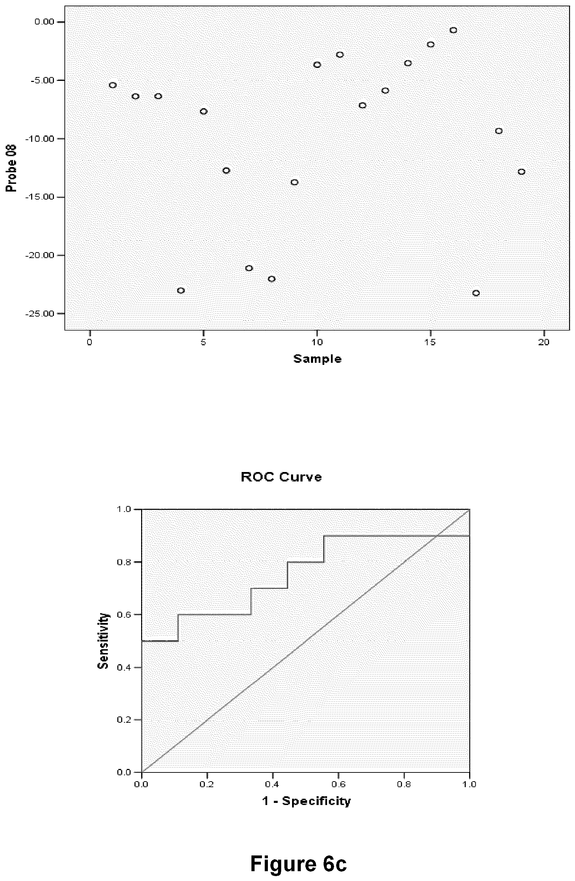

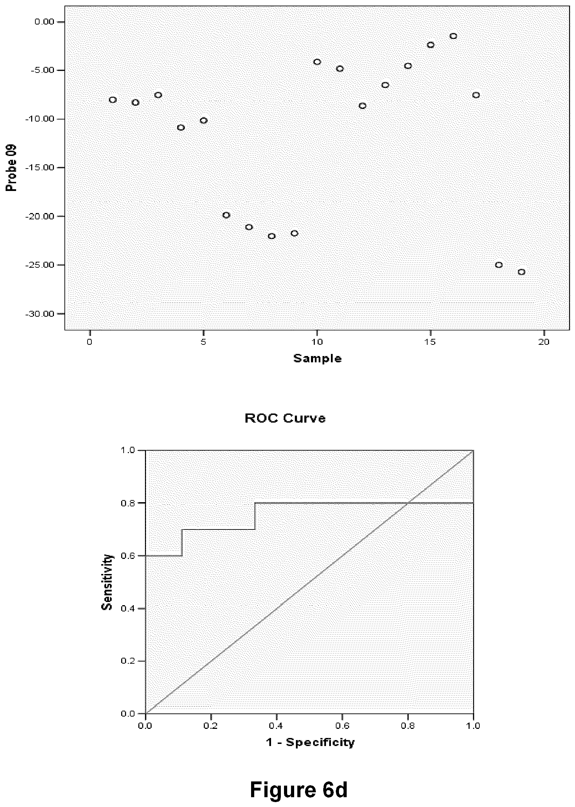

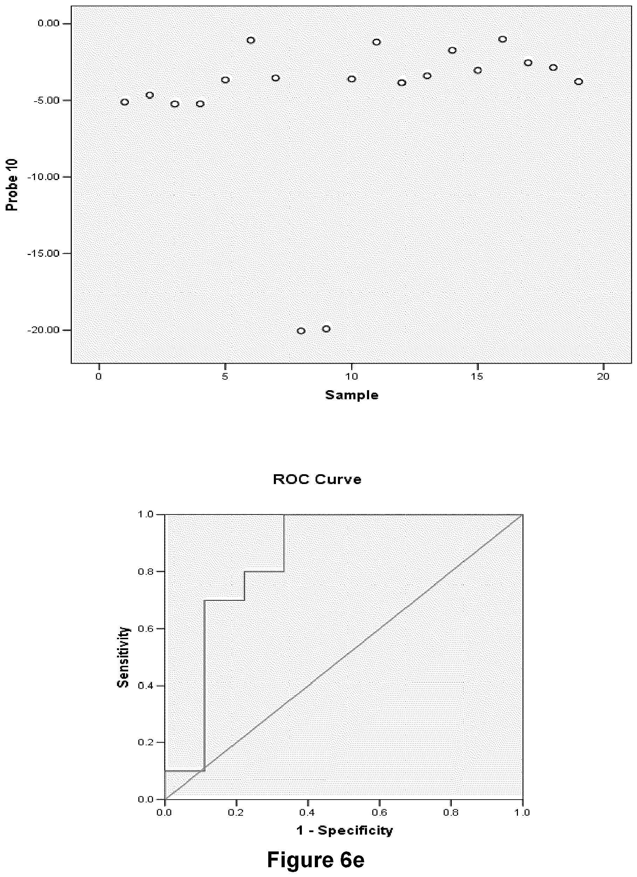

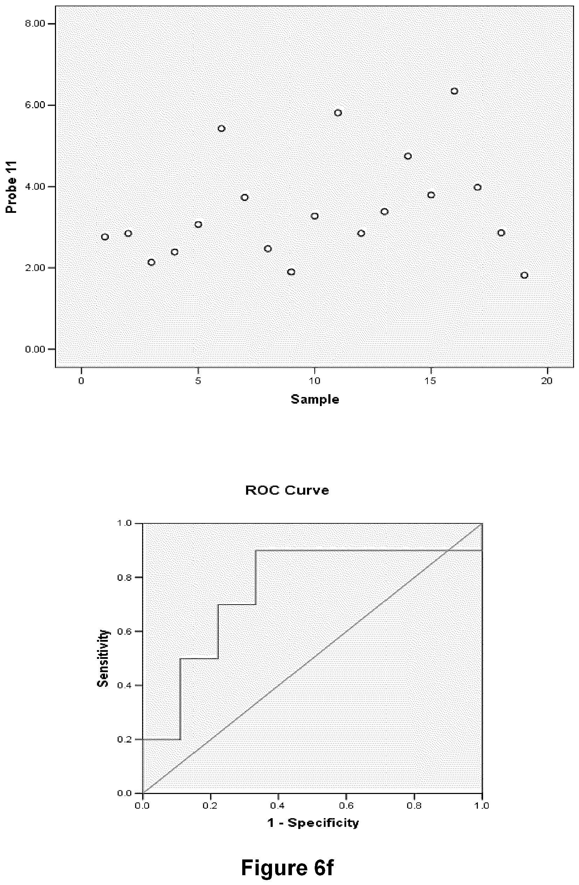

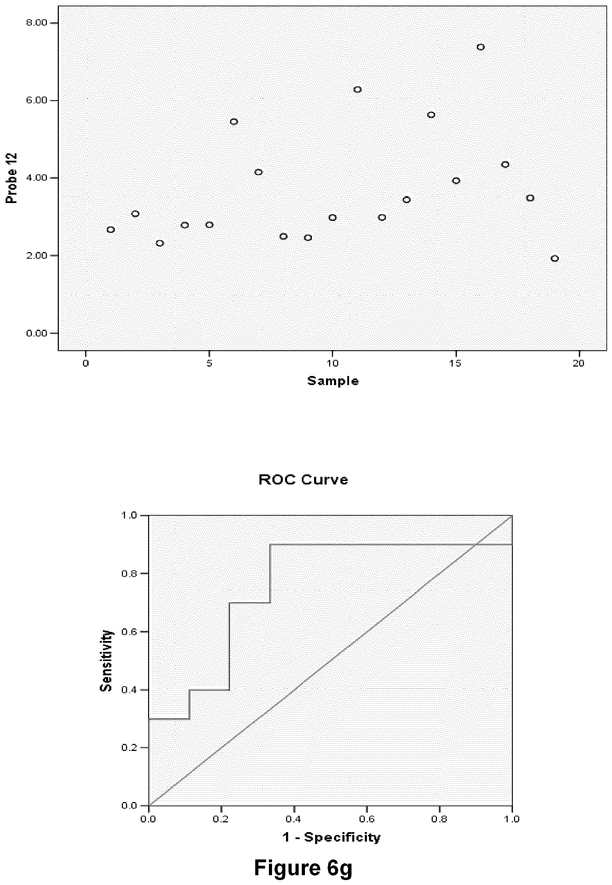

The results of the above analysis are illustrated in Figures 6a to 6g, which comprise plots of the log₂ *a* RLU - log₂ *h* RLU against sample number. Also illustrated are the respective ROC (Receiver Operating Characteristic) curves determined from the results for each transcript.

Transcript 2: There exists a statistically significant difference between the means (p<0.10) of the normal and malignant groups (p>0.09), using a cutoff value of 3.6129 as demonstrated by the ROC curve results in a sensitivity of 60% and specificity of 89% and the area under the curve is 0.73 indicating fair test accuracy. The threshold value chosen may be adjusted to increase either the specificity or sensitivity of the test for a particular application.

Transcript 3: There exists a statistically significant difference between the means (p<0.05) of the normal and malignant groups (p=0.03), using a cutoff value of 4.0813 as demonstrated by the ROC curve results in a sensitivity of 60% and specificity of 78% and the area under the curve is 0.79 indicating fair to good test accuracy. The threshold value chosen may be adjusted to increase either the specificity or sensitivity of the test for a particular application.

Transcript 8: There exists a statistically significant difference between the means (p<0.1) of the normal and malignant groups (p=0.06). Using a cutoff value of -6.0975 as demonstrated by the ROC curve results in a sensitivity of 60% and specificity of 89% and the area under the curve is 0.76 indicating fair test accuracy. The threshold value chosen may be adjusted to increase either the specificity or sensitivity of the test for a particular application.

Transcript 9: There exists a statistically significant difference between the means (p<0.1) of the normal and malignant groups (p=0.06). Using a cutoff value of -7.5555 as demonstrated by the ROC curve results in a sensitivity of 60% and specificity of 89% and the area under the curve is 0.76 indicating fair to good test accuracy. The threshold value chosen may be adjusted to increase either the specificity or sensitivity of the test for a particular application.

Transcript 10: There is a statistically significant difference between the means (p≤0.01) of the normal and malignant groups (p=0.01). Using a cutoff value of -3.8272as demonstrated by the ROC curve results in a sensitivity of 90% and specificity of 67% and the area under the curve is 0.84, indicating good test accuracy. The threshold value chosen may be adjusted to increase either the specificity or sensitivity of the test for a particular application.

Transcript 11: There exists a statistically significant difference between the means (p<0.1) of the normal and malignant groups (p=0.06), using a cutoff value of 3.1753 as demonstrated by the ROC curve results in a sensitivity of 70% and specificity of 78% and the area under the curve is 0.76 indicating fair to good test accuracy. The threshold value chosen may be adjusted to increase either the specificity or sensitivity of the test for a particular application.

Transcript 12: There exists a statistically significant difference between the means (p<0.1) of the normal and malignant groups (p=0.06), using a cut-off value of 3.2626 as demonstrated by the ROC curve results in a sensitivity of 70% and specificity of 78% and the area under the curve is 0.76 indicating fair to good test accuracy. The threshold value chosen may be adjusted to increase either the specificity or sensitivity of the test for a particular application.

### Conclusions:

The above results illustrate the utility of transcripts 2, 3, 8, 9, 10, 11, and 12 in the detection of colorectal cancer and in distinguishing malignant from normal colorectal tissue. As indicated above, transcripts 2 and 3 were also found to have utility in the detection of prostate cancer. Transcript 2 was also found to have utility in the detection of breast cancer. Transcript 11 was also found to have utility in the detection of melanoma skin cancer. Transcript 10 was also found to have utility in the detection of lung cancer and melanoma. Transcript 8 was also found to have utility in the detection of lung cancer. Any of the 7 transcripts listed may be used individually or in combination as a tool for the detection of characterization of colorectal cancer in a clinical setting.

### Example 6: Application to Lung Cancer

This study sought to determine the effectiveness of several transcripts of the disclosure in the detection of lung cancer. As in Example 5, nine control (benign) tissue samples (samples 1 to 9) and ten tumour (malignant) tissue samples (samples 10 to 19) were homogenized according to the manufacturer's recommendations (Quantigene® Sample Processing Kit for Fresh or Frozen Animal Tissues; and Quantigene 2.0 Reagent System User Manual). Homogenates were diluted 1:8 and the quantity of 4 target transcripts and 1 housekeeper transcript was measured in Relative Luminenscence Units RLU on a Glomax™ Multi Detection System (Promega). All samples were assayed in triplicate for each transcript. Background measurements (no template) were done in triplicate as well.

The following transcripts were prepared for this example:

**Table 8: Characteristics of Lung Cancer Transcripts**

| **Transcript ID** | **Junction Site** | **Gene Junction** |
|---|---|---|
| 6 | 8828:14896 | ATPase6:Cytb |
| 8 | 6075:13799 | COI:ND5 |
| 10 | 7438:13476 | COI:ND5 |
| 20 | 8469:13447 | ATPase8:ND5 |
| Peptidylpropyl isomerase B (PPIB) ("housekeeper") | N/A | N/A |

The tissue samples used in this example had the following characteristics:

**Table 9: Characteristics of Lung Cancer Samples**

| **Sample** | **Malignant** | **Comments (source of tissue)** |
|---|---|---|
| 1 | NO | interstitial lung disease |
| 2 | NO | emphysema |
| 3 | NO | aneurysm |
| 4 | NO | bronchopneumonia, COPD |
| 5 | NO | malignant neoplasm in liver, origin unknown, calcified granulomas in lung |
| 6 | NO | 12 hours post mortem, mild emphysema |
| 7 | NO | 12 hours post mortem, large B cell lymphoma, pulmonary edema, pneumonia |
| 8 | NO | pneumonia, edema, alveolar damage |
| 9 | NO | congestion and edema |
| 10 | YES | adenocarcinoma, non-small cell |
| 11 | YES | small cell |
| 12 | YES | squamous cell carcinoma, NSC, emphysema |
| 13 | YES | adenocarcinoma, lung cancer, nsc, metastatic |
| 14 | YES | squamous cell carcinoma, non-small cell |
| 15 | YES | mixed squamous and adenocarcinoma |
| 16 | YES | non-small cell carcinoma, squamous |
| 17 | YES | small cell carcinoma |
| 18 | YES | adenocarcinoma, lung cancer, nsc |
| 19 | YES | adenocarcinoma, lung cancer, nsc, metastatic |

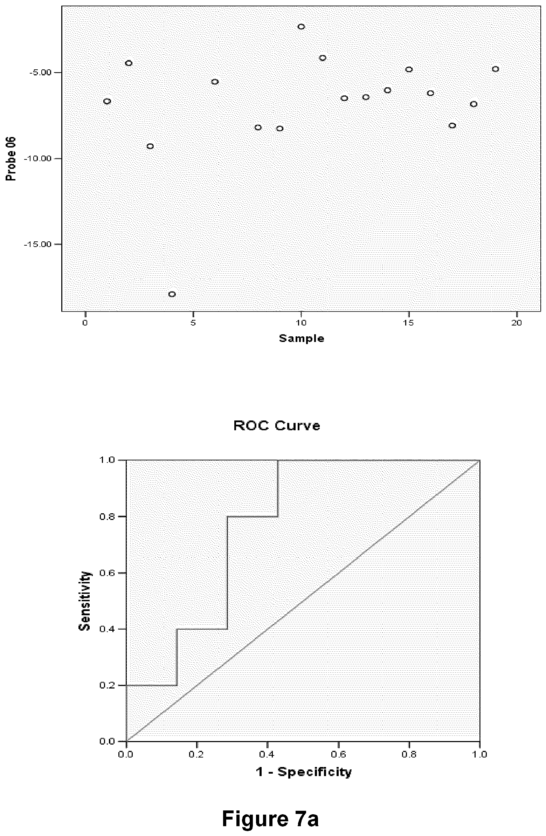

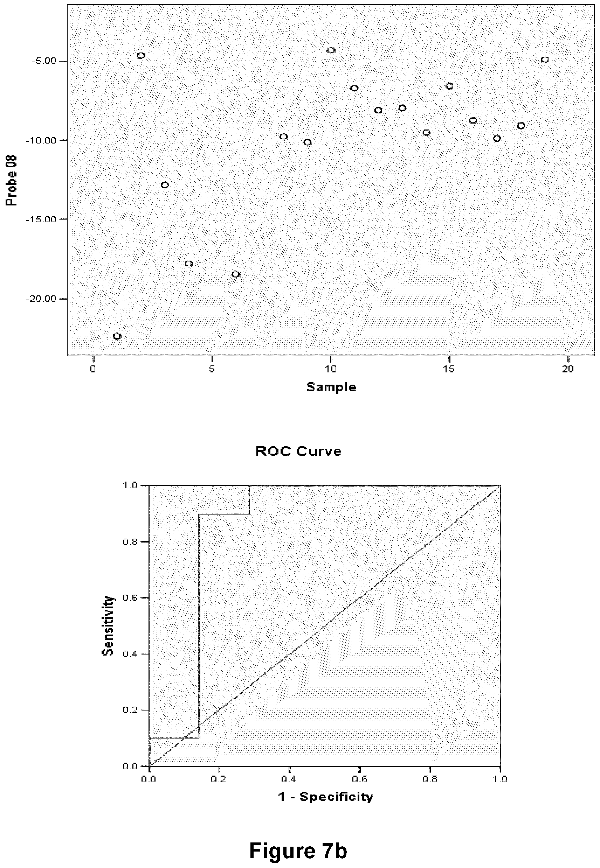

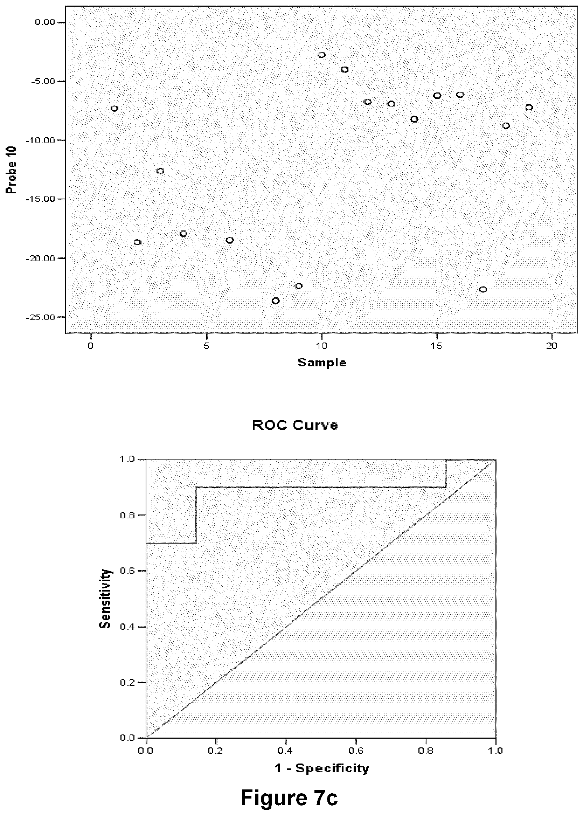

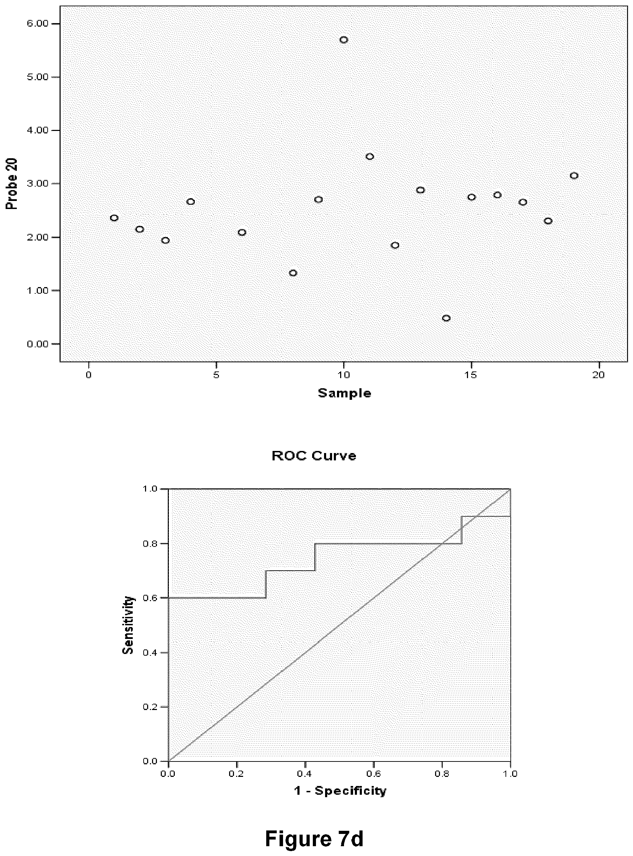

The analysis of data was performed according to the method described in Example 5. The results are illustrated in figures 7a, 7b, 7c and 7d.

### Summary of Results:

Transcript 6: There exists a statistically significant difference between the means (p<0.1) of the normal (benign) and malignant groups (p=0.06), using a cutoff value of -6.5691 as demonstrated by the ROC curve results in a sensitivity of 80% and specificity of 71% and the area under the curve is 0.77, indicating fair test accuracy. The threshold value chosen may be adjusted to increase either the specificity or sensitivity of the test for a particular application.

Transcript 8: The difference between the means of the normal and malignant groups is statistically significant, p<0.05 (p=0.02). Using a cutoff value of -9.6166 as demonstrated by the ROC curve results in a sensitivity of 90% and specificity of 86% and the area under the curve is 0.86 indicating good test accuracy. The threshold value chosen may be adjusted to increase either the specificity or sensitivity of the test for a particular application.

Transcript 10: The difference between the means of the normal and malignant groups is statistically significant, p≤0.01 (p=0.01). Using a cutoff value of -10.6717 as demonstrated by the ROC curve results in a sensitivity of 90% and specificity of 86% and the area under the curve is 0.89 indicating good test accuracy. The threshold value chosen may be adjusted to increase either the specificity or sensitivity of the test for a particular application.

Transcript 20: The difference between the means of the normal and malignant groups is statistically significant, p≤0.1 (p=0.1). Using a cutoff value of 2.5071 as demonstrated by the ROC curve results in a sensitivity of 70% and specificity of 71% and the area under the curve is 0.74 indicating fair test accuracy. The threshold value chosen may be adjusted to increase either the specificity or sensitivity of the test for a particular application.

### Conclusions:

The results from example 6 illustrate the utility of transcripts 6, 8, 10, and 20 of the disclosure in the detection of lung cancer tumours and the distinction between malignant and normal lung tissues. Any of these three transcripts may be used for the detection or characterization of lung cancer in a clinical setting.

### Example 7: Application to Melanoma

This study sought to determine the effectiveness of several transcripts of the disclosure in the detection of melanomas. In this study a total of 14 samples were used, comprising five control (benign) tissue samples and nine malignant tissue samples. All samples were formalin fixed, paraffin embedded (FFPE). The FFPE tissue samples were sectioned into tubes and homogenized according to the manufacturer's recommendations (Quantigene® 2.0 Sample Processing Kit for FFPE Samples; and Quantigene 2.0 Reagent System User Manual) such that each sample approximated 20 microns prior to homogenization. Homogenates were diluted 1:4 and the quantity of 7 target transcripts and 1 housekeeper transcript was measured in Relative Luminenscence Units RLU on a Glomax™ Multi Detection System (Promega). All samples were assayed in triplicate for each transcript. Background measurements (no template) were done in triplicate as well.

The 14 tissue samples used in this example had the following characteristics:

**Table 10: Characteristics of Melanoma Cancer Samples**

| **Sample** | **Malignant** | **Comments (source of tissue)** |
|---|---|---|
| 1 | NO | breast reduction tissue (skin) |
| 2 | NO | breast reduction tissue (skin) |
| 3 | NO | breast reduction tissue (skin) |
| 4 | NO | breast reduction tissue (skin) |
| 5 | NO | breast reduction tissue (skin) |
| 6 | YES | lentigo maligna, (melanoma in situ) invasive melanoma not present |
| 7 | YES | invasive malignant melanoma |
| 8 | YES | nodular melanoma, pT3b, associated features of lentigo maligna |
| 9 | YES | residual superficial spreading invasive malignant melanoma, Clark's level II |
| 10 | YES | superficial spreading malignant melanoma, Clark's Level II |
| 11 | YES | nodular malignant melanoma, Clark's level IV |
| 12 | YES | superficial spreading malignant melanoma in situ, no evidence of invasion |
| 13 | YES | superficial spreading malignant melanoma, Clark's level II, focally present vertical phase |
| 14 | YES | superficial spreading malignant melanoma in situ, Clark's level I |

The following transcripts were prepared for this example:

**Table 11: Characteristics of Melanoma Cancer Transcripts**

| **Transcript ID** | **Junction Site** | **Gene Junction** |
|---|---|---|
| 6 | 8828:4896 | ATPase6:Cytb |
| 10 | 7438:13476 | COI:ND5 |
| 11 | 7775:13532 | COII:ND5 |
| 14 | 9191:12909 | ATPase6:ND5 |
| 15 | 9574:12972 | COIII:ND5 |
| 16 | 10367:12829 | ND3:ND5 |
| 20 | 8469:13447 | ATPase8:ND5 |
| Peptidylpropyl isomerase B (PPIB) ("housekeeper") | N/A | N/A |

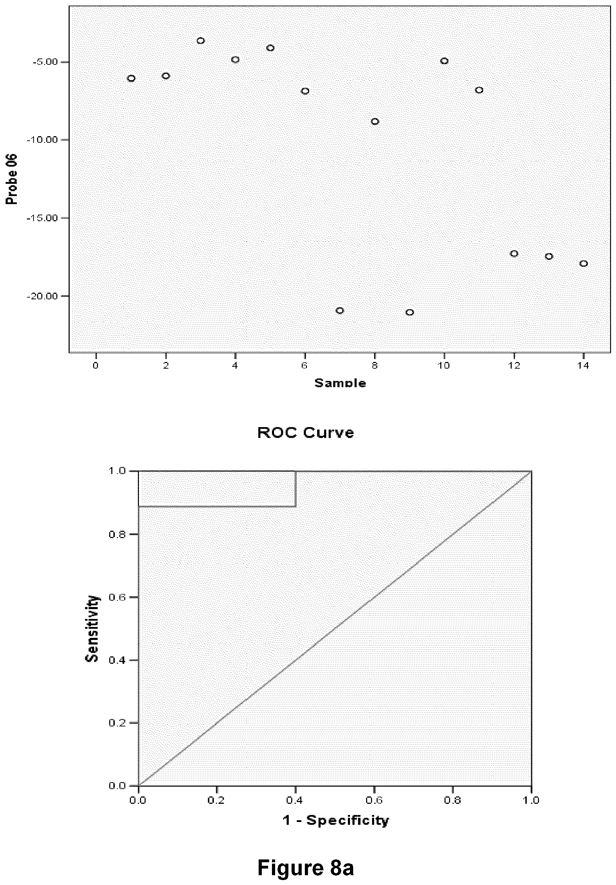

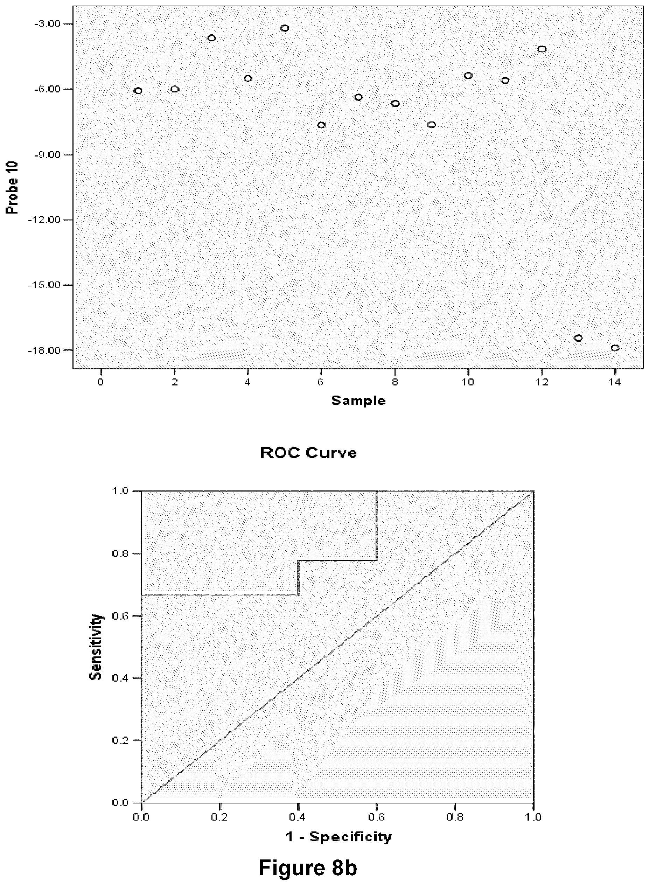

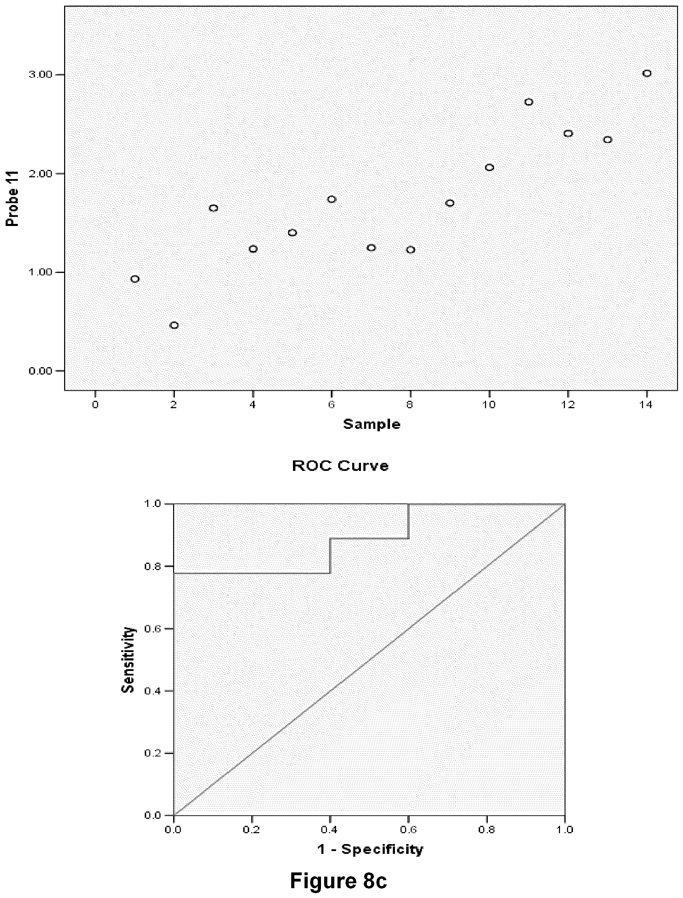

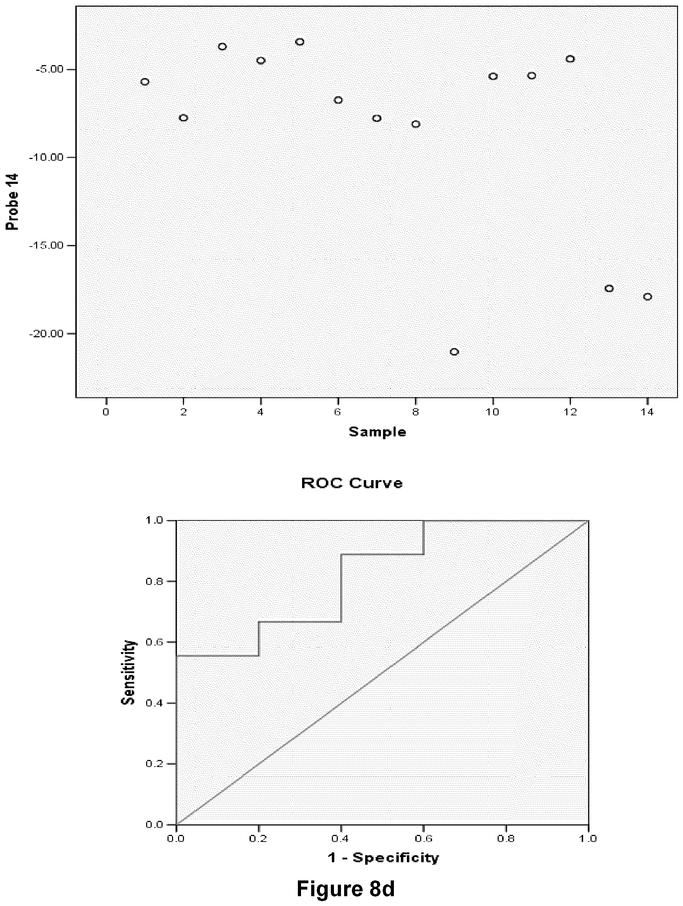

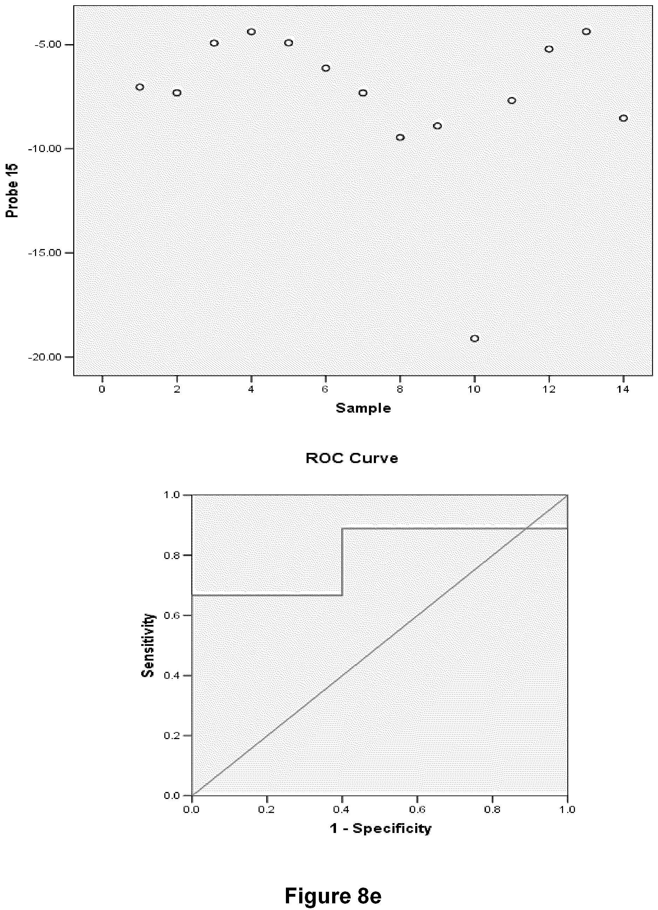

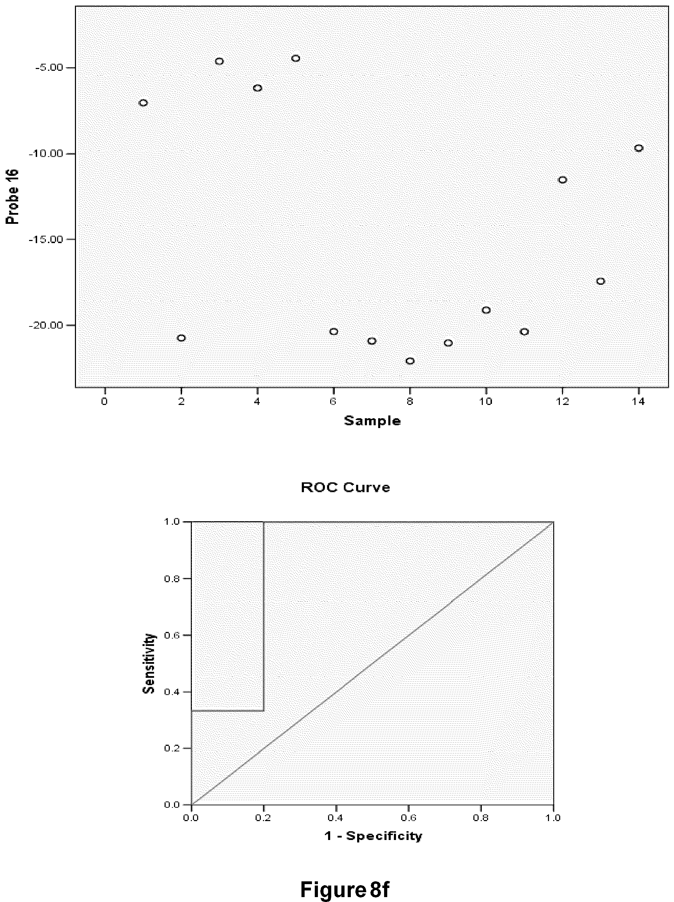

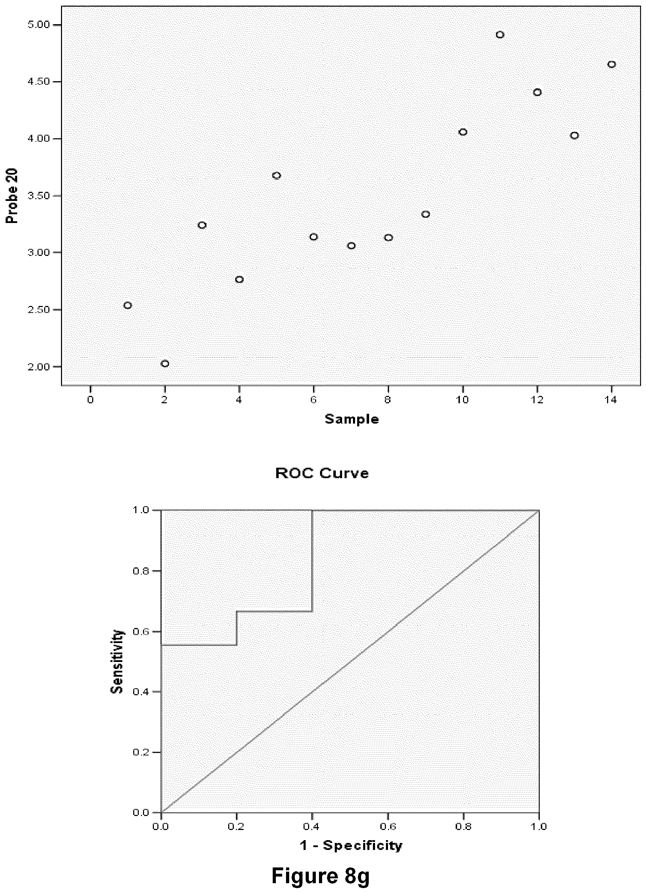

As indicated, transcripts 10 and 11 were also used in Example 5. The analysis of data was performed according to the method described in Example 5. The results are illustrated in figures 8a -8g.

### Summary of Results:

Transcript 6: There exists a statistically significant difference between the means (p≤0.01) of the normal and malignant groups (p=0.01). Further, using a cutoff value of -5.9531 as demonstrated by the ROC curve results in a sensitivity of 89% and specificity of 80% and the area under the curve is 0.96, indicating very good test accuracy. The threshold value chosen may be adjusted to increase either the specificity or sensitivity of the test for a particular application.

Transcript 10: There exists a statistically significant difference between the means (p≤0.05) of the normal and malignant groups (p=0.05), using a cutoff value of -4.7572as demonstrated by the ROC curve results in a sensitivity of 89% and specificity of 40% and the area under the curve is 0.82, indicating good test accuracy. The threshold value chosen may be adjusted to increase either the specificity or sensitivity of the test for a particular application.

Transcript 11: There exists a statistically significant difference between the means (p<0.05) of the normal and malignant groups (p=0.02). Further, using a cutoff value of 1.6762 as demonstrated by the ROC curve results in a sensitivity of 78% and specificity of 100% and the area under the curve is 0.89, indicating good test accuracy. The threshold value chosen may be adjusted to increase either the specificity or sensitivity of the test for a particular application.

Transcript 14: There exists a statistically significant difference between the means (p≤0.05) of the normal and malignant groups (p=0.05). Further, using a cutoff value of -4.9118 as demonstrated by the ROC curve results in a sensitivity of 89% and specificity of 60% and the area under the curve is 0.82, indicating good test accuracy. The threshold value chosen may be adjusted to increase either the specificity or sensitivity of the test for a particular application.

Transcript 15: There exists a statistically significant difference between the means (p<0.1) of the normal and malignant groups (p=0.07), using a cutoff value of -7.3107as demonstrated by the ROC curve results in a sensitivity of 100% and specificity of 67% and the area under the curve is 0.80, indicating good test accuracy. The threshold value chosen may be adjusted to increase either the specificity or sensitivity of the test for a particular application.

Transcript 16: There exists a statistically significant difference between the means (p<0.05) of the normal and malignant groups (p=0.03). Further, using a cutoff value of -10.5963as demonstrated by the ROC curve results in a sensitivity of 89% and specificity of 80% and the area under the curve is 0.878, indicating good test accuracy. The threshold value chosen may be adjusted to increase either the specificity or sensitivity of the test for a particular application.

Transcript 20: There exists a statistically significant difference between the means (p<0.05) of the normal and malignant groups (p=0.04). Further, using a cutoff value of -8.3543as demonstrated by the ROC curve results in a sensitivity of 100% and specificity of 80% and the area under the curve is 0.89, indicating good test accuracy. The threshold value chosen may be adjusted to increase either the specificity or sensitivity of the test for a particular application.

### Conclusions:

The results from example 7 illustrate the utility of transcripts 6, 10, 11, 14, 15, 16 and 20 of the disclosure in the detection of malignant melanomas. As indicated above, transcripts 10 and 11 were also found have utility in detecting colorectal cancer while transcript 6 has utility in the detection of lung cancer. A transcript summary by disease is provided at Table 6.

### Example 8: Application to Ovarian Cancer

This study sought to determine the effectiveness of several transcripts of the disclosure in detecting ovarian cancer. A total of 20 samples were prepared comprising ten control (benign) tissue samples (samples 1 to 10) and ten tumour (malignant) tissue samples (samples 11 to 20). The samples were homogenized according to the manufacturer's recommendations (Quantigene® Sample Processing Kit for Fresh or Frozen Animal Tissues; and Quantigene 2.0 Reagent System User Manual). Eight target transcripts and one housekeeper transcript were prepared in the manner as outlined above in previous examples.

The 20 tissue samples used in this example had the following characteristics:

**Table 12: Characteristics of Ovarian Cancer Samples**

| **Sample** | **Diagnosis** | **Comments** |
|---|---|---|
| 1 | Normal | follicular cyst |
| 2 | Normal | fibroma |
| 3 | Normal | No pathological change in ovaries |
| 4 | Normal | follicular cysts |
| 5 | Normal | cellular fibroma |
| 6 | Normal | benign follicular and simple cysts |
| 7 | Normal | leiomyomata, corpora albicantia |
| 8 | Normal | copora albicantia and an epithelial inclusions cysts |
| 9 | Normal | corpora albicantia |
| 10 | Normal | corpora albicantia, surface inclusion cysts, follicullar cysts |
| 11 | Malignant | high grade poorly differentiated papillary serous carcinoma involving omentum |
| 12 | Malignant | endometrioid adenocarcinoma, well to moderately differentiated with focal serous differentiation |
| 13 | Malignant | papillary serous carcinoma |
| 14 | Malignant | mixed epithelial carcinoma predominantly papillary serous carcinoma |
| 15 | Malignant | High grade: serous carcinoma, papillary and solid growth patterns |
| 16 | Malignant | High Grade (3/3) Papillary serous carcinoma |
| 17 | Malignant | papillary serous carcinoma, high nuclear grade |
| 18 | Malignant | Papillary serous cystadenocarcinomas Grade:III |
| 19 | Malignant | poorly differentiated papillary serous carcinoma |
| 20 | Malignant | Well-differentiated adnocarcinoma, Endometrioid type, Grade 1 |

The characteristics of the transcripts are summarized as follows:

**Table 13: Characteristics of Ovarian Cancer Transcripts**

| **Transcript ID** | **Junction Site** | **Gene Junction** |
|---|---|---|
| 1 | 8469:13447 | ATPase8:ND5 |
| 2 | 10744:14124 | ND4L:ND5 |
| 3 | 7974:15496 | COII:Cytb |
| 6 | 8828:14896 | ATPase6:Cytb |
| 11 | 7775:13532 | COII:ND5 |
| 12 | 8213:13991 | COII:ND5 |
| 15 | 9574:12972 | COIII:ND5 |
| 20 | 8469:13447 | ATPase8:ND5 |
| Ribosomal Protein Large PO (LRP) Housekeeper | N/A | N/A |

It is noted that transcripts 1, 2, 3, 6, 11, 12, 15 and 20 are the same as those discussed above with respect to Examples 3-7.

Homogenates were prepared using approximately 25mg of frozen tissue and diluted 1:4. The quantity of the transcripts was measured in Relative Luminenscence Units RLU on a Glomax™ Multi Detection System (Promega). All samples were assayed in triplicate for each transcript. Background measurements (no template) were done in triplicate as well. The analysis accounted for background by subtracting the lower limit from the RLU values for the samples. Input RNA was accounted for by using the formula log₂ a RLU - log₂ *h* RLU where a is the target fusion transcript and *h* is the housekeeper transcript.

The analysis of the data comprised the following steps:
a) Establish CV's (coefficients of variation) for triplicate assays; acceptable if ≤ 15%.
b) Establish average RLU value for triplicate assays of target fusion transcript(a) and housekeeper transcript (h).
c) Establish lower limit from triplicate value of background RLU (I).
d) Subtract lower limit (I) from (a).
e) Calculate log₂ a RLU - log₂ *h* RLU.

### Summary of Results:

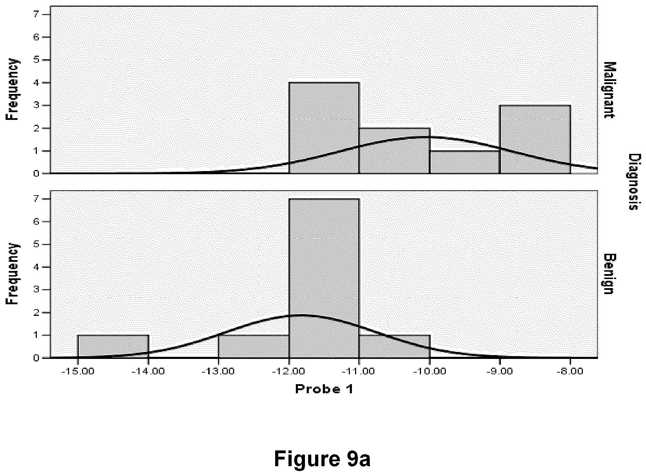

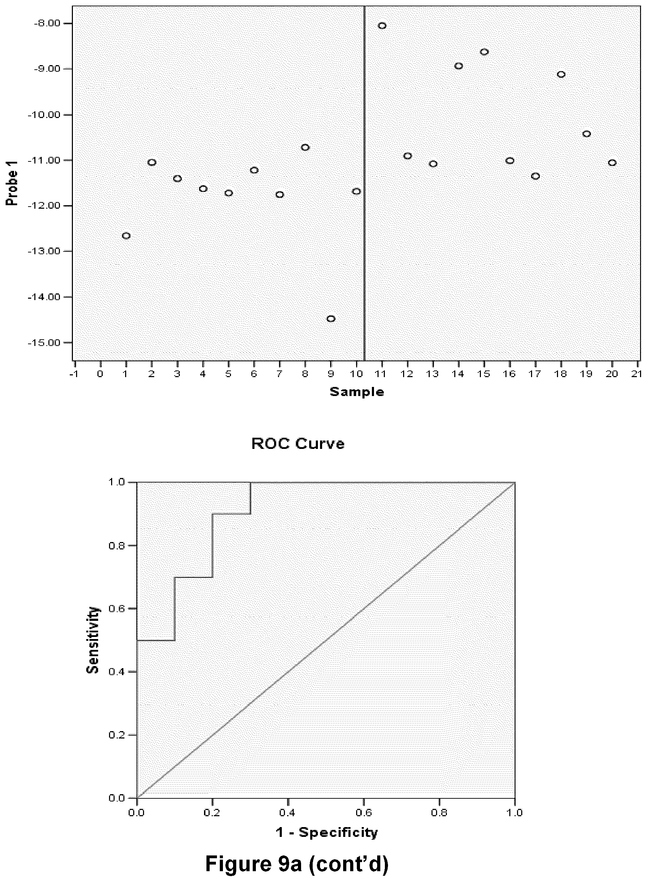

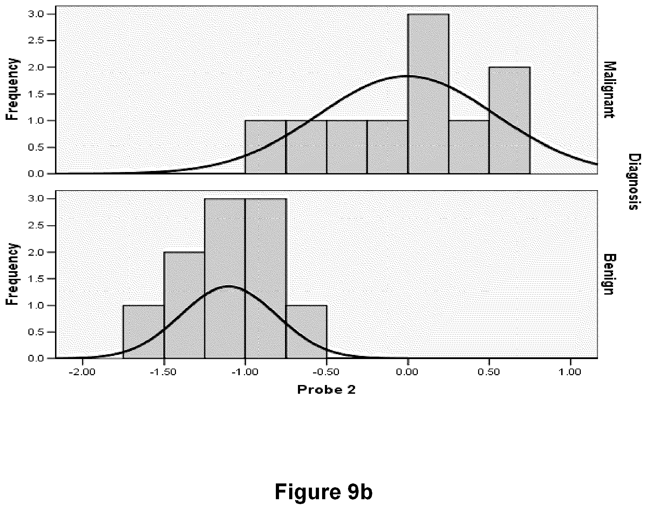

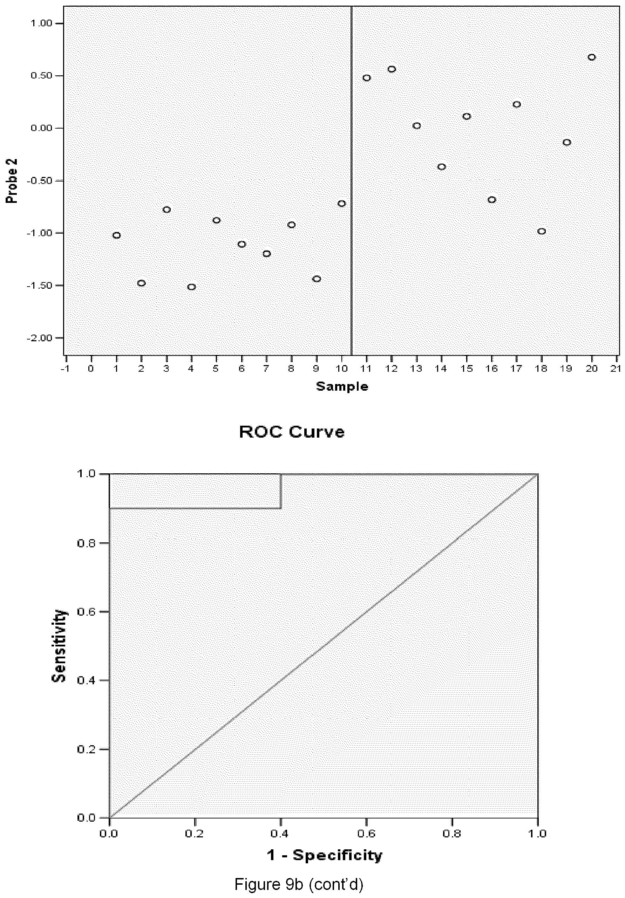

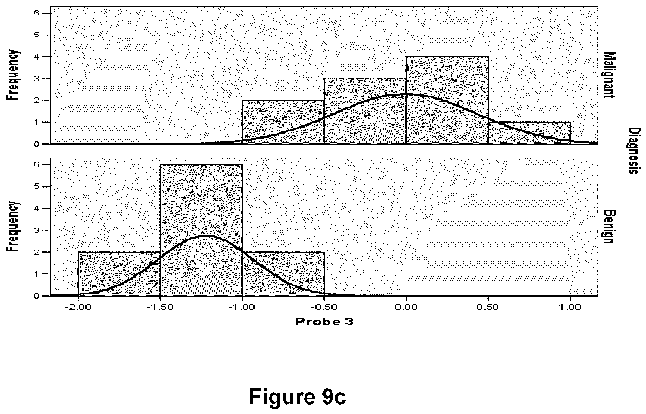

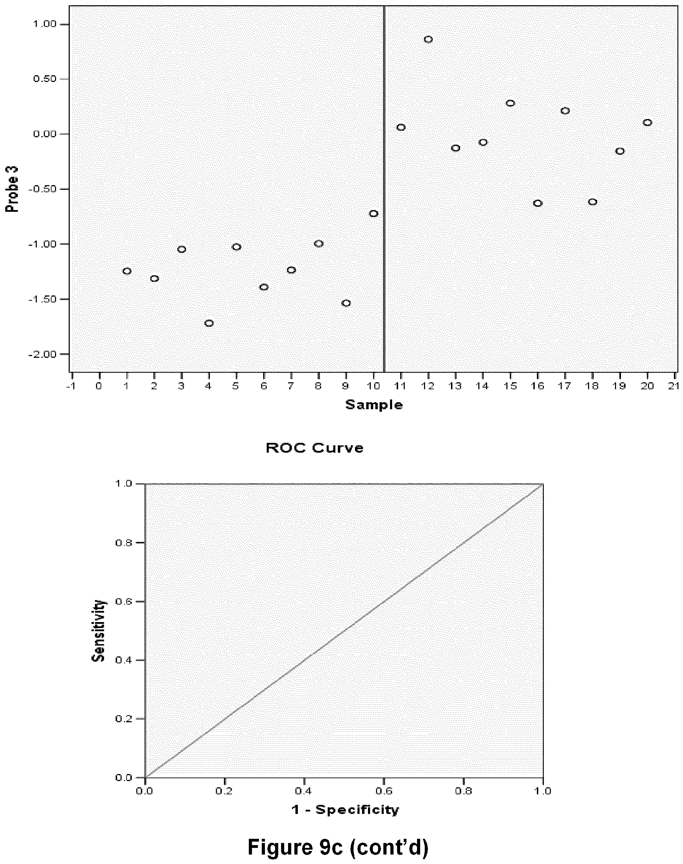

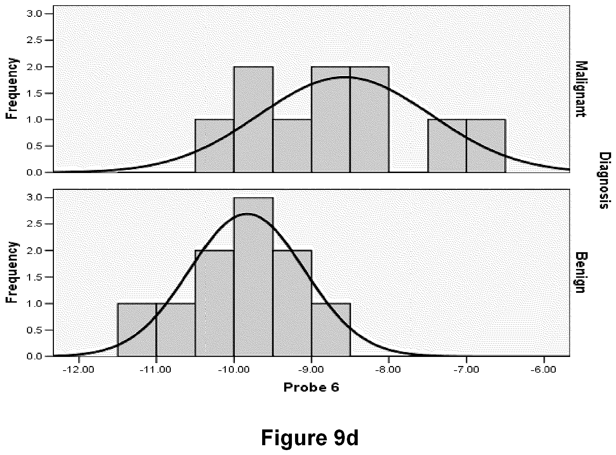

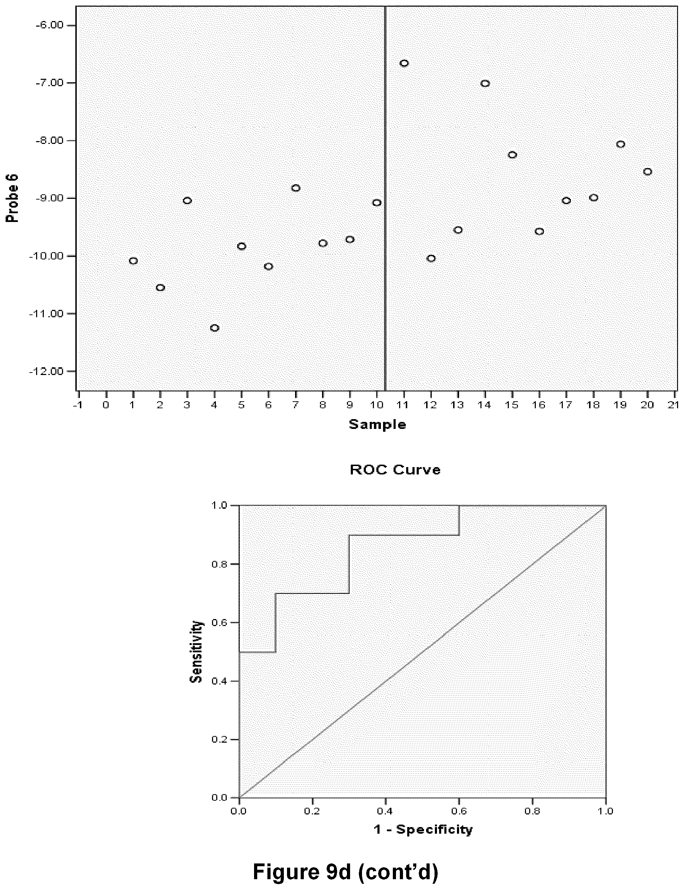

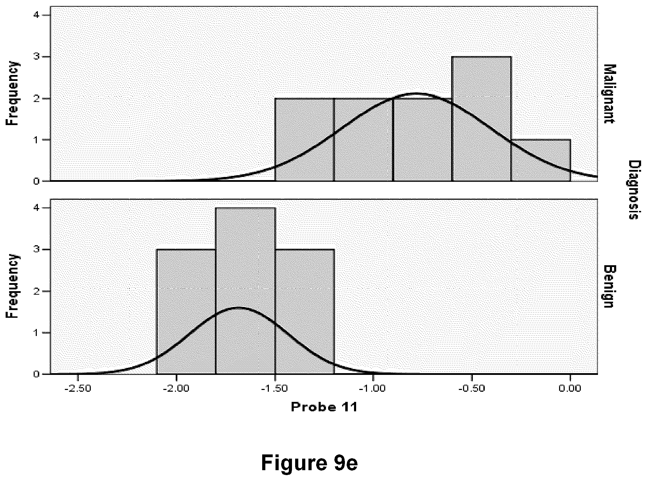

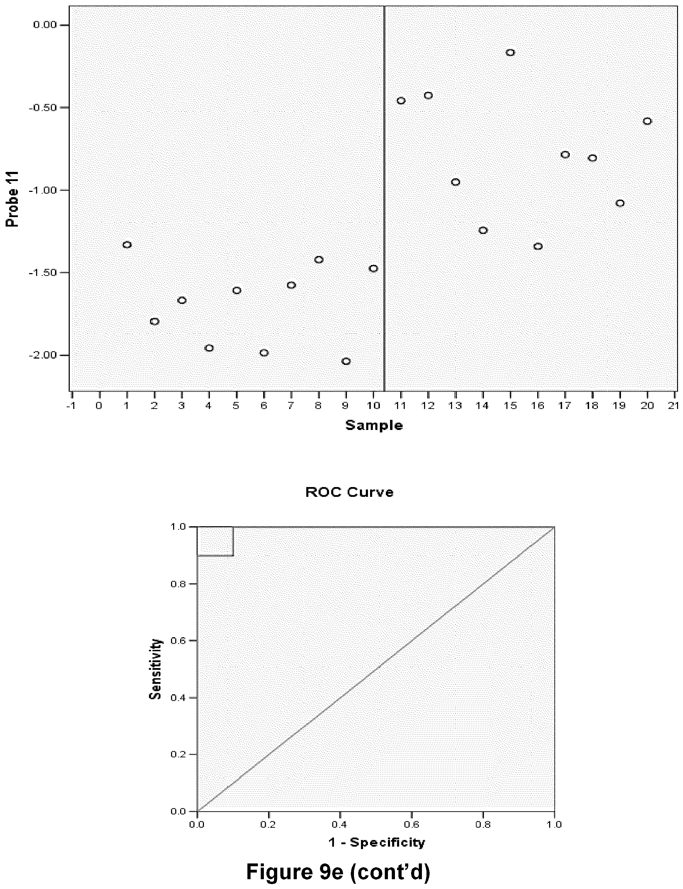

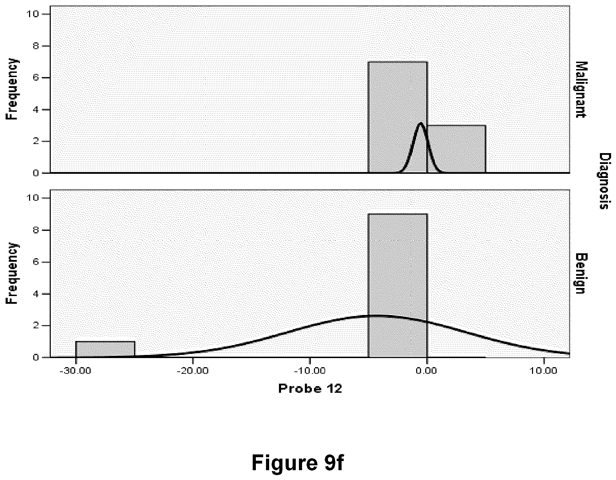

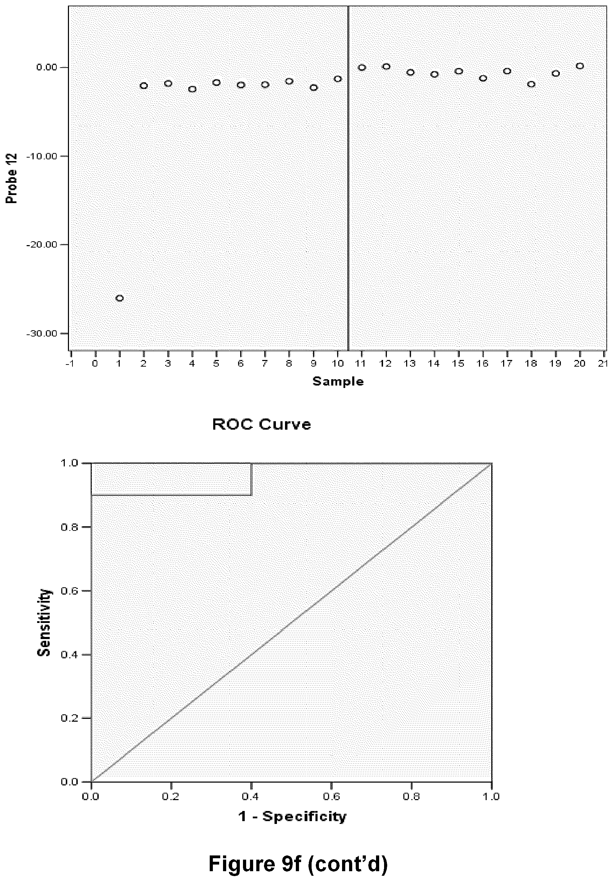

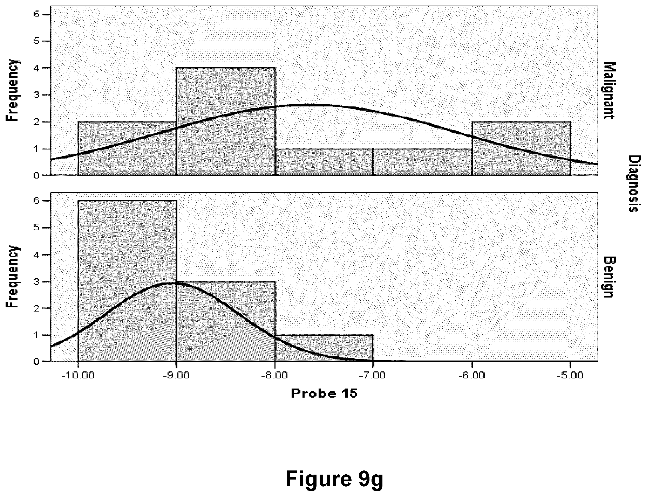

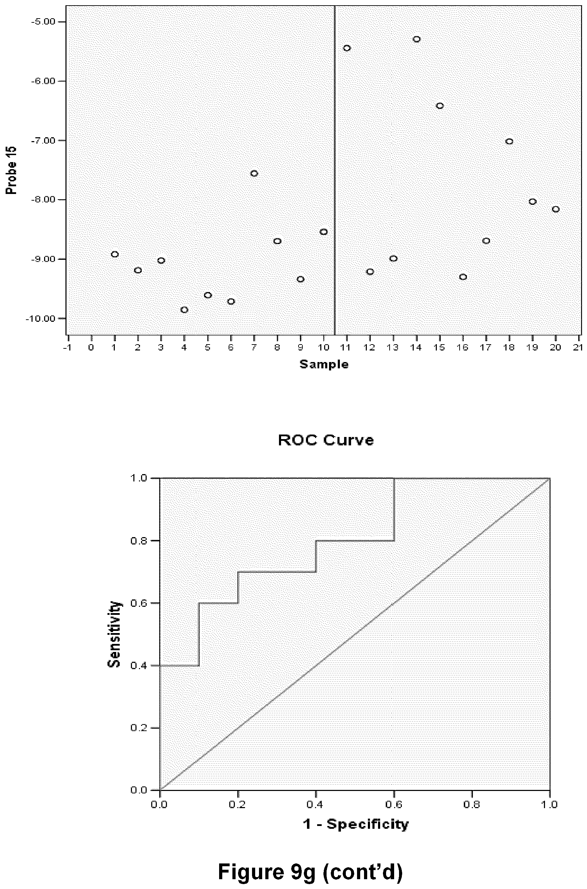

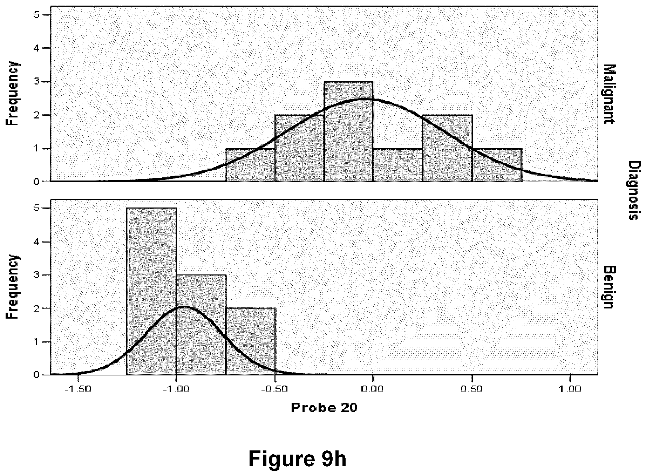

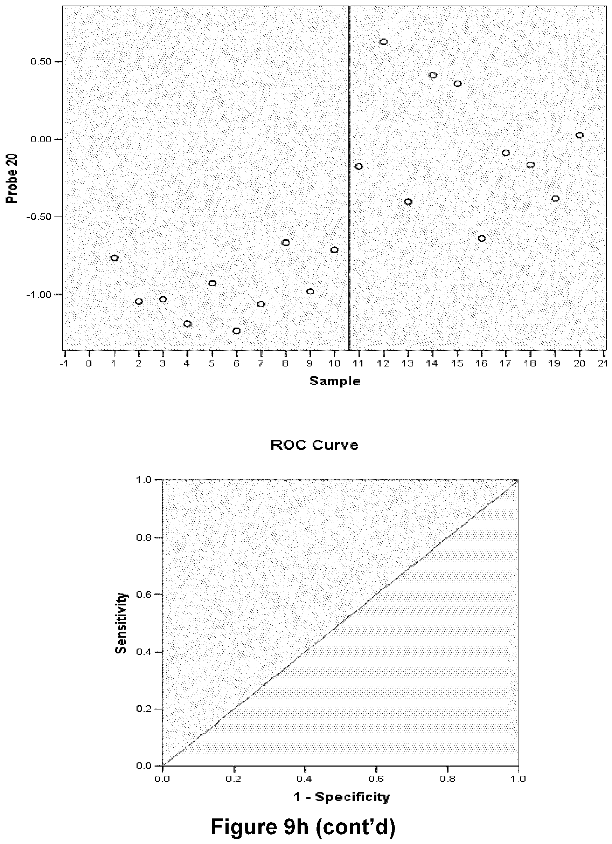

The results of the above analysis are illustrated in Figures 9a to 9h, which comprise plots of the log₂ *a* RLU - log₂ *h* RLU against sample number. Also illustrated are the respective ROC (Receiver Operating Characteristic) curves determined from the results for each transcript.

Transcript 1: There exists a statistically significant difference between the means (p<0.05) of the normal and malignant groups (p=0.002). Using a cutoff value of -11.1503 as demonstrated by the ROC curve results in a sensitivity of 90% and specificity of 80% and the area under the curve is 0.91 indicating very good test accuracy. The threshold value chosen may be adjusted to increase either the specificity or sensitivity of the test for a particular application.

Transcript 2: There exists a statistically significant difference between the means (p<0.01) of the normal and malignant groups (p=0.001). Using a cutoff value of 0.6962 as demonstrated by the ROC curve results in a sensitivity of 90% and specificity of 100% and the area under the curve is 0.96 indicating very good test accuracy. The threshold value chosen may be adjusted to increase either the specificity or sensitivity of the test for a particular application.

Transcript 3: There exists a statistically significant difference between the means (p<0.01) of the normal and malignant groups (p=0.000). Using a cutoff value of 0.6754 as demonstrated by the ROC curve results in a sensitivity of 100% and specificity of 100% and the area under the curve is 1.00 indicating excellent test accuracy. The threshold value chosen may be adjusted to increase either the specificity or sensitivity of the test for a particular application.

Transcript 6: There exists a statistically significant difference between the means (p<0.01) of the normal and malignant groups (p=0.007). Using a cutoff value of -9.6479 as demonstrated by the ROC curve results in a sensitivity of 90% and specificity of 70% and the area under the curve is 0.86 indicating good test accuracy. The threshold value chosen may be adjusted to increase either the specificity or sensitivity of the test for a particular application.

Transcript 11: There is a statistically significant difference between the means (p<0.01) of the normal and malignant groups (p=0.000). Using a cutoff value of -1.3794 demonstrated by the ROC curve results in a sensitivity of 100% and specificity of 90% and the area under the curve is 0.99, indicating excellent test accuracy. The threshold value chosen may be adjusted to increase either the specificity or sensitivity of the test for a particular application.

Transcript 12: There exists a statistically significant difference between the means (p<0.01) of the normal and malignant groups (p=0.001). Using a cutoff value of -1.2379 as demonstrated by the ROC curve results in a sensitivity of 90% and specificity of 100% and the area under the curve is 0.96 indicating excellent test accuracy. The threshold value chosen may be adjusted to increase either the specificity or sensitivity of the test for a particular application.

Transcript 15: There exists a statistically significant difference between the means (p<0.05) of the normal and malignant groups (p=0.023). Using a cut-off value of -8.6926 as demonstrated by the ROC curve results in a sensitivity of 70% and specificity of 80% and the area under the curve is 0.80 indicating good test accuracy. The threshold value chosen may be adjusted to increase either the specificity or sensitivity of the test for a particular application.

Transcript 20: There exists a statistically significant difference between the means (p<0.01) of the normal and malignant groups (p=0.000). Using a cut-off value of 0.6521 as demonstrated by the ROC curve results in a sensitivity of 100% and specificity of 100% and the area under the curve is 0.76 indicating fair to good test accuracy. The threshold value chosen may be adjusted to increase either the specificity or sensitivity of the test for a particular application.

### Conclusions:

The above results illustrate the utility of transcripts 1, 2, 3, 6, 11, 12, 15, and 20 in the detection of ovarian cancer and in distinguishing malignant from normal ovarian tissue. Transcripts 1, 2 and 3 were also found to have utility in the detection of prostate cancer. Transcript 6 was also found to have utility in the detection of melanoma and lung cancer. Transcript 11 was also found to have utility in the detection of melanoma skin cancer, colorectal cancer and testicular cancer. Transcript 12 was also found to have utility in the detection of colorectal cancer and testicular cancer. Transcript 15 was also found to have utility in the detection of melanoma and testicular cancer. Transcript 20 was also found to have utility in the detection of colorectal cancer, melanoma, and testicular cancer. Any of the 8 transcripts listed may be used individually or in combination as a tool for the detection or characterization of ovarian cancer in a clinical setting.

### Example 9: Application to Testicular Cancer

This study sought to determine the effectiveness of several transcripts of the disclosure in detecting testicular cancer. A total of 17 samples were prepared comprising eight control (benign) tissue samples (samples 1 to 8) and 9 tumour (malignant) tissue samples (samples 9 to 17), 5 of the malignant samples were non-seminomas (samples 9-13)and 4 were seminomas (samples 14-17). The samples were homogenized according to the manufacturer's recommendations (Quantigene® Sample Processing Kit for Fresh or Frozen Animal Tissues; and Quantigene 2.0 Reagent System User Manual). 10 target transcripts and one housekeeper transcript were prepared in the manner as outlined above in previous examples.

The 17 tissue samples used in this example had the following characteristics:

**Table 14: Characteristics of Testicular Cancer Samples**

| Sample | General Diagnosis | Stratified Malignant Diagnosis |
|---|---|---|
| 1 | Benign | Benign |
| 2 | Benign | Benign |
| 3 | Benign | Benign |
| 4 | Benign | Benign |
| 5 | Benign | Benign |
| 6 | Benign | Benign |
| 7 | Benign | Benign |
| 8 | Benign | Benign |
| 9 | Malignant | Non-Seminoma |
| 10 | Malignant | Non-Seminoma |
| 11 | Malignant | Non-Seminoma |
| 12 | Malignant | Non-Seminoma |
| 13 | Malignant | Non-Seminoma |
| 14 | Malignant | Seminoma |
| 15 | Malignant | Seminoma |
| 16 | Malignant | Seminoma |
| 17 | Malignant | Seminoma |

The characteristics of the transcripts are summarized as follows:

**Table 15: Characteristics of Testicular Cancer Transcripts**

| **Transcript ID** | **Junction Site** | **Gene Junction** |
|---|---|---|
| 2 | 10744:14124 | ND4L:ND5 |
| 3 | 7974:15496 | COII:Cytb |
| 4 | 7992:15730 | COII:Cytb |
| 11 | 7775:13532 | COII:ND5 |
| 12 | 8213:13991 | COII:ND5 |
| 13 | 9144:13816 | ATPase6:ND5 |
| 15 | 9574:12972 | COIII:ND5 |
| 16 | 10367:12829 | ND3:ND5 |
| 20 | 8469:13447 | ATPase8:ND5 |
| Peptidylpropyl isomerase B (PPIB) | N/A | N/A |

It is noted that transcripts 2, 3, 4, 11, 12, 15, 16 and 20 are the same as those discussed above with respect to Examples 3-8.

Homogenates were prepared using approximately 25mg of frozen tissue and diluted 1:4. The quantity of the transcripts was measured in Relative Luminenscence Units RLU on a Glomax™ Multi Detection System (Promega). All samples were assayed in triplicate for each transcript. Background measurements (no template) were done in triplicate as well. The analysis accounted for background by subtracting the lower limit from the RLU values for the samples. Input RNA was accounted for by using the formula log₂ a RLU - log₂ *h* RLU where a is the target fusion transcript and *h* is the housekeeper transcript.

The analysis of the data comprised the following steps:
a) Establish CV's (coefficients of variation) for triplicate assays; acceptable if ≤ 15%.
b) Establish average RLU value for triplicate assays of target fusion transcript(a) and housekeeper transcript (h).
c) Establish lower limit from triplicate value of background RLU (I).
d) Subtract lower limit (I) from (a).
e) Calculate log₂ *a* RLU - log₂ *h* RLU.

### Summary of Results:

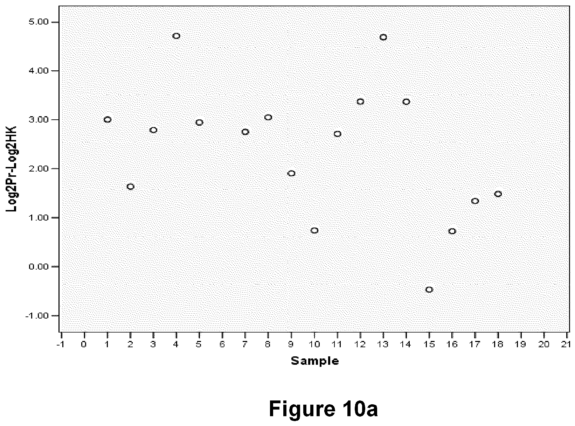

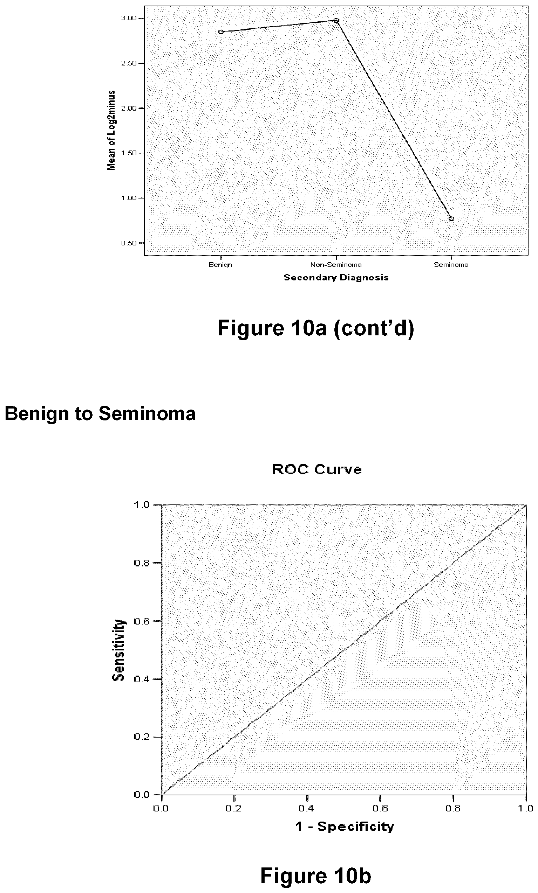

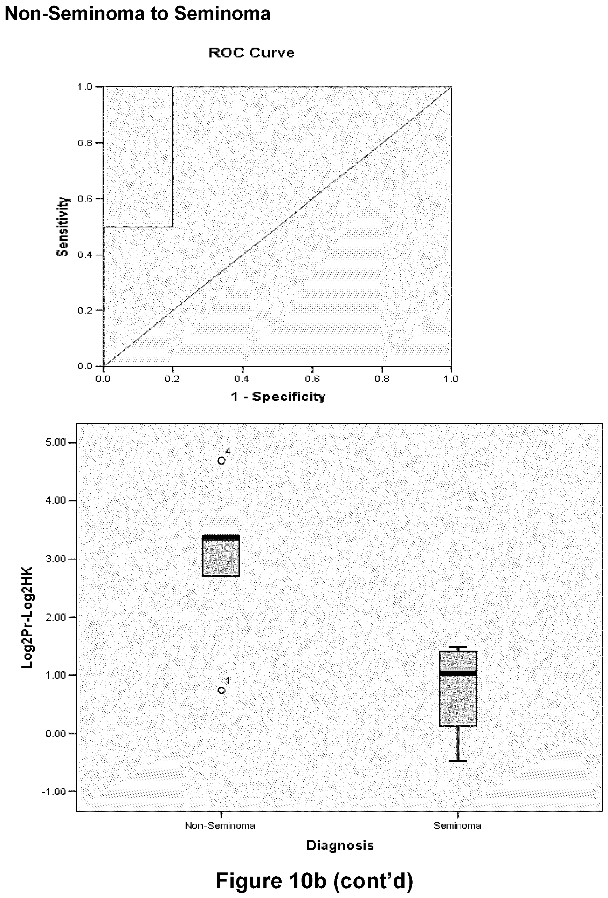

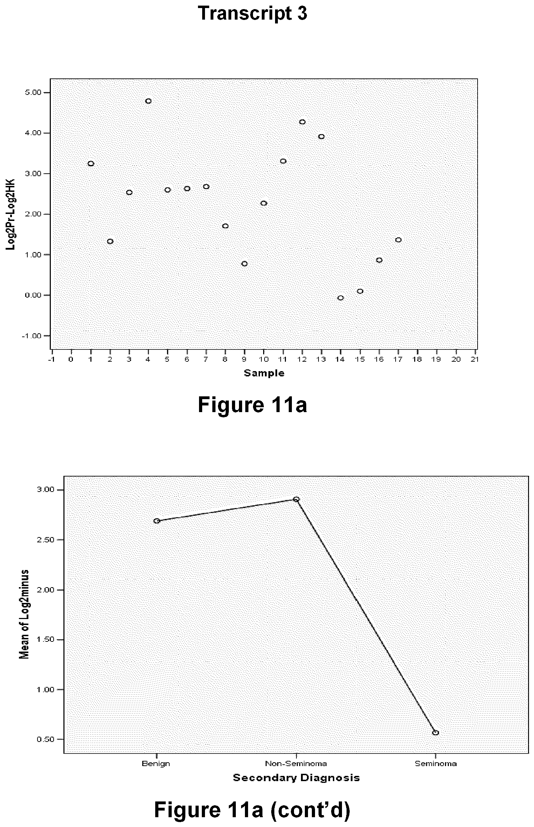

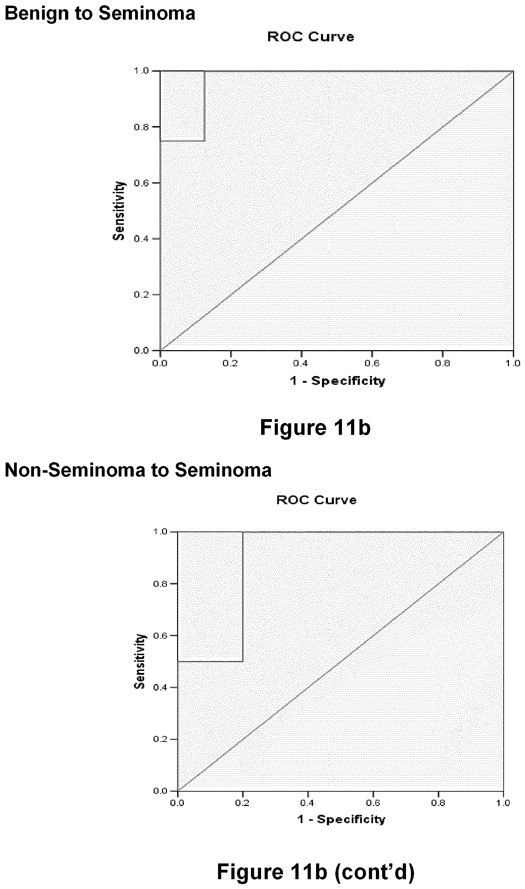

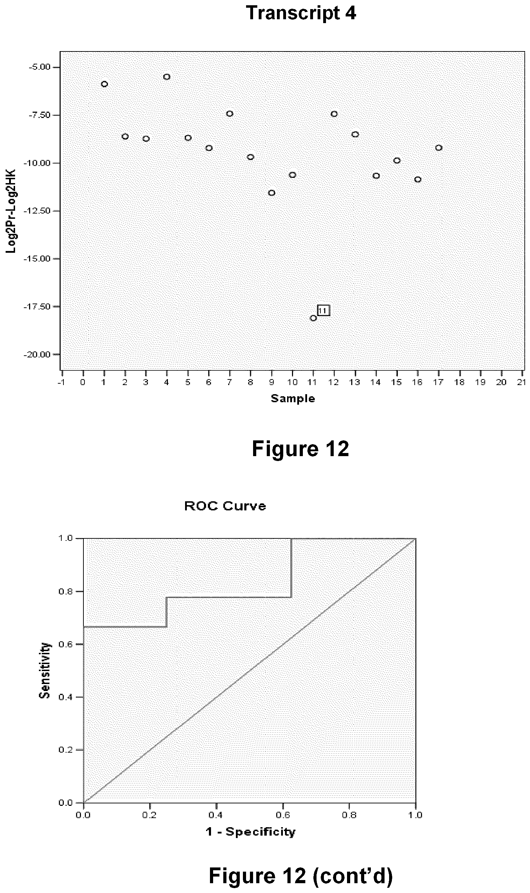

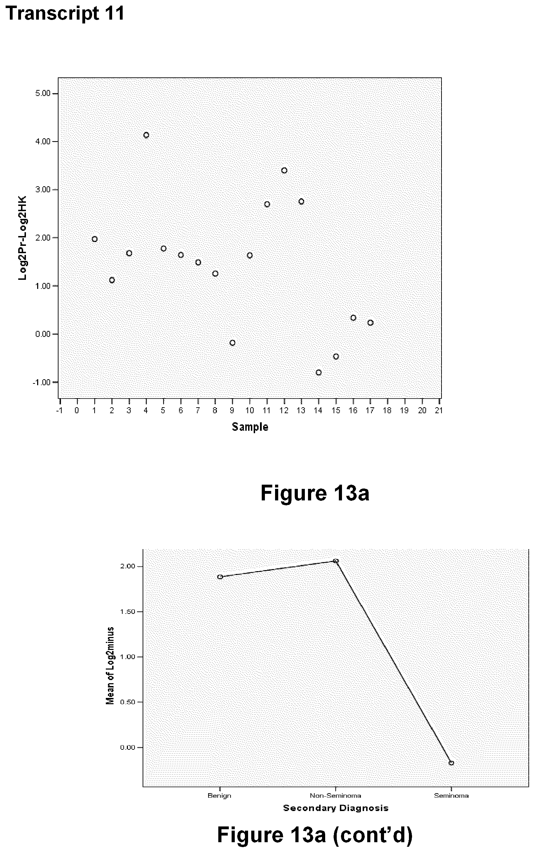

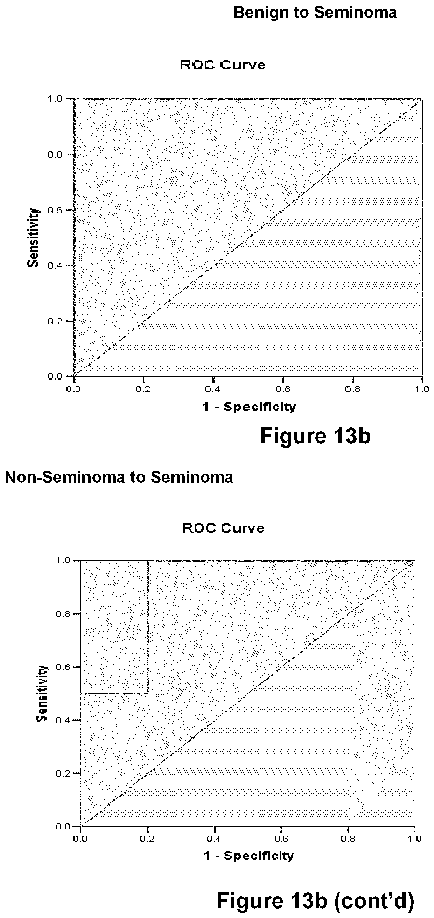

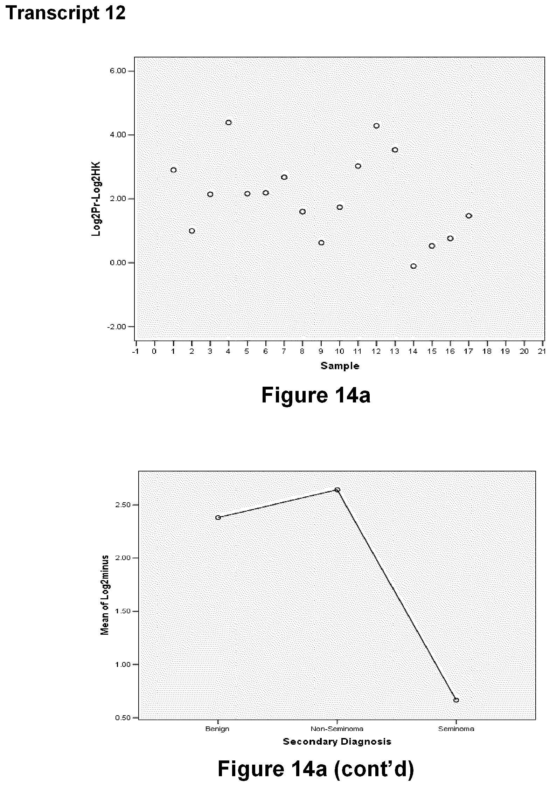

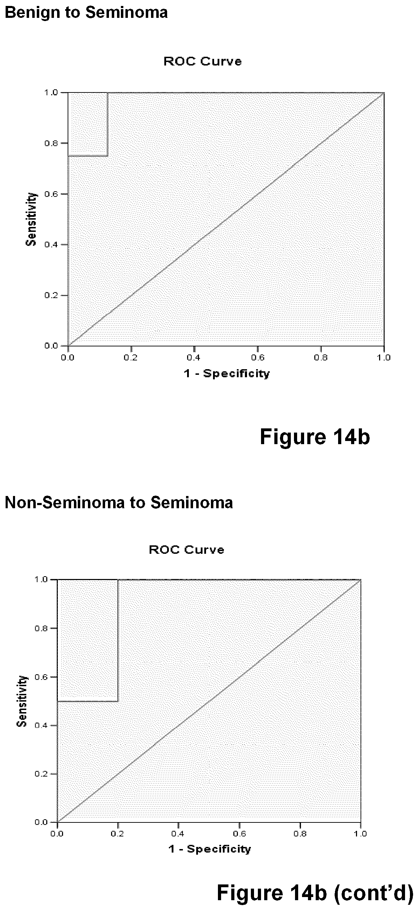

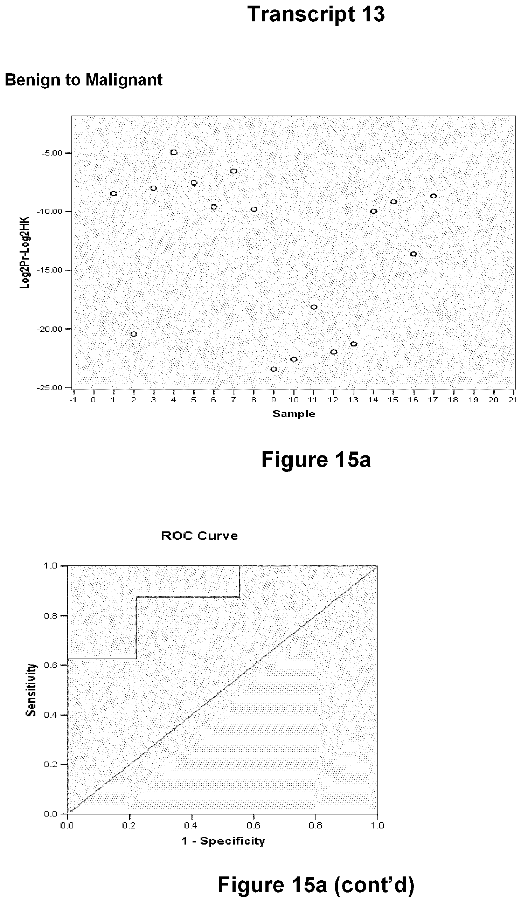

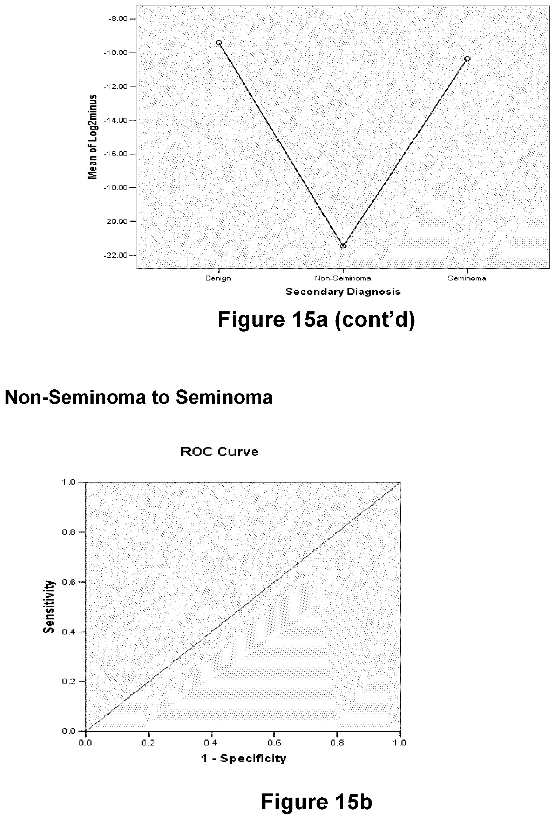

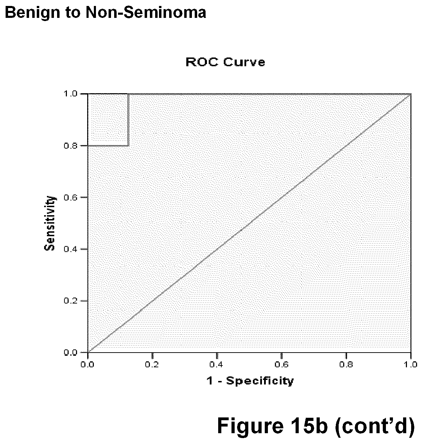

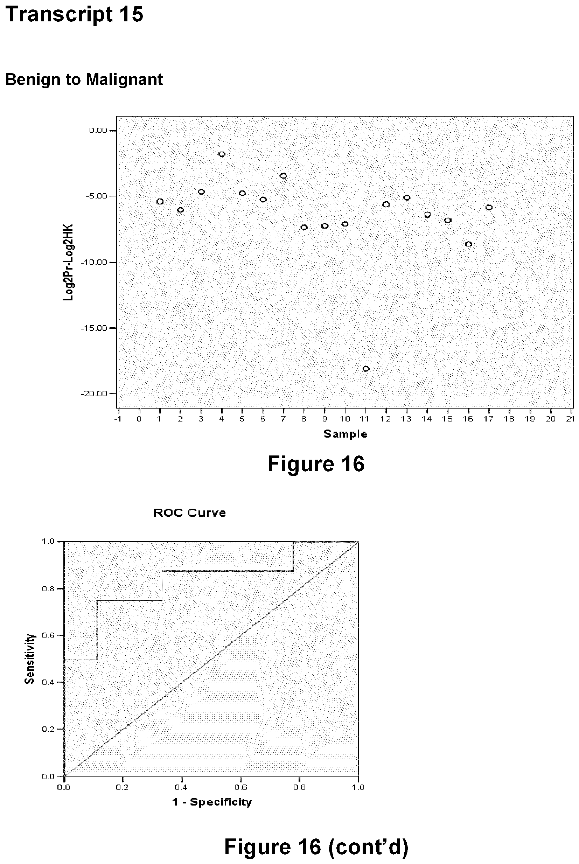

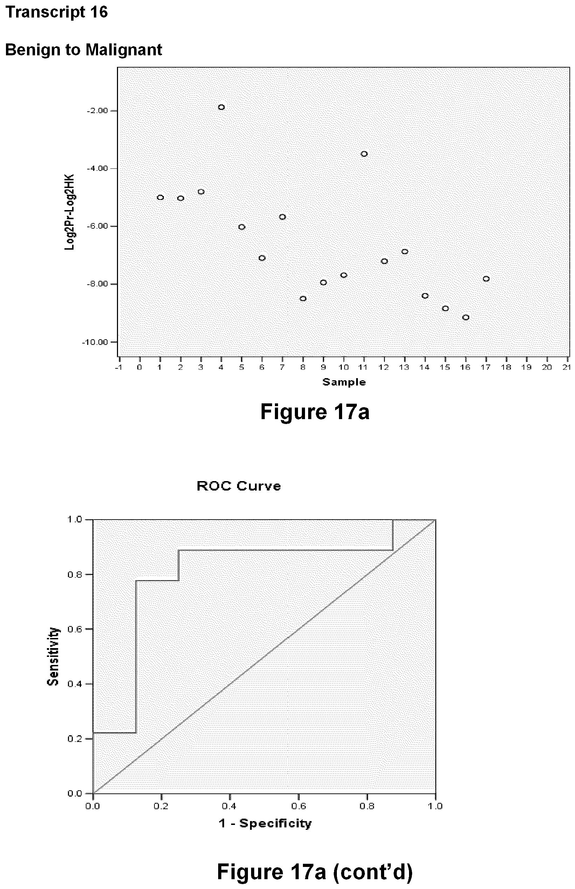

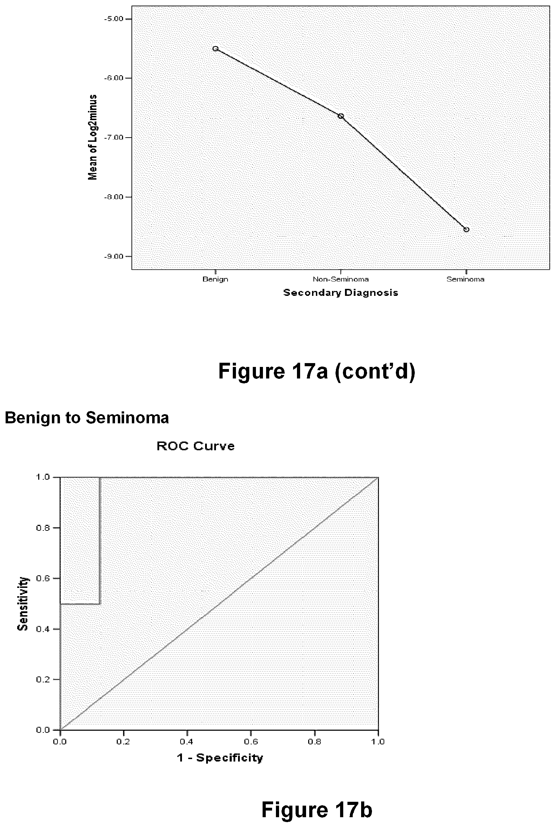

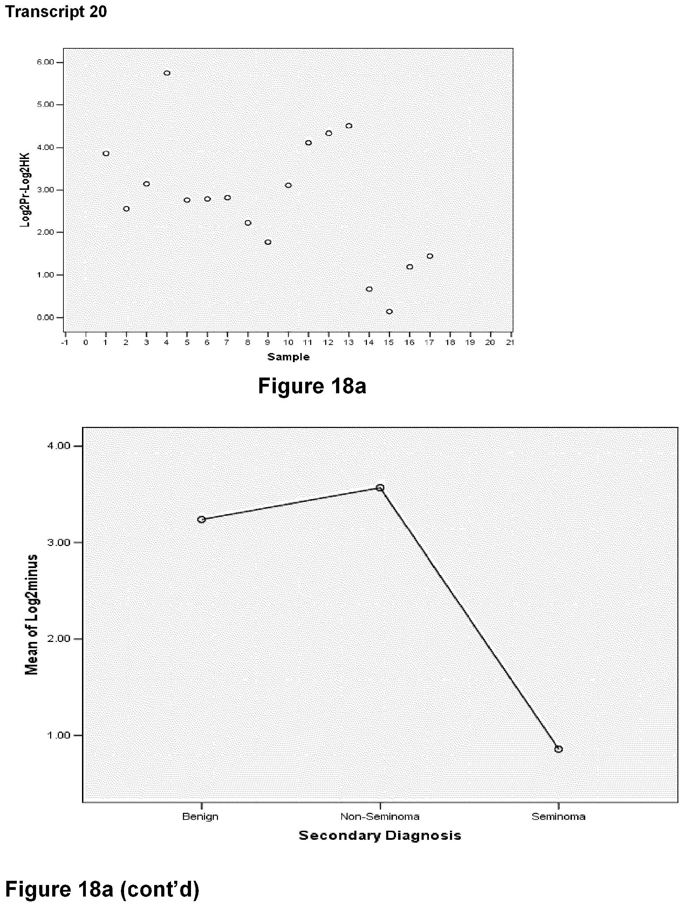

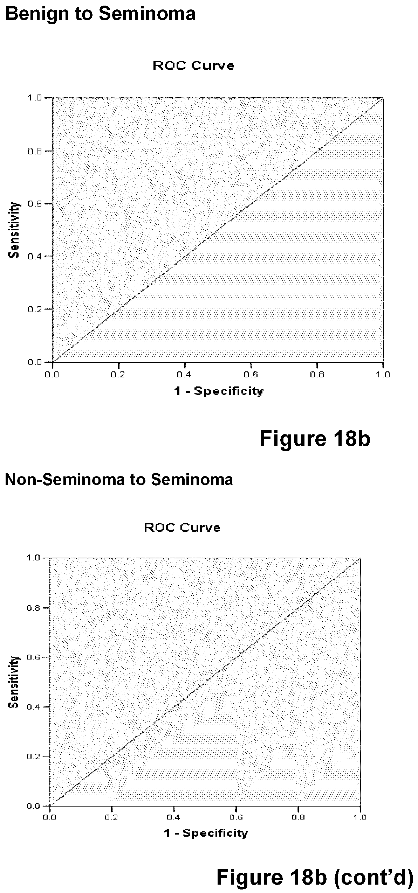

The results of the above analysis are illustrated in Figures 10 to 18, which comprise plots of the log₂ *a* RLU - log₂ *h* RLU against sample number. Also illustrated are the respective ROC (Receiver Operating Characteristic) curves determined from the results for each transcript.

While some transcripts distinguish between benign and malignant testicular tissue, others demonstrate distinction between the tumour subtypes of seminoma and non-seminoma and/or benign testicular tissue. It is therefore anticipated that combining transcripts from each class will facilitate not only detection of testicular cancer but also classification into subtype of seminoma or non-seminomas.

Transcript 2: There exists a statistically significant difference between the means (p<0.05) of the normal group and the malignant seminomas (p=0.02). Using a cutoff value of 1.5621 as demonstrated by the ROC curve results in a sensitivity of 100% and specificity of 100% and the area under the curve is 1.00 indicating excellent test accuracy. There also exists a statistically significant difference between the means (p<0.05) of the malignant seminomas and the malignant non-seminomas (p=0.024). Using a cutoff value of 2.1006 as demonstrated by the ROC curve results in a sensitivity of 100% and specificity of 80% and the area under the curve is 0.90 indicating excellent test accuracy. The threshold value chosen may be adjusted to increase either the specificity or sensitivity of the test for a particular application.

Transcript 3: There exists a statistically significant difference between the means (p<0.05) of the normal group and the malignant seminomas (p=0.018). Using a cutoff value of 0.969 as demonstrated by the ROC curve results in a sensitivity of 100% and specificity of 87.5% and the area under the curve is 0.969 indicating excellent accuracy. There also exists a statistically significant difference between the means (p<0.05) of the malignant seminomas and the malignant non-seminomas (p=0.017). Using a cutoff value of 1.8181 as demonstrated by the ROC curve results in a sensitivity of 100% and specificity of 80% and the area under the curve is 0.9 indicating excellent test accuracy. The threshold value chosen may be adjusted to increase either the specificity or sensitivity of the test for a particular application.

Transcript 4: There exists a statistically significant difference between the means (p<0.05) of the normal and malignant groups (p=0.034). Using a cutoff value of -9.7628 as demonstrated by the ROC curve results in a sensitivity of 67% and specificity of 100% and the area under the curve is 0.833 indicating good test accuracy. The threshold value chosen may be adjusted to increase either the specificity or sensitivity of the test for a particular application.

Transcript 11: There exists a statistically significant difference between the means (p<0.05) of the normal group and the malignant seminomas (p=0.016). Using a cutoff value of 0.732 as demonstrated by the ROC curve results in a sensitivity of 100% and specificity of 100% and the area under the curve is 1.00 indicating excellent test accuracy. There also exists a statistically significant difference between the means (p<0.05) of the malignant seminomas and the malignant non-seminomas (p=0.016). Using a cutoff value of 0.9884 as demonstrated by the ROC curve results in a sensitivity of 100% and specificity of 80% and the area under the curve is 0.90 indicating excellent test accuracy. The threshold value chosen may be adjusted to increase either the specificity or sensitivity of the test for a particular application.

Transcript 12: There exists a statistically significant difference between the means (p<0.1) of the normal group and the malignant seminomas (p=0.056). Using a cutoff value of 1.5361 as demonstrated by the ROC curve results in a sensitivity of 100% and specificity of 87.5% and the area under the curve is 0.969 indicating excellent test accuracy. There also exists a statistically significant difference between the means (p<0.05) of the malignant seminomas and the malignant non-seminomas (p=0.044). Using a cutoff value of 1.6039 as demonstrated by the ROC curve results in a sensitivity of 100% and specificity of 80% and the area under the curve is 0.9 indicating excellent test accuracy. The threshold value chosen may be adjusted to increase either the specificity or sensitivity of the test for a particular application.

Transcript 13: There exists a statistically significant difference between the means (p<0.05) of the normal group and the malignant group (p=0.019). Using a cutoff value of -9.8751 as demonstrated by the ROC curve results in a sensitivity of 87.5% and specificity of 78% and the area under the curve is 0.875 indicating very good test accuracy. There also exists a statistically significant difference between the means (p<0.01) of the malignant non-seminomas and the benign group (p=0.000). Using a cutoff value of -13.9519 as demonstrated by the ROC curve results in a sensitivity of 100% and specificity of 87.5% and the area under the curve is 0.975 indicating excellent test accuracy. There also exists a statistically significant difference between the means (p<0.01) of the malignant seminomas and the malignant non-seminomas (p=0.001). Using a cutoff value of -15.8501 as demonstrated by the ROC curve results in a sensitivity of 100% and specificity of 100% and the area under the curve is 1.00 indicating excellent test accuracy. The threshold value chosen may be adjusted to increase either the specificity or sensitivity of the test for a particular application.

Transcript 15: There exists a statistically significant difference between the means (p<0.1) of the normal and malignant groups (p=0.065). Using a cut-off value of -5.4916 as demonstrated by the ROC curve results in a sensitivity of 75% and specificity of 89% and the area under the curve is 0.833 indicating good test accuracy. The threshold value chosen may be adjusted to increase either the specificity or sensitivity of the test for a particular application.

Transcript 16: There exists a statistically significant difference between the means (p<0.05) of the normal and malignant groups including both seminomas and non-seminomas (p=0.037). Using a cut-off value of -6.448 as demonstrated by the ROC curve results in a sensitivity of 89% and specificity of 75% and the area under the curve is 0.806 indicating good test accuracy. There also exists a statistically significant difference between the means (p<0.05) of the normal and malignant seminomas (p=0.037). Using a cut-off value of -7.4575 as demonstrated by the ROC curve results in a sensitivity of 100% and specificity of 87.5% and the area under the curve is 0.938 indicating excellent test accuracy. The threshold value chosen may be adjusted to increase either the specificity or sensitivity of the test for a particular application.

Transcript 20: There exists a statistically significant difference between the means (p<0.01) of the normal group and the malignant seminomas (p=0.006). Using a cutoff value of 1.8364 as demonstrated by the ROC curve results in a sensitivity of 100% and specificity of 100% and the area under the curve is 1.00 indicating excellent test accuracy. There also exists a statistically significant difference between the means (p<0.01) of the malignant seminomas and the malignant non-seminomas (p=0.004). Using a cutoff value of 1.6065 as demonstrated by the ROC curve results in a sensitivity of 100% and specificity of 100% and the area under the curve is 1.00 indicating excellent test accuracy. The threshold value chosen may be adjusted to increase either the specificity or sensitivity of the test for a particular application.

### Conclusions:

The above results illustrate the utility of transcripts 2, 3, 4, 11, 12, 13, 15, 16, and 20 in the detection of testicular cancer, and testicular cancer subtypes, and in distinguishing malignant from normal testicular tissue. Transcript 2 was also found to have utility in the detection of prostate, breast, colorectal and ovarian cancer. Transcript 3 was also found to have utility in the detection of prostate, breast, melanoma, colorectal, and ovarian cancers. Transcript 4 was also found to have utility in the detection of prostate and colorectal cancers. Transcript 11 was also found to have utility in the detection of colorectal, melanoma, and ovarian cancers. Transcript 12 was also found to have utility in the detection of colorectal and ovarian cancers. Transcript 15 was also found to have utility in the detection of melanoma and ovarian cancers. Transcript 16 was also found to have utility in the detection of melanoma skin cancer. Transcript 20 was also found to have utility in the detection of colorectal cancer, melanoma, and ovarian cancer. Any of the 9 transcripts listed may be used individually or in combination as a tool for the detection or characterization of ovarian cancer in a clinical setting.

In one aspect, the invention provides a kit for conducting an assay for determining the presence of cancer in a tissue sample. The kit includes the required reagents for conducting the assay as described above. In particular, the kit includes one or more containers containing one or more hybridization probes corresponding to transcripts 1 to 17, and 20 described above. As will be understood, the reagents for conducting the assay may include any necessary buffers, salts, detection reagents etc. Further, the kit may include any necessary sample collection devices, containers etc. for obtaining the needed tissue samples, reagents or materials to prepare the tissue samples for example by homogenization or nucleic acid extraction, and for conducting the subject assay or assays. The kit may also include control tissues or samples to establish or validate acceptable values for diseased or non-diseased tissues.

### Example 10: Detection of Fusion Protein

### Cell lines

The presence of fusion proteins was investigated in two human prostate cell lines. Firstly the normal prostate cell line RWPE-1 (ATCC Cat# CRL-11609), these cells are non tumourigenic in nude mice and were established by infection with human papilloma virus 18 of histologically normal adult human prostate cells. Secondly a tumorigenic cell line WPE1-NA22 were examined (ATCC Cat# CRL-2849). These cells were derived from the RWPE-1 cells following exposure to N-methyl-N-nitrosourea. These cells are tumourogenic in nude mice unlike it's parent cell line RWPE-1.

Both cell lines were grown in Keratinicyte Serum Free Medium (Invitrogen Cat#17005-042), medium is supplemented with bovine pituitary extract and human recombinant epidermal growth factor. Cells were grown to 90% confluence then trypsinised using TrypLE Select (Invitrogen Cat#12563029). Cells were then counted using an automated counting system (Invitrogen Countess Cat#C10227), aliquots were then snap frozen and stored at -80°C.

### Protein Extraction

Cell fractions were extracted from both RWPE1 and WPE1-NA22 cell lines using the Qproteome Mitochondria Isolation Kit (Qiagen Cat#37612). Both mitochondrial and cytoplasmic fractions were extracted from 1x10⁷ cells. Protein concentration was then calculated using a fluorescent protein assay (Quant-IT Protein, Invitrogen Cat#Q33211) measured on a Qubit fluorometer (Invitrogen Cat#Q32857).

### SDS-Page Gel electrophoresis

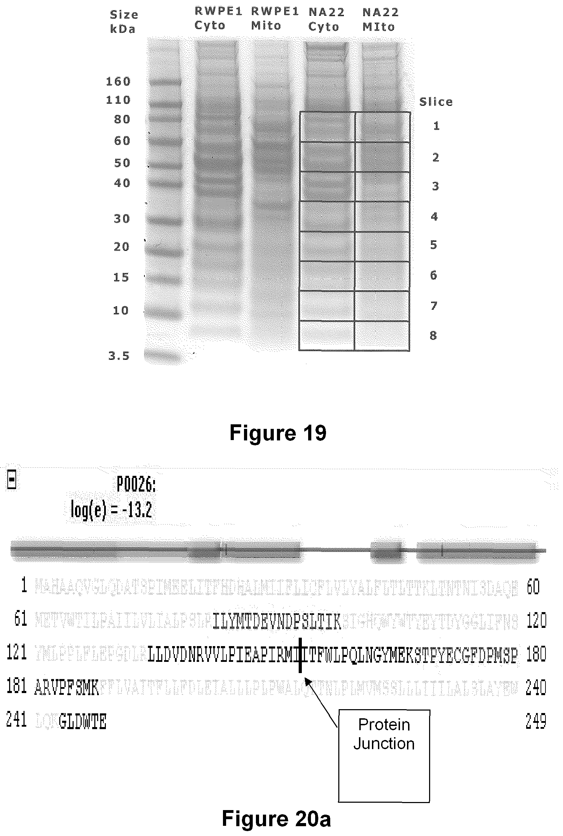

SDS-Page electrophoresis was carried out on mitochondrial and cytosolic fractions prepared using the Qproteome mitochondrial isolation kit. 20µg of protein was run in each lane on a 4-12% precast (invitrogen Nupage Cat#NP0321) bis-tris gel reducing gel, using a MES running buffer (Invitrogen Cat# NP00020). The gel was stained overnight with colloidal blue gel stain (Invitrogen Cat# LC6025). The results are illustrated in Figure 19. The approximate size (kD) range of proteins predicted to be contained in each of the 8 gel slices illustrated in Figure 19 are as follows:

| | |
|---|---|
| 1 | 60-80 |
| 2 | 50-60 |
| 3 | 40-50 |
| 4 | 30-40 |
| 5 | 20-30 |
| 6 | 15-20 |
| 7 | 10-15 |
| 8 | 3.5-10 |

### LCMS

Eight gel slices were cut out from each lane of a colloidal blue stained 1D SDS-PAGE (Figure 19) and in gel digested with trypsin following standard procedures.

The digestion products were eluted form the gel and evaporated. An aliquot was injected onto an LCMS system (Dionex / LC Packings Ultimate3000 coupled online to a Thermo LTQ XL orbitrap) and separated on a 25 cm (75 um ID) PepMap (Dionex) column at a flow rate of 300 ml/min with formic acid as a ion pairing agent and a linear gradient starting at 5% MeCN going to 40% MeCN over 110 min. MS spectra were collected in the orbitrap at a resolution of 60000 (400 Da) and MSMS spectra in the linear ion trap at low resolution.

Data were processed using Thermo Proteome Discoverer to generate .mgf (mascot generic format) peak list files, which were submitted in house to X!Tandem, searching a custom database comprised of the human proteome (ensembl) and predicted fusion proteins based on the fusion transcripts described previously. To calculate a false discovery rate (FDR), the searched database also included the reverse sequence of all proteins.

### Protein Complexes Analysis

Upon completion of the X!Tandem custom database search all identified proteins and fusion transcripts were returned. The proteins were scored by their log(e)⁺ values and classified as significant when the log(e)⁺ was less than negative one, with preference given to proteins with a log(e)⁺ less than negative three. Fusion proteins were identified by the presence of at least one peptide from each of the contributing genes of the fusion transcript present in the same gel slice. Protein sequence coverage from the identified peptides by the LC/MS-MS are displayed in red. The sequence of the protein which may be difficult to observe a peptide due to experimental conditions are indicated in green. Finally, protein sequence that is displayed in black represents a neutral possibility of identifying a peptide.

### Examples of Identified Fusion Proteins

Many mitochondrial fusion proteins were identified using this methodology. Four of such fusion proteins are described below as representative examples.

### Example Fusion Protein 1

Figures 20a illustrates the amino acid sequence of the fusion protein corresponding to the fusion transcript identified as P0026, which was identified (log(e)⁺ = -13.2) in slice 7 of the mitochondrial NA22 cell line (Figure 19) from the presence of the Cytochrome c oxidase subunit 2 (CO2) N-terminus peptide ILYMTDEVNDPSLTIK and the NADH-ubiquinone oxidoreductase chain 3 (ND3) C-terminus peptide STPYECGFDPMSP (Figure 20a).

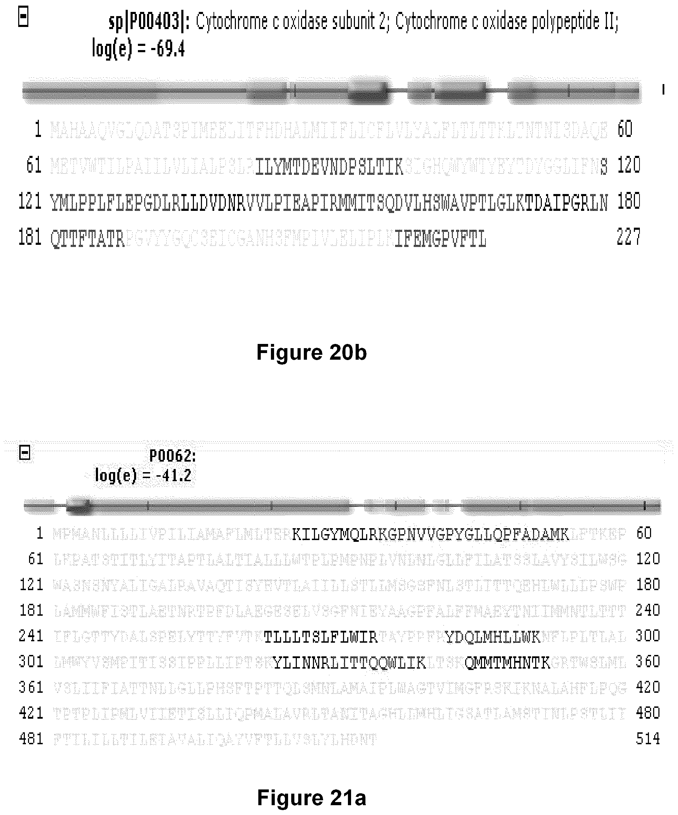

The most C-terminus tryptic peptide of wild-type CO2, IFEMGPVFTL, was searched against all mitochondrial NA22 cell line gel slices .xml data. This peptide was only observed in gel slice 5 (Figure 19). This was further confirmed by identifying CO2 wild-type (log(e)⁺ = -42.9) (Figure 20b) in mitochondrial NA22 cell line gel slice 5 only after searching the Human (SwissProt) database (no fusion transcripts) with all gel slices.

Cytochrome c oxidase subunit 2 peptide ILYMTDEVNDPSLTIK was observed in gel slices 5 and 7. This indicates that wild type CO2, with a molecular weight of ∼25kDa is present in the 20-30kDa gel slice 5, and a fragment of CO2 N-terminus exists in gel slice 7. The tryptic peptide STPYECGFDPMSP from ND3 is only identified in gel slice 7 (10-15kDa), which identifies the wild-type gene (13kDa) and the C-terminus of P0026.

The sequences for the fusion transcript P0026, the mutant DNA from which it is derived and the resulting protein are provided herein, respectively, as SEQ ID NO: 56, SEQ ID NO: 57 and SEQ ID NO: 58.

### Example Fusion Protein 2

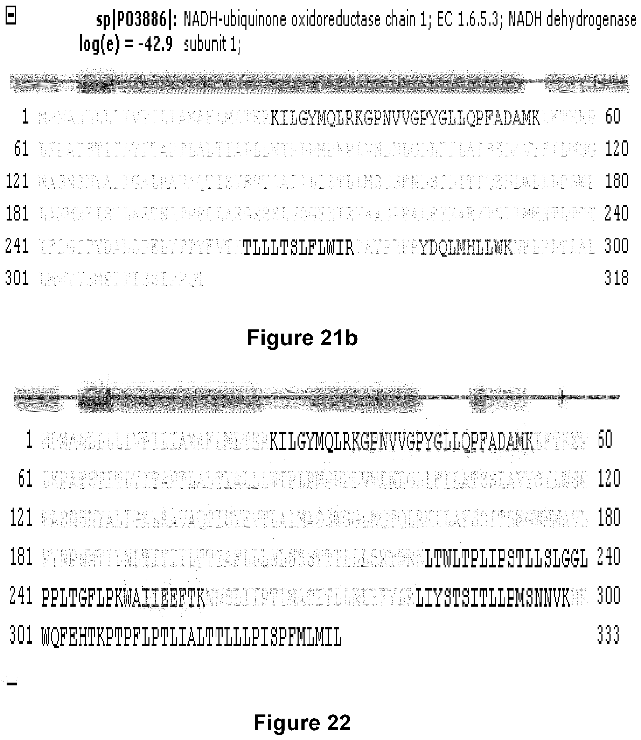

Figure 21 a illustrates the amino acid sequence of the fusion protein corresponding to fusion transcript P0062, which was identified (log(e)⁺ = -41.2) in slice 5 (20-30kDa), shown in Figure 19, of the mitochondrial NA22 cell line from the presence of the NADH dehydrogenase subunit 1 (ND1) N-terminus peptides KGPNVVGPYGLLQPFADAMK and YDQLMHLLWK and the ATP synthase subunit 6 C-terminus peptide LITTQQWLIK. All three peptides were identified in the mitochondrial NA22 cell line gel slice 5 (Figure 19) but due to the most C-terminus peptide of ND1 (YDQLMHLLWK) being present only in gel slice 5, the presence of both wild-type (Figure 21b) and the fusion protein corresponding to fusion transcript P0062 is possible.

The sequences for the fusion transcript P0062, the mutant DNA from which it is derived and the resulting protein are provided herein, respectively, as SEQ ID NO: 59, SEQ ID NO: 60 and SEQ ID NO: 61.

### Example Fusion Protein 3

Figure 22 illustrates the amino acid sequence of the fusion protein corresponding to fusion transcript P0064, which was identified in slice 4 (log(e)+ = -22), shown in Figure 19, of the mitochondrial NA22 cell line with the peptide KGPNVVGPYGLLQPFADAMK from the N-terminus of ND1 and the NADH dehydrogenase subunit 2 (ND2) C-terminus peptide WAIIEEFTK. The ND1 C-terminus peptide YDQLMHLLWK was not observed in gel slice 4, and based on the expected sizes of P0064 and ND2 it is suggested that gel slice 4 contains P0064 and ND2.

The sequences for the fusion transcript P0064, the mutant DNA from which it is derived and the resulting protein are provided herein, respectively, as SEQ ID NO: 62, SEQ ID NO: 63 and SEQ ID NO: 64.

### Example Fusion Protein 4

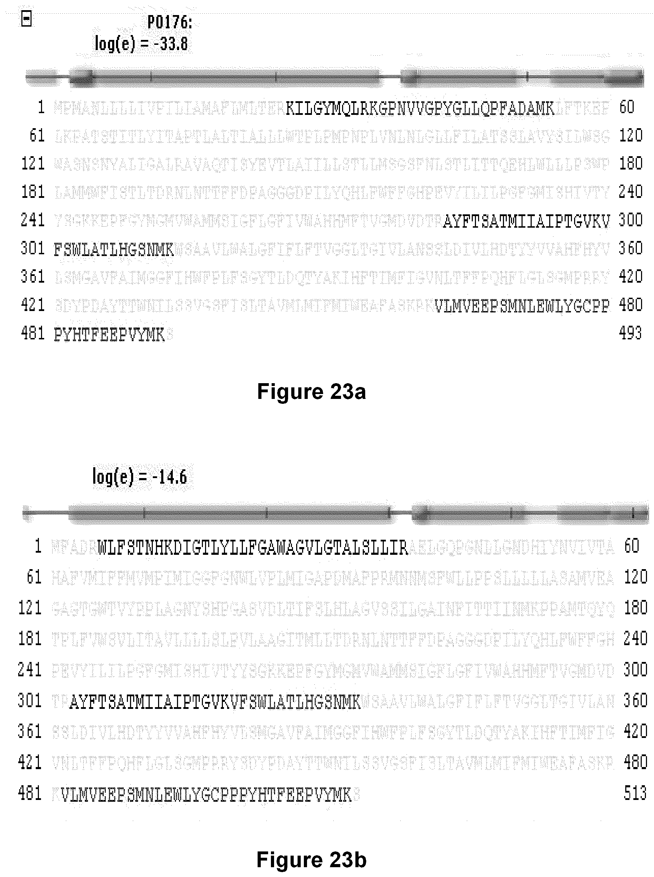

Figure 23a illustrates the amino acid sequence of the fusion protein corresponding to fusion transcript P0176, which was identified in slice 4 (log(e)+ = -33.8), shown in Figure 19, of the mitochondrial NA22 cell line with the peptide KGPNVVGPYGLLQPFADAMK from the N-terminus of ND1 and the Cytochrome c oxidase subunit 1 (CO1) C-terminus peptides VFSWLATLHGSNMK and VLMVEEPSMNLEWLYGCPPPYHTFEEPVYMK. Both of the CO1 peptides were only observed together in gel slice 4 (30-40kDa) of the mitochondrial NA22 cell line despite an expected size of 55 kDa. This was further confirmed by identifying CO1 wild-type (log(e)⁺ = -14.6) (Figure 23b) in mitochondrial NA22 cell line gel slice 4 only after searching the Human (SwissProt) database (no fusion transcripts) with all gel slices.

The only ND1 peptide observed in gel slice 4 was KGPNVVGPYGLLQPFADAMK. Since the ND1 C-terminus peptide YDQLMHLLWK was not present, wild-type ND1 is not present in the slice, which supports the presence of P0176.

The sequences for the fusion transcript P0176, the mutant DNA from which it is derived and the resulting protein are provided herein, respectively, as SEQ ID NO: 65, SEQ ID NO: 66 and SEQ ID NO: 67.

### Corresponding Fusion Transcripts

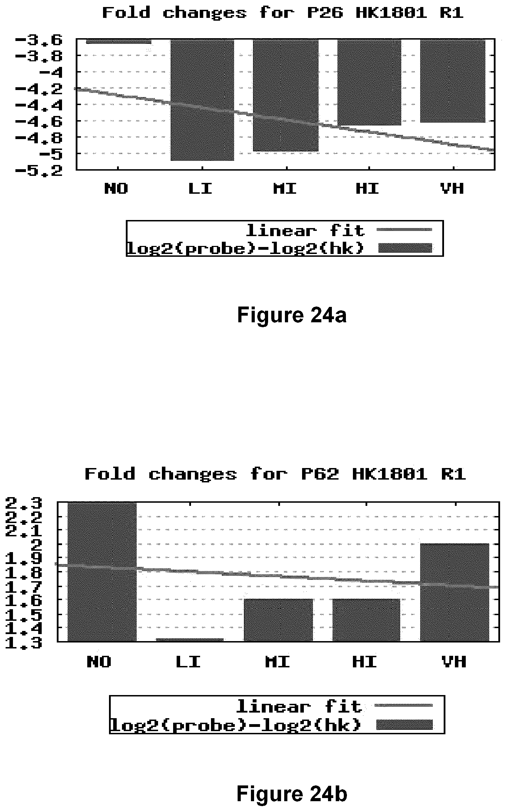

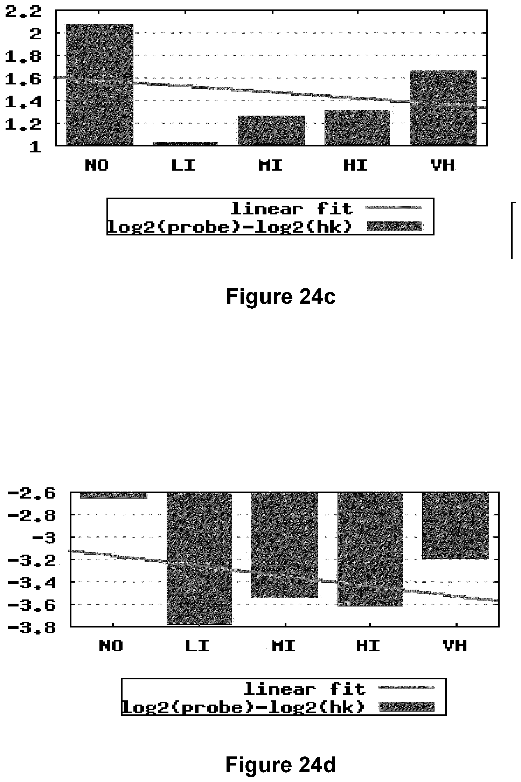

Quantitative measurements of the fusion transcripts associated with each of these four fusion proteins were conducted in a series of cell lines of which two were those used in the LC-MS/MS experiment, specifically RWPE-1 and WPE1-NA22, which is a malignant cell line with low invasive potential. The results of these measurements is illustrated in Figures 24a-24d, corresponding to the four proteins discussed above. In Figures 24a-d, cell line RWPE-1 is indicated as NO and cell line WPE1-NA22 is indicated as LI. The additional cell lines included in this experiment represent a continued progression of malignancy with moderate invasive potential (MI), high invasive potential (HI), and very high invasive potential (VH).

The cells were lysed and assayed using custom probes specific to each of the fusion transcripts on the branching DNA platform as described herein or previously in PCT application no. PCT/CA2009/000351 (published under number WO 2009/117811). Results indicated high levels of expression (with RLU values ranging from 10⁶⁻10⁸). A general trend was observed in the quantity of each fusion transcript in that the initial transformation from normal cells to malignant cells (NO-LI) was punctuated by a marked change in quantity of the transcript, followed by either a continued increase or continued decrease in the quantity as malignant progression proceeds from LI to VH).

Any examples provided herein are included solely for the purpose of illustrating the invention and are not intended to limit the claims in any way. Any drawings provided herein are solely for the purpose of illustrating various aspects of the invention and are not intended to be drawn to scale or to limit the claims in any way.

### Bibliography

The following references, amongst others, were cited in the foregoing description..

| **Author** | **Journal** | **Title** | **Volume** | **Date** |
|---|---|---|---|---|
| Anderson et al | Nature | Sequence and Organization of the Human Mitochondrial Genome | 290(5806):457-65 | 1981 |
| Andrews et al | Nat Genet | Reanalysis and revision of the Cambridge reference sequence for human mitochondrial DNA. | 23(2):147 | 1999 |
| Modica-Napolitano et al | Expert Rev Mol Med | Mitochondria as targets for detection and treatment of cancer | 4:1-19 | 2002 |
| Sherratt et al | Clin Sci (Lond) | Mitochondrial DNA defects: a widening clinical spectrum of disorders. | 92(3):225-35 | 1997 |
| Croteau et al | Mutat Res | Mitochondrial DNA repair pathways. | 434(3):137-48 | 1999 |
| Green and Kroemer | J Clin Invest | Pharmacological manipulation of cell death: clinical applications in sight? | 115(10): 2610-2617 | 2005 |
| Dai et al | Acta Otolaryngol | Correlation of cochlear blood supply with mitochondrial DNA common deletion in presbyacusis. | 24(2):130-6 | 2004 |
| Ro et al | Muscle Nerve | Deleted 4977-bp mitochondrial DNA mutation is associated with sporadic amyotrophic lateral sclerosis: a hospital-based case-control study. | 28(6):737-43 | 2003 |
| Barron et al | Invest Ophthalmol Vis Sci | Mitochondrial abnormalities in ageing macular photoreceptors. | 42(12):3016-22 | 2001 |
| Lewis et al | J Pathol | Detection of damage to the mitochondrial genome in the oncocytic cells of Warthin's tumour. | 191(3):274-81 | 2000 |
| Muller-Hocker et al | Mod Pathol | The common 4977 base pair deletion of mitochondrial DNA preferentially accumulates in the cardiac conduction system of patients with Kearns-Sayre syndrome. | 11(3):295-301. | 1998 |
| Porteous et al | Eur J Biochem | Bioenergetic consequences of accumulating the common 4977-bp mitochondrial DNA deletion. | 257(1):192-201 | 1998 |
| Parr et al | J Mol Diagn | Somatic mitochondrial DNA mutations in prostate cancer and normal appearing adjacent glands in comparison to age-matched prostate samples without malignant histology. | 8(3):312-9. | 2006 |
| Maki et al | Am J Clin Pathol | Mitochondrial genome deletion aids in the identification of false- and true-negative prostate needle core biopsy specimens. | 129(1):57-66 | 2008 |
| Nakase et al | Am J Hum Genet | Transcription and translation of deleted mitochondrial genomes in Kearns-Sayre syndrome: implications for pathogenesis. | 46(3):418-27. | 1990 |
| Libura et al | Blood | Therapy-related acute myeloid leukemia-like MLL rearrangements are induced by etoposide in primary human CD34+ cells and remain stable after clonal expansion. | 105(5):2124-31 | 2005 |
| Meyer et al | Proc Natl Acad Sci U S A | Diagnostic tool for the identification of MLL rearrangements including unknown partner genes. | 102(2):449-54 | 2005 |
| Eguchi et al | Genes Chromosomes Cancer | MLL chimeric protein activation renders cells vulnerable to chromosomal damage: an explanation for the very short latency of infant leukemia. | 45(8):754-60 | 2006 |
| Hayashi et al | Proc Natl Acad Sci U S A | Introduction of disease-related mitochondrial DNA deletions into HeLa cells lacking mitochondrial DNA results in mitochondrial dysfunction | 88:10614-10618 | 1991 |

**Table 1: Known mitochondrial deletions having an ORF**

| Deletion Junction (nt:nt) | Deletion Size (bp) | Repeat Location (nt/nt) | Number of Repeats | References |
|---|---|---|---|---|
| COXI - ND5 | | | | |
| 6075:13799 | -7723 | 6076-6084/13799-13807 | D, 9/9 | ▪Mita, S., Rizzuto, R., Moraes, C.T., Shanske, S., Arnaudo, E., Fabrizi, G.M., Koga, Y., DiMauro, S., Schon, E.A. (1990) "Recombination via flanking direct repeats is a major cause of large-scale deletions of human mitochondrial DNA" Nucleic Acids Research 18(3):561-567 |
| 6238:14103 | -7864 | 6235-6238/14099-14102 | D, 4/4 | ▪Blok, R.B., Thorburn, D.R., Thompson, G.N., Dahl, H.H. (1995) "A topoisomerase II cleavage site is associated with a novel mitochondrial DNA deletion" Human Genetics 95 (1): 75-81 |
| 6325:13989 | -7663 | 6326-6341/13889-14004 | D, 16/17 | ▪Larsson, N.G., Holme, E., Kristiansson, B., Oldfors, A., Tulinius, M. (1990) "Progressive increase of the mutated mitochondrial DNA fraction in Kearns-Sayre syndrome " Pediatric Research 28 (2): 131-136 ▪Larsson, N.G., Holme, E. (1992) "Multiple short direct repeats associated with single mtDNA deletions "Biochimica et Biophysica Acta 1139 (4): 311-314 |
| 6330:13994 | -7663 | 6331-6341/13994-14004 | D, 11/11 ' | ▪Mita, S., Rizzuto, R., Moraes, C.T., Shanske, S., Arnaudo, E., Fabrizi, G.M., Koga, Y., DiMauro, S., Schon, E.A. (1990) "Recombination via flanking direct repeats is a major cause of large-scale deletions of human mitochondrial DNA" Nucleic Acids Research 18(3):561-567 |

| COX II - ND5 | | | | |
|---|---|---|---|---|
| 7829:14135 | -6305 | 7824-7829/14129-14134 | D, 6/6 | ▪Bet, L., Moggio, M., Comi, G.P., Mariani, C., Prelle, A., Checcarelli, N., Bordoni, A., Bresolin, N., Scarpini, E., Scarlato, G. (1994) "Multiple sclerosis and mitochondrial myopathy: an unusual combination of diseases" Journal of Neurology 241 (8): 511-516 |
| 8213:13991 | -5777 | 8214-8220/13991-13997 | D, 7/7 | ▪Hinokio, Y., Suzuki, S., Komatu, K., Ohtomo, M., Onoda, M., Matsumoto, M., Hirai, S., Sato, Y., Akai, H., Abe, K., Toyota, T. (1995) "A new mitochondrial DNA deletion associated with diabetic amyotrophy, diabetic myoatrophy and diabetic fatty liver" Muscle and Nerve 3 (9): S142-149 |

| ATPase - ND5 | | | | |
|---|---|---|---|---|
| 8631:13513 | -4881 | 8625-8631/13506-13512 | D, 7/7 | ▪Zhang, C., Baumer, A., Mackay, I.R., Linnane, A.W., Nagley, P. (1995) "Unusual pattern of mitochondrial DNA deletions in skeletal muscle of an adult human with chronic fatigue syndrome" Human Molecular Genetics 4 (4): 751-754 |
| 9144:13816 | -4671 | 9137-9144/13808-13815 | D, 8/8 | ▪Ota, Y., Tanaka, M., Sato, W., Ohno, K., Yamamoto, T., Maehara, M., Negoro, T., Watanabe, K., Awaya, S., Ozawa, T. (1991) "Detection of platelet mitochondrial DNA deletions in Kearns-Sayre syndrome" Investigative Ophthalmology and Visual Science 32 (10): 2667-2675 |
| 9191:12909 | -3717 | 9189-9191/12906-12908 | D, 3/3 | ▪Tanaka, M., Sato, W., Ohno, K., Yamamoto, T., Ozawa, T. (1989) "Direct sequencing of mitochondrial DNA in myopathic patients" Biochemical and Biophysical Research Communications 164 (): 156-163 |

| COX III - ND5 | | | | |
|---|---|---|---|---|
| 10190:13753 | -3562 | 10191-101 98/13753-13760 | D, 8/8 | ▪Rotig, A., Bourgeron, T., Chretien, D., Rustin, P., Munnich, A. (1995) "Spectrum of mitochondrial DNA rearrangements in the Pearson marrow-pancreas syndrome" Human Molecular Genetics 4 (8): 1327-1330 ▪Rotig, A., Cormier, V., Koll, F., Mize, C. E., Saudubray, J.-M., Veerman, A., Pearson, H. A., Munnich, A. (1991) "Site-specific deletions of the mitochondrial genome in Pearson marrow-pancreas sundrome" Genomics 10 (2): 502-504 |
| 10367:12829 | -2461 | 10365-10367/12826-12828 | D, 3/3 | ▪Kapsa, R., Thompson, G.N., Thorburn, D.R., Dahl, H.H., Marzuki, S., Byrne, E., Blok, R.B. (1994) "A novel mtDNA deletion in an infant with Pearson syndrome" Journal of Inherited Metabolic Disease 17 (5): 521 - 526 |

| ND4L - ND5 | | | | |
|---|---|---|---|---|
| 10744:14124 | -3379 | 10745-10754/14124-14133 | D, 9/10 | ▪Cormier-Daire, V., Bonnefont, J.P., Rustin, P., Maurage, C., Ogler, H., Schmitz, J., Ricour, C., Saudubray, J.M., Munnich, A., Rotig, A. (1994) "Mitochondrial DNA rearrangements with onset as chronic diarrhea with villous atrophy" Journal of Pediatrics 124 (1): 63-70 |

| ND4 - ND5 | | | | |
|---|---|---|---|---|
| 11232:13980 | -2747 | 11234-11242/13981-13989 | D, 9/9 | ▪Rotig, A., Cormier, V., Koll, F., Mize, C. E., Saudubray, J.-M., Veerman, A., Pearson, H. A., Munnich, A. (1991) "Site-specific deletions of the mitochondrial genome in Pearson marrow-pancreas syndrome" Genomics 10 (2): 502-504 ▪Rotig, A., Cormier, V., Blanche, S., Bonnefont, J.P., Ledeist, F., Romero, N., Schmitz, J., Rustin, P., Fischer, A., Saudubray, J.M. (1990) "Pearson's marrow-pancreas syndrome. A multi-system mitochondrial disorder in infancy" Journal of Clinical Investigation 86 (): 1601-1608 ▪Cormier, V., Rotig, A., Quartino, A.R., Forni, G.L., Cerone, R., Maier, M., Saudubray, J.M., Munnich, A. (1990) "Widespread multitissue deletions of the mitochondrial genome in Pearson marrow-pancreas syndrome" Journal of Pediatrics 117 (4): 599-602 ▪Awata, T., Matsumoto, T., Iwamoto, Y., Matsuda, A., Kuzuya, T., Saito, T. (1993) "Japanese case of diabetes mellitus and deafness with mutations in mitochondrial tRNALeu(UUR) gene [letter]" Lancet 341 (8855) 1291-1292 |

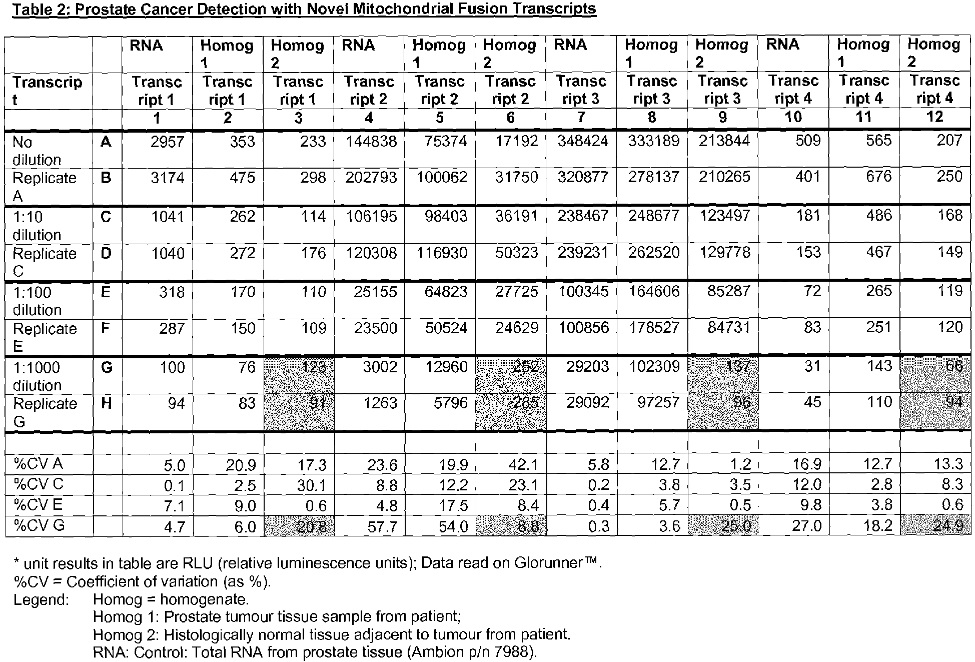

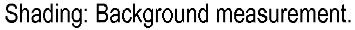

**Table 3: Deletion/Transcript/Hypothetical translation product relationships**

| **Deletion** | **RNA transcript** | **DNA sequence with deletion complementary to RNA transcript** | **Transcript No.** | **Hypothetical Fusion Protein** |
|---|---|---|---|---|
| ATP synthase F0 subunit 8 to NADH dehydrogenase subunit mitochondrial positions 8366-14148 (with reference to SEQ ID NO: 1). Translated sequence begins at position 8389 | SEQ ID NO: 19 | SEQ ID NO: 2 | **1** | SEQ ID NO: 36 |
| NADH dehydrogenase subunit 4L (ND4L) to NADH dehydrogenase subunit 5 (ND5); Mitochondrial positions 10470-14148 (with reference to SEQ ID NO: 1) | SEQ ID NO: 20 | SEQ ID NO: 3 | **2** | SEQ ID NO: 37 |
| Cytochrome c oxidase subunit II (COII) to Cytochrome b (Cytb); Mitochondrial positions 7586-15887 (with reference to SEQ ID NO: 1) | SEQ ID NO: 21 | SEQ ID NO:4 | **3** | SEQ ID NO: 38 |
| Cytochrome c oxidase subunit II (COII) to Cytochrome b (Cytb); Mitochondrial positions 7586-15887 (with reference to SEQ ID NO:1) | SEQ ID NO: 22 | SEQ ID NO:5 | **4** | SEQ ID NO: 39 |

**Table 4: Breast and Prostate Cancer Detection**

| | | **Breast Tumour 1** | **Normal adjacent Breast Tumour 1** | | **Breast Tumour 2** | **Normal Adjacent to Breast Tumour 2** | | **Prostate Tumour 3** | **Prostate Tumour 4** | **Prostate Tumour 5** | **Normal Adjacent to Prostate Tumour 5** |
|---|---|---|---|---|---|---|---|---|---|---|---|
| | | **1** | **2** | | **3** | **4** | | **5** | **6** | **7** | **8** |
| 1:100 dilution | **E** | 68920 | 2971 | | 49108 | 1245 | | 46723 | 56679 | 99836 | 35504 |
| 1:100 dilution replicate | **F** | 92409 | 3017 | | 60637 | 1512 | | 53940 | 56155 | 100582 | 44221 |
| | **G** | 420 | 3 | | 31 | 6 | | 26 | 25 | 44 | 23 |
| | **H** | 518 | 3 | | 4 | 5 | | 5 | 3 | 4 | 2 |
| | | | | | | | | | | | |
| | **%CV** | 20.6 | 1.1 | | 14.9 | 13.7 | | 10.1 | 0.7 | 0.5 | 15.5 |

| | | | | | | | | | | | |
|---|---|---|---|---|---|---|---|---|---|---|---|
| - unit results in table are RLU (relative luminescence units) - background G1, H1 - empty well G2-G8, H2- H8 | | | | | | | | | | | |

**Table 5a: Assay Conditions**

| | **Template for the assay** | | | | | | | | | | | |
|---|---|---|---|---|---|---|---|---|---|---|---|---|
| | **RNA** | **Homogen 1** | **Homogen 2** | **RNA** | **Homogen 1** | **Homogen 2** | **RNA** | **Homogen 1** | **Homogen 2** | **RNA** | **Homogen 1** | **Homogen 2** |
| | **Transcript 1** | **Transcript 1** | **Transcript 1** | **Transcript 2** | **Transcript 2** | **Transcript 2** | **Transcript 3** | **Transcript 3** | **Transcript 3** | **Transcript 4** | **Transcript 4** | **Transcript 4** |
| | **1** | **2** | **3** | **4** | **5** | **6** | **7** | **8** | **9** | **10** | **11** | **12** |
| **A** | RNA | Homog 1 | Homog 2 | RNA | Homog 1 | Homog 2 | RNA | Homog 1 | Homog 2 | RNA | Homog 1 | Homog 2 |
| **B** | Dil 1 | Dil 1 | Dil 1 | Dil 1 | Dil 1 | Dil 1 | Dil 1 | Dil 1 | Dil 1 | Dil 1 | Dil 1 | Dil 1 |
| **C** | RNA | Homog 1 | Homog 2 | RNA | Homog 1 | Homog 2 | RNA | Homog 1 | Homog 2 | RNA | Homog 1 | Homog 2 |
| **D** | Dil 2 | Dil 2 | Dil 2 | Dil 2 | Dil 2 | Dil 2 | Dil 2 | Dil 2 | Dil 2 | Dil 2 | Dil 2 | Dil 2 |
| **E** | RNA | Homog 1 | Homog 2 | RNA | Homog 1 | Homog 2 | RNA | Homog 1 | Homog 2 | RNA | Homog 1 | Homog 2 |
| **F** | Dil 3 | Dil 3 | Dil 3 | Dil 3 | Dil 3 | Dil 3 | Dil 3 | Dil 3 | Dil 3 | Dil 3 | Dil 3 | Dil 3 |
| **G** | RNA | Homog 1 | **Transcript** 1 | RNA | Homog 1 | **Transcript** 1 | RNA | Homog 1 | **Transcript** 1 | RNA | Homog 1 | **Transcript** 1 |
| **H** | Dil 4 | Dil 4 | Background | Dil 4 | Dil 4 | Background | Dil 4 | Dil 4 | Background | Dil 4 | Dil 4 | Background |

| | | | | | | | | | | | | |
|---|---|---|---|---|---|---|---|---|---|---|---|---|
| Homogenate1- Used 26 mg of tissue to homogenize in 700ul H soln with Proteinase K (PK). Used Qiagen TissueRuptor. Used 40ul homogenate supernatant, 20, 10 and 5 ul for dilution Homogenate1= Tumour tissue from the tumorous Prostate Homogenate2- Used 29 mg of tissue to homogenize in 700ul H soln with PK. Used Qiagen TissueRuptor. Used 40ul homogenate supernatant, 20, 10 and 5 ul for dilution Homogenate2= Normal tissue from the tumorous Prostate RNA dilution was made as below. RNA was from Prostate Normal from Ambion. Assay was done in duplicates. | | | | | | | | | | | | |

**Table 5b: RNA dilution**

| **RNA Dilution** | | ng/ul |
|---|---|---|
| | Dil 1 | 3000 |
| **1:3 dil** | Dil 2 | 1000 |
| **Serial dil** | Dil 3 | 333 |
| | Dil 4 | 111 |

**Table 6: Transcript Summary by Disease**

| Probe | Prostate Cancer | Breast Cancer | Colorectal Cancer | Melanoma Skin Cancer | Lung Cancer | Ovarian Cancer | Testicular Cancer |
|---|---|---|---|---|---|---|---|
| 1 | • | | | | | • | |
| 2 | • | • | • | | | • | • |
| 3 | • | | • | • | | • | • |
| 4 | • | | • | | | | • |
| 5 | | | • | • | | | |
| 6 | | | | • | • | • | |
| 7 | | | • | • | | | |
| 8 | | | | | • | | |
| 9 | | | | • | | | |
| 10 | | | • | | • | | |
| 11 | | | • | • | | • | • |
| 12 | | | • | | | • | • |
| 13 | | | | | | | • |
| 14 | | | | • | | | |
| 15 | | | | • | | • | • |
| 16 | | | | • | | | • |
| 17 | | | | • | | | |
| 20 | | | • | • | | • | • |

## Claims

1. An isolated mitochondrial fusion protein, the fusion protein having an amino acid sequence resulting from the translation of a mitochondrial fusion transcript corresponding to a mutation in mitochondrial DNA, wherein the fusion protein has an amino acid sequence as set forth in SEQ ID NO: 58.

2. A method of detecting the presence of a cancer in a mammal, the method comprising assaying a tissue sample from the mammal for the presence of at least one mitochondrial fusion protein, the fusion protein having an amino acid sequence resulting from the translation of a mitochondrial fusion transcript corresponding to a mutation in mitochondrial DNA, wherein the fusion protein has an amino acid sequence as set forth in SEQ ID NO: 58.

3. The method of claim 2, wherein the cancer is selected from the group consisting of prostate cancer, testicular cancer, ovarian cancer, breast cancer, colorectal cancer, lung cancer and melanoma skin cancer.

4. The method of claim 2 or 3, wherein said assaying comprises an immunological assay, and wherein said assaying is conducted with an antibody, or an antigen-binding fragment thereof, having a specificity to the fusion protein of claim 1.

5. A kit for conducting an assay for detecting the presence of a cancer in a mammal, said kit comprising an antibody, or an antigen-binding fragment thereof, having a specificity to the fusion protein of claim 1 and instructions for detecting cancer.

6. A kit according to claim 5, wherein the wherein the cancer is selected from the group consisting of prostate cancer, testicular cancer, ovarian cancer, breast cancer, colorectal cancer, lung cancer and melanoma skin cancer.

7. An isolated mitochondrial fusion transcript having a nucleic acid sequence as set forth in SEQ ID NO: 56.

8. An isolated mtDNA encoding the fusion transcript of claim 7.

9. The isolated mtDNA of claim 8 having a sequence as set forth in SEQ ID NO: 57.

10. A method of detecting the presence of a cancer in a mammal, the method comprising assaying a tissue sample from the mammal for the presence of the mitochondrial fusion transcript according to claim 7, by quantifying the amount of the fusion transcript in the sample and comparing the quantity detected with a reference value.

11. The method of claim 10, wherein the cancer is selected from the group consisting of prostate cancer, testicular cancer, ovarian cancer, breast cancer, colorectal cancer, lung cancer and melanoma skin cancer.

12. The method of claim 4, wherein the antibody is a polyclonal or monoclonal antibody.

13. The kit of claim 5, wherein the antibody is a polyclonal or monoclonal antibody.

## Patentansprüche

1. Isoliertes mitochondriales Fusionsprotein, wobei das Fusionsprotein eine Aminosäuresequenz aufweist, die aus der Translation eines mitochondrialen Fusionstranskripts resultiert, das einer Mutation in mitochondrialer DNA entspricht, wobei das Fusionsprotein eine Aminosäuresequenz gemäß SEQ ID NO: 58 aufweist.

2. Verfahren zum Nachweis der Anwesenheit von Krebs bei einem Säugetier, wobei das Verfahren das Testen einer Gewebeprobe von dem Säugetier auf die Anwesenheit von mindestens einem mitochondrialen Fusionsprotein umfasst, wobei das Fusionsprotein eine Aminosäuresequenz aufweist, die aus der Translation eines mitochondrialen Fusionstranskripts resultiert, das einer Mutation in mitochondrialer DNA entspricht, wobei das Fusionsprotein eine Aminosäuresequenz gemäß SEQ ID NO: 58 aufweist.

3. Verfahren nach Anspruch 2, wobei der Krebs aus der Gruppe ausgewählt ist, bestehend aus Prostatakrebs, Hodenkrebs, Eierstockkrebs, Brustkrebs, Darmkrebs, Lungenkrebs und Melanomhautkrebs.

4. Verfahren nach Anspruch 2 oder 3, wobei der Assay einen immunologischen Assay umfasst und wobei der Assay mit einem Antikörper oder einem Antigen-bindenden Fragment davon mit einer Spezifizität zu dem Fusionsprotein nach Anspruch 1 durchgeführt wird.

5. Kit zur Durchführung eines Assays zum Nachweis der Anwesenheit von Krebs bei einem Säugetier, wobei der Kit einen Antikörper oder ein Antigen-bindendes Fragment davon mit einer Spezifizität zu dem Fusionsprotein nach Anspruch 1 und Anleitungen zum Nachweis von Krebs umfasst.

6. Kit nach Anspruch 5, wobei der Krebs aus der Gruppe ausgewählt ist, bestehend aus Prostatakrebs, Hodenkrebs, Eierstockkrebs, Brustkrebs, Darmkrebs, Lungenkrebs und Melanomhautkrebs.

7. Isoliertes mitochondriales Fusionstranskript mit einer Nukleinsäuresequenz gemäß SEQ ID NO: 56.

8. Isolierte mtDNA, die für das Fusionstranskript nach Anspruch 7 kodiert.

9. Isolierte mtDNA nach Anspruch 8, welche eine Sequenz gemäß SEQ ID NO: 57 aufweist.

10. Verfahren zum Nachweis der Anwesenheit von Krebs bei einem Säugetier, wobei das Verfahren das Testen einer Gewebeprobe von dem Säugetier auf die Anwesenheit von mindestens einem mitochondrialen Fusionstranskript nach Anspruch 7 umfasst, durch Quantifizierung der Menge des Fusionstranskripts in der Probe und Vergleich der nachgewiesenen Menge mit einem Vergleichswert.

11. Verfahren nach Anspruch 10, wobei der Krebs aus der Gruppe ausgewählt ist, bestehend aus Prostatakrebs, Hodenkrebs, Eierstockkrebs, Brustkrebs, Darmkrebs, Lungenkrebs und Melanomhautkrebs.

12. Verfahren nach Anspruch 4, wobei der Antikörper ein polyklonaler oder monoklonaler Antikörper ist.

13. Kit nach Anspruch 5, wobei der Antikörper ein polyklonaler oder monoklonaler Antikörper ist.

## Revendications

1. Protéine de fusion mitochondriale isolée, la protéine de fusion présentant une séquence d'acides aminés résultant de la traduction d'un transcrit de fusion mitochondriale correspondant à une mutation de l'ADN mitochondrial, dans laquelle la protéine de fusion présente une séquence d'acides aminés telle que définie par SEQ ID NO : 58.

2. Procédé de détection de la présence d'un cancer chez un mammifère, le procédé comprenant le dosage d'un échantillon de tissu en provenance du mammifère pour la présence d'au moins une protéine de fusion mitochondriale, la protéine de fusion présentant une séquence d'acides aminés résultant de la traduction d'un transcrit de fusion mitochondriale correspondant à une mutation de l'ADN mitochondrial, dans laquelle la protéine de fusion présente une séquence d'acides aminés telle que définie par SEQ ID NO : 58.

3. Procédé selon la revendication 2, dans laquelle le cancer est choisie dans le groupe constitué en le cancer de la prostate, le cancer des testicules, le cancer ovarien, le cancer du sein, le cancer colorectal, le cancer du poumon et le cancer de la peau avec présence de mélanome.

4. Procédé selon la revendication 2 ou 3, dans lequel ladite dosage comprend une dosage immunologique et dans lequel ladite dosage est réalisée avec un anticorps, ou un fragment de liaison à un antigène de celui-ci, présentant une spécificité pour la protéine de fusion selon la revendication 1.

5. Kit d'analyse pour détecter la présence d'un cancer chez un mammifère, ledit kit comprenant un anticorps, ou un fragment de liaison à un antigène de celui-ci, présentant une spécificité pour la protéine de fusion selon la revendication 1 et des instructions pour détecter un cancer.

6. Kit selon la revendication 5, dans lequel le cancer est choisie dans le groupe constitué en le cancer de la prostate, le cancer des testicules, le cancer ovarien, le cancer du sein, le cancer colorectal, le cancer du poumon et le cancer de la peau avec présence de mélanome.

7. Transcrit de fusion mitochondriale isolé présentant une séquence d'acides nucléiques telle que définie par SEQ ID NO : 56.

8. ADN mitochondrial isolé codant pour le transcrit de fusion selon la revendication 7.

9. ADN mitochondrial isolé selon la revendication 8 présentant une séquence telle que définie par SEQ ID NO : 57.

10. Procédé de détection de la présence d'un cancer chez un mammifère, le procédé comprenant le dosage d'un échantillon de tissu en provenance du mammifère pour la présence du transcrit de fusion mitochondriale selon la revendication 7, en quantifiant la quantité du transcrit de fusion dans l'échantillon et en comparant la quantité détectée avec une valeur de référence.

11. Procédé selon la revendication 10, dans lequel le cancer est choisie dans le groupe constitué en le cancer de la prostate, le cancer des testicules, le cancer ovarien, le cancer du sein, le cancer colorectal, le cancer du poumon et le cancer de la peau avec présence de mélanome.

12. Procédé selon la revendication 4, dans lequel l'anticorps est un anticorps polyclonal ou monoclonal.

13. Kit selon la revendication 5, dans lequel l'anticorps est un anticorps polyclonal ou monoclonal.
